# EUROPEAN PATENT APPLICATION

(11) **EP 4 438 100 A2**
(43) Date of publication of application: **02.10.2024**
(21) Application number: 24175374.8
(22) Date of filing: 15.11.2018
(51) Int. Cl.: A61M 25/10

(54) **INTRAGASTRIC DEVICE WITH SELF-DEFLATION**

(30) Priority: 07.12.2017 US 201762596067 P
(62) Divisional of application: 18886542.2
(71) Applicant: ReShape Lifesciences Inc., Irvine, CA 92618 (US)
(72) Inventor: BRISTER, Mark C., Carlsbad, 92008 (US); DRAKE, Neil R., Carlsbad, 92008 (US); RAMASWAMY, Rajan, Carlsbad, 92008 (US); FAUCHER, Paul Daniel, Carlsbad, 92008 (US); RHOADS, William Wistar, Carlsbad, 92008 (US); MACCOLLUM, Michael W., Carlsbad, 92008 (US)
(74) Representative: Boult Wade Tennant LLP

(57) **Abstract**

Devices and methods for treating obesity are provided. More particularly, intragastric devices and devices for inflating and methods of fabricating, deploying, inflating, monitoring, and retrieving the same are provided.

## Description

### INCORPORATION BY REFERENCE TO RELATED APPLICATION

This application claims the benefit of U.S. Application No. 62/596,067, filed December 7, 2017. The aforementioned application is incorporated by reference herein in its entirety, and is hereby expressly made a part of this specification.

### FIELD OF THE INVENTION

Devices and methods for treating obesity are provided. More particularly, intragastric devices and methods of fabricating, deploying, inflating, monitoring, and retrieving the same are provided.

### BACKGROUND OF THE INVENTION

Obesity is a major health problem in developed countries. Obesity puts you at greater risk of developing high blood pressure, diabetes and many other serious health problems. In the United States, the complications of being overweight or obese are estimated to affect nearly one in three American adults, with an annual medical cost of over $80 billion and, including indirect costs such as lost wages, a total annual economic cost of over $120 billion. Except for rare pathological conditions, weight gain is directly correlated to overeating.

Noninvasive methods for reducing weight include increasing metabolic activity to burn calories and/or reducing caloric intake, either by modifying behavior or with pharmacological intervention to reduce the desire to eat. Other methods include surgery to reduce the stomach's volume, banding to limit the size of the stoma, and intragastric devices that reduce the desire to eat by occupying space in the stomach.

Intragastric volume-occupying devices provide the patient a feeling of satiety after having eaten only small amounts of food. Thus, the caloric intake is diminished while the person is satisfied with a feeling of fullness. Currently available volume-occupying devices have many shortcomings. For example, complex gastric procedures are required to insert some devices.

U.S. Pat. No. 4,133,315, the contents of which are incorporated herein by reference in their entirety, discloses an apparatus for reducing obesity comprising an inflatable, elastomeric bag and tube combination. The bag can be inserted into the patient's stomach 20 by swallowing. The end of the attached tube distal to the bag remains in the patient's mouth. A second tube is snaked through the nasal cavity and into the patient's mouth. The tube ends located in the patient's mouth are connected to form a continuous tube for fluid communication through the patient's nose to the bag. Alternatively, the bag can be implanted by a gastric procedure. The bag is inflated through the tube to a desired degree before the patient eats so that the desire for food is reduced. After the patient has eaten, the bag is deflated. The tube extends out of the patient's nose or abdominal cavity throughout the course of treatment.

U.S. Pat. Nos. 5,259,399, 5,234,454 and 6,454,785, the contents of which are incorporated herein by reference in their entirety, disclose intragastric volume-occupying devices for weight control that must be implanted surgically.

U.S. Pat. Nos. 4,416,267, 4,485,805, 4,607,618, 4,694,827, 4,723,547, 4,739,758, and 4,899,747 and European Patent No. 246,999, the contents of which are incorporated herein by reference in their entirety, relate to intragastric, volume-occupying devices for weight control that can be inserted endoscopically. Of these, U.S. Pat. Nos. 4,416,267, 4,694,827, 4,739,758 and 4,899,747, the contents of which are incorporated herein by reference in their entirety relate to balloons whose surface is contoured in a certain way to achieve a desired end. In U.S. Pat. Nos. 4,416,267 and 4,694,827, the contents of which are incorporated herein by reference in their entirety, the balloon is torus-shaped with a flared central opening to facilitate passage of solids and liquids through the stomach cavity. The balloon of U.S. Pat. No. 4,694,827, the contents of which are incorporated herein by reference in their entirety, has a plurality of smooth-surfaced convex protrusions. The protrusions reduce the amount of surface area which contacts the stomach wall, thereby reducing the deleterious effects resulting from excessive contact with the gastric mucosa. The protrusions also define channels between the balloon and stomach wall through which solids and liquids may pass. The balloon of U.S. Pat. No. 4,739,758, the contents of which are incorporated herein by reference in their entirety, has blisters on its periphery that prevent it from seating tightly against the cardia or pylorus.

The balloons of U.S. Pat. Nos. 4,899,747 and 4,694,827, the contents of which are incorporated herein by reference in their entirety, are inserted by pushing the deflated balloon and releasably attached tubing down a gastric tube. U.S. Pat. No. 4,723,547, the contents of which are incorporated herein by reference in their entirety discloses a specially adapted insertion catheter for positioning its balloon. In U.S. Pat. No. 4,739,758, the contents of which are incorporated herein by reference in their entirety, the filler tube effects insertion of the balloon. In U.S. Pat. No. 4,485,805, the contents of which are incorporated herein by reference in their entirety, the balloon is inserted into a finger cot that is attached by string to the end of a conventional gastric tube that is inserted down the patient's throat. The balloon of European Patent No. 246,999 is inserted using a gastroscope with integral forceps.

In U.S. Pat. Nos. 4,416,267, 4,485,805, 4,694,827, 4,739,758, and 4,899,747 and European Patent No. 246,999, the contents of which are incorporated herein by reference in their entirety, the balloon is inflated with a fluid from a tube extending down from the patient's mouth. In these patents, the balloon also is provided with a self-sealing hole (U.S. Pat. No. 4,694,827, the contents of which are incorporated herein by reference in their entirety), injection site (U.S. Pat. Nos. 4,416,267 and 4,899,747, the contents of which are incorporated herein by reference in their entirety), self-sealing fill valve (U.S. Pat. No. 4,485,805, the contents of which are incorporated herein by reference in their entirety), self-closing valve (European Patent No. 246,999, the contents of which are incorporated herein by reference in their entirety) or duck-billed valve (U.S. Pat. No. 4,739,758, the contents of which are incorporated herein by reference in their entirety). U.S. Pat. No. 4,723,547, the contents of which are incorporated herein by reference in their entirety, uses an elongated thick plug and the balloon is filled by inserting a needle attached to an air source through the plug.

U.S. Pat. No. 4,607,618, the contents of which are incorporated herein by reference in their entirety, describes a collapsible appliance formed of semi-rigid skeleton members joined to form a collapsible hollow structure. The appliance is not inflatable. It is endoscopically inserted into the stomach 20 using an especially adapted bougie having an ejector rod to release the collapsed appliance. Once released, the appliance returns to its greater relaxed size and shape.

U.S. Pat. No. 5,129,915, the contents of which are incorporated herein by reference in their entirety, the contents of which are incorporated herein by reference, relates to an intragastric balloon that is intended to be swallowed and that inflates automatically under the effect of temperature. Three ways that an intragastric balloon might be inflated by a change in temperature are discussed. A composition comprising a solid acid and non-toxic carbonate or bicarbonate is separated from water by a coating of chocolate, cocoa paste or cocoa butter that melts at body temperature. Alternatively, citric acid and an alkaline bicarbonate coated with non-toxic vegetable or animal fat melting at body temperature and which placed in the presence of water, can produce the same result. Lastly, the solid acid and non-toxic carbonate or bicarbonate are isolated from water by an isolation pouch of low-strength synthetic material which it will suffice to break immediately before swallowing the bladder. Breaking the isolation pouches causes the acid, carbonate or bicarbonate and water to mix and the balloon to begin to expand immediately. A drawback of thermal triggering of inflation is that it does not afford the degree of control and reproducibility of the timing of inflation that is desirable and necessary in a safe self-inflating intragastric balloon.

### SUMMARY OF THE INVENTION

A free-floating, intragastric, volume-occupying device that can be inserted into the stomach by the patient swallowing it and letting peristalsis deliver it into the stomach in the same manner that food is delivered, or by positioning it with a catheter, is desirable.

Volume-occupying devices and methods for manufacturing, deploying, inflating, tracking, deflating and retrieving of such devices are provided. The devices and methods of the embodiments may be employed for treating over weight and obese individuals. Methods employing the device of the embodiments may be swallowed by a patient, with or without a catheter attached. Once in the stomach of the patient, the device is inflated with a preselected gas or mixture of gases, to a preselected volume. After a predetermined time period, the device can be removed using endoscopic tools or decreases in volume or deflate so as to pass through the remainder of the patient's digestive tract.

Inflation may be achieved by use of a removable catheter that initially remains in fluid contact with the device after it has been swallowed by the patient.

In a first aspect, an intragastric balloon system is provided that includes a volume-occupying component, a self-sealing valve system, and a deflation subcomponent. The volume-occupying component includes a polymeric wall that has an outer surface and an inner surface. The inner surface of the polymeric wall defines a central lumen of the volume-occupying component. The self-sealing valve system is attached to the inner surface of the polymeric wall and includes a head structure with a rear orifice, a retaining structure that is housed within the head structure, and a self-sealing septum. The retaining structure includes a smaller inner cylinder that is concentrically housed with a larger outer cylinder. The self-sealing septum is housed within the smaller inner cylinder and is configured for piercing by a needle of an inflation catheter. The deflation subcomponent is configured to deflate the intragastric balloon system after the passage or lapse of a preselected period of time *in vivo.* The deflation subcomponent has a vent member and a releasable sealing member. The vent member includes a fluid pathway that fluidly joins the central lumen of the volume-occupying component with the wall outer surface. The fluid pathway has two configurations, an open configuration and a sealed configuration. The releasable sealing member cooperates with the vent member to bias the fluid pathway in the sealed configuration. Upon release of the sealing member, the vent member transitions the fluid pathway from the sealed configuration to the open configuration. When the fluid pathway is in the sealed configuration, a gas-tight seal is provided between the central lumen and the wall outer surface, so as to block the flow of inflation fluid through the fluid pathway. Conversely, when the fluid pathway is in the open configuration, the sealing member is released, thereby enabling the flow of the inflation fluid through the fluid pathway, out of the central lumen, thereby deflating the volume-occupying component.

In a first embodiment of the first aspect, the vent member includes a cylindrical channel that extends from the head rear orifice toward the wall inner surface. A portion of the outer cylinder is received into the cylindrical channel; and the thickness of the vent member is greater than the thickness of the outer cylinder of the self-sealing valve system. In a further embodiment of the first aspect, the releasable sealing member biases the cylindrical channel against the larger outer cylinder, so as to seal the pathway. In another further embodiment of the first aspect, the releasable sealing member is released, the fluid pathway is open.

In a second embodiment of the first aspect, the releasable sealing member includes a biodegradable or dissolvable material that is configured to, upon degradation, allow inflation fluid to escape from the central lumen. In a further embodiment, a first portion of the releasable sealing member is configured to degrade faster than a second portion of the releasable sealing member. In a still further embodiment, the first portion of the releasable sealing member is configured to stabilize the second portion of the releasable sealing member such that, when the first portion of the releasable sealing member degrades, the second portion of the releasable sealing member destabilizes and is released from the deflation subcomponent, thereby accelerating a process of deflation of the volume occupying subcomponent

In a third embodiment of the first aspect, the retaining structure is configured to be dislodged, upon release of the releasable sealing member. Dislodging the retaining structure opens a larger deflation channel that fluidly joins the central lumen with the wall outer surface. When the larger deflation channel is opened, the process of deflating of the volume occupying subcomponent is accelerated.

In a fourth embodiment of the first aspect, the releasable sealing member includes a tension member secured around an outer side surface of the head so as to maintain the vent member in a closed configuration. The tension member includes a dissolvable or degradable material. Upon exposure to conditions within the central lumen of the volume-occupying subcomponent for a predetermined period of time, the dissolvable or degradable material of the tension member dissolves, thereby releasing the sealing member.

In a fifth embodiment of the first aspect, the predetermined period of time is at least 60 days.

In a sixth embodiment of the first aspect, the predetermined period of time is at least six months.

In a seventh embodiment of the first aspect, the sealing properties of the deflation subcomponent is substantially unaffected by exposure to the gastric environment or by mechanical forces during processing, compacting, or application of external gastric pressures.

In an eighth embodiment of the first aspect, the intragastric balloon system is retains more than 75% of an original nominal volume in the gastric environment for at least 60 days.

In a ninth embodiment of the first aspect, the intragastric balloon system further includes a swallowable balloon outer container; an inflation catheter with a bell-shaped needle sleeve for releasably engaging the self-sealing valve system; and an inflation system with inflation fluid for inflating the volume-occupying component.

In a tenth embodiment of the first aspect, the intragastric balloon system is swallowable by normal peristaltic motion.

In an eleventh embodiment of the first aspect, the fluid pathway includes a through-bore that extends longitudinally through the head structure so as to fluidly join the central lumen with the wall outer surface. In a further embodiment, the releasable sealing member includes a tension member that biases the through-bore in the sealed configuration. The tension element includes a dissolvable or degradable subcomponent, and degradation of the dissolvable or degradable subcomponent enables escape of the inflation fluid from the central lumen. In a still further embodiment, the tension member includes at least one of a spring, a tension band, a suture, a cap and a lock. In another still further embodiment, the dissolvable or degradable subcomponent degrades after a pre-determined period of time. In some embodiments, the pre-determined period of time is at least 60-days. In other embodiments, the pre-determined period of time is at least six months. In another further embodiment, when the releasable sealing member is released, the through-bore is open.

In a twelfth embodiment of the first aspect, the self-sealing valve system includes the deflation subcomponent.

In a thirteenth embodiment of the first aspect, the intragastric balloon system further includes at least one additional deflation subcomponent.

In a fourteenth embodiment of the first aspect, the fluid pathway is elastically deformable.

In a second aspect, a method of deflating an intragastric balloon system is provided. The an intragastric balloon system includes a volume-occupying component with an outer surface and a central lumen, and a deflation subcomponent configured to deflate the intragastric balloon system after a preselected period of time has lapsed since deployment of the system *in vivo.* The method includes the steps of releasing a releasable sealing member from the deflation subcomponent after a pre-determined period of time; transitioning a fluid pathway from a sealed configuration to an open configuration, whereby the central lumen is fluidly joined with the outer surface of the volume-occupying component; and then allowing inflation fluid within the central lumen to flow through the fluid pathway and out of the volume-occupying subcomponent, whereby the volume-occupying component is deflated.

In a first embodiment of the second aspect, releasing a releasable sealing member includes degrading a dissolvable or biodegradable portion of the releasable sealing member. In a further embodiment, degrading a dissolvable or biodegradable portion includes degrading a first portion of the releasable sealing member faster than a second portion of the releasable sealing member. In a still further embodiment, the method includes destabilizing the second portion of the releasable sealing member, thereby accelerating a process of deflation of the volume occupying subcomponent.

In a second embodiment of the second aspect, releasing the releasable sealing member includes allowing at least a portion of the deflation subcomponent to fall into or out of the volume-occupying device.

In a third embodiment of the second aspect, releasing a releasable sealing member comprises releasing a tension member.

In a fourth embodiment of the second aspect, when in the sealed configuration, the fluid pathway is elastically deformed. In a further embodiment, transitioning the fluid pathway from the sealed configuration to the open configuration comprises allowing recovery of the fluid pathway from the elastic deformation.

In a fifth embodiment of the second aspect, upon release of the releasable sealing member, the retaining structure is dislodged from the head structure, such that a larger deflation channel is opened, thereby fluidly joining the central lumen with the wall outer surface. In a further embodiment, dislodging the retaining structure from the head structure includes allowing the retaining structure to fall into the central lumen.

In a sixth embodiment of the second aspect, releasing the releasable sealing member includes the step of releasing a tension member secured around an outer side surface of the head. In a further embodiment, the dissolvable or degradable material is exposed to conditions within the central lumen of the volume-occupying subcomponent for a predetermined period of time. In some embodiments, the predetermined period of time is at least 60 days. In some other embodiments, the predetermined period of time is at least six months.

In a sixth embodiment of the second aspect, the intragastric balloon system retains more than 75% of an original nominal volume of the volume-occupying component while in the gastric environment for at least 60 days.

In a seventh embodiment of the second aspect, the patient swallows the intragastric balloon system by normal peristaltic motion.

In an eighth embodiment of the second aspect, transitioning a fluid pathway includes the step of opening a through-bore extending longitudinally through the head structure and thereby fluidly joining the central lumen with the wall outer surface.

In a ninth embodiment of the second aspect, releasing the releasable sealing member includes releasing the tension member, which is configured to bias the through-bore in the sealed configuration. In a further embodiment, releasing the tension member includes a dissolving a dissolvable or degradable subcomponent, and thereby enabling escape of the inflation fluid from the central lumen. In some further embodiments, a pre-determined period of time is allowed to pass before the dissolvable or degradable subcomponent dissolves. In some embodiments, the pre-determined period of time is at least 60-days. In some other embodiments, the pre-determined period of time is at least six months.

A third aspect provides an intragastric balloon system with a volume-occupying subcomponent and a deflation subcomponent. The volume-occupying subcomponent includes an outer surface, and an inner surface that defines a central lumen of the volume-occupying component. The deflation subcomponent is attached to the inner surface of the volume-occupying subcomponent, and is configured to deflate the volume-occupying subcomponent after a preselected period of time has lapsed since deployment of the system *in vivo.* The deflation subcomponent includes a vent member and a releasable sealing member. The vent member includes a fluid pathway that fluidly joins the central lumen with the outer surface of the volume-occupying subcomponent. The fluid pathway is configured to transition between a sealed configuration and an open configuration. The releasable sealing member cooperates with the vent member so as to bias the fluid pathway in the sealed configuration. Upon release of the sealing member, the fluid pathway transitions from the sealed configuration to the open configuration.

In a first embodiment of the third aspect, when the fluid pathway is in the sealed configuration, a gas-tight seal is provided between the central lumen and the outer surface of the volume-occupying subcomponent, whereby a flow of an inflation fluid through the fluid pathway is substantially blocked.

In a second embodiment of the third aspect, when the fluid pathway is in the open configuration, the sealing member is released, thereby enabling a flow of the inflation fluid through the fluid pathway, whereby the volume-occupying component is deflated.

In a third embodiment of the third aspect, the preselected period of time is at least 60-days.

In a fourth embodiment of the third aspect, the preselected period of time is at least six months.

In a fifth embodiment of the third aspect, the deflation subcomponent includes a polymeric head that coaxially houses a cylindrical retaining structure. In a further embodiment, the retaining structure also includes a smaller inner cylinder concentrically housed with a larger outer cylinder. In another further embodiment, the releasable sealing member has a biodegradable or dissolvable material. When the material degrades, inflation fluid escapes from the central lumen. In still another further embodiment, a first portion of the releasable sealing member degrades at a faster rate than a second portion of the releasable sealing member. In some embodiments, the first portion of the releasable sealing member stabilizes the second portion of the releasable sealing member. Accordingly, when the first portion of the releasable sealing member degrades, the second portion of the releasable sealing member is destabilized and is thereby released from the deflation subcomponent. Consequently, deflation of the volume occupying subcomponent is accelerated.

In a sixth embodiment of the third aspect, the vent structure also includes a head structure and a retaining structure. Upon release of the releasable sealing member, the retaining structure is dislodged from the head structure, thereby opening a larger deflation channel that fluidly joins the central lumen of the volume-occupying component with the wall outer surface, and thereby accelerating a process of deflation of the volume-occupying component.

In a seventh embodiment of the third aspect, the vent structure also includes a head structure and a retaining structure. The releasable sealing member includes a tension member that is secured around an outer side surface of the head. Securing the tension member around the outer side surface of the head maintains the vent member in a closed configuration. The tension member includes a dissolvable or degradable material that dissolves or degrades when it is exposed to conditions within the central lumen of the volume-occupying subcomponent for a predetermined period of time.

In an eighth embodiment of the third aspect, the predetermined period of time is at least 60 days.

In a ninth embodiment of the third aspect, the predetermined period of time is at least six months.

In a tenth embodiment of the third aspect, the sealing properties of the deflation subcomponent are not substantially affected by exposure to the gastric environment or by mechanical forces during processing, compacting, or application of external gastric pressures.

In an eleventh embodiment of the third aspect, the intragastric balloon system is retains more than 75% of its original nominal volume in the gastric environment for at least 60 days.

In a twelfth embodiment of the third aspect, the intragastric balloon system also includes a swallowable balloon outer container, an inflation catheter with a bell-shaped needle sleeve configured to releasably engage the self-sealing valve system, and an inflation system including an inflation fluid for inflating the volume-occupying component.

In a thirteenth embodiment of the third aspect, the intragastric balloon system is swallowable by normal peristaltic motion.

In a fourteenth embodiment of the third aspect, the fluid pathway includes a through-bore that extends longitudinally through the head structure and fluidly joins the central lumen with the wall outer surface. In a further embodiment, the releasable sealing member includes a tension member that biases the through-bore in the sealed configuration. The tension element includes a dissolvable or degradable subcomponent, and degradation of the dissolvable or degradable subcomponent enables escape of the inflation fluid from the central lumen. In some further embodiments, the tension member includes at least one of a spring, a tension band, a suture, a cap and a lock. In some other further embodiments, the dissolvable or degradable subcomponent degrades after a pre-determined period of time. In some embodiments, the pre-determined period of time is at least 60-days. In other embodiments, the pre-determined period of time is at least six months. In still other embodiments, when the releasable sealing member is released, the through-bore is open.

In a fifteenth embodiment of the third aspect, the self-sealing valve system comprises the deflation subcomponent.

In a sixteenth embodiment of the third aspect, the intragastric balloon system also includes at least one additional deflation subcomponent.

In a seventeenth embodiment of the third aspect, the fluid pathway is elastically deformable.

In an eighteenth embodiment of the third aspect, the intragastric balloon system also includes a self-sealing valve system that is attached to the wall inner surface of the volume-occupying subcomponent. The self-sealing valve system includes a head structure with a rear orifice, a retaining structure that is housed within the head structure and includes a smaller inner cylinder concentrically housed with a larger outer cylinder, and a self-sealing septum that is configured for piercing by a needle of an inflation catheter and is housed within the smaller inner cylinder.

### BRIEF DESCRIPTION OF THE DRAWINGS

FIG. 1 depicts two intragastric balloons 10 placed within a patient's stomach 20.
FIG. 2 depicts an intragastric balloon 10, including a polymeric film wall 15 defining a central lumen and a self-sealing balloon valve system 100.
FIG. 3 depicts an Obalon Gastric Balloon Assembly 30, including a balloon outer container 40 ("capsule") containing an intragastric balloon of FIG. 2 (not shown) and an inflation catheter 200 coupled to the balloon within the capsule 40. The inflation catheter 200 includes tubing 210 and a bell-shaped needle sleeve 220.
FIG. 4 depicts an exemplary embodiment of a proximal end 230 of a catheter luer connector 240 with an over-molded sleeve 250 joined with the luer connector 240 and the tubing 210.
FIGS. 5 depicts a perspective view of a head assembly of a self-sealing balloon valve system 100, with a longitudinal axis A, a metallic retaining structure 105 (or metallic concentric cylinder or tube septum), a polymeric head 110 with a lip 111, and a self-sealing septum (plug) 114.
FIG. 6 depicts a top view of the self-sealing balloon valve system 100 of FIG. 5.

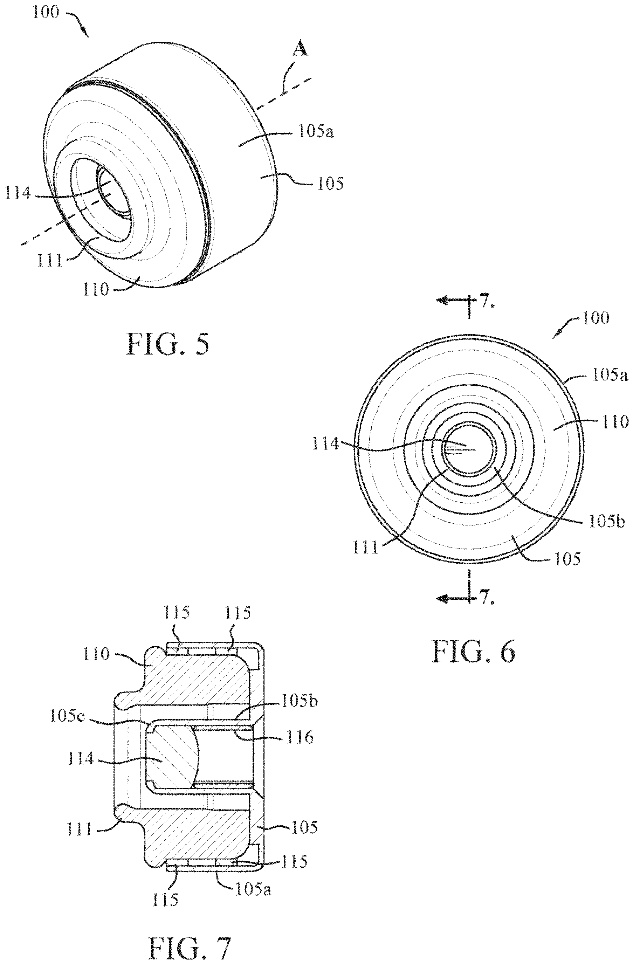

FIG. 7 is a cross-sectional view of the self-sealing balloon valve system 100 of FIG. 5, with the cross-section being taken along line 7-7 of FIG. 6. The retaining structure 105 includes tube septum 105, which includes a portion that is a larger outer cylinder 105a and a portion that is a smaller inner cylinder 105b, the inner cylinder 105b housing the septum 114 and the larger outer cylinder 105a housing the head unit 110, which is made of a material configured to provide compressive forces against a bell-shaped needle sleeve (not depicted). Retaining rings 115 compress the head unit 110 against the inner cylinder 105b. The inner cylinder 105b of the tube septum 105 includes a lip 105c configured for an interference lip with a bell-shaped needle sleeve (not depicted). The entire outer cylinder 105a is filled with a material forming the head unit 110, and the small circular lip 111 of this same material is provided, which is slightly larger than the diameter of the inner cylinder 105b and extends to the outside surface of the balloon (not depicted). A ring-stop 116 compresses the self-sealing septum against the lip 105c of the inner cylinder 105b.
FIG. 8 a perspective cross-sectional view of the distal end of the catheter 200 of FIG. 3, in an exemplary embodiment, including tubing 210 with a single lumen 230 attached to a bell-shaped needle sleeve 220. The needle sleeve 220 houses a hollow needle 230. The tubing includes a tensile cord 240 joined with the hollow needle 230 and acting as a structural member providing increased tensile strength to the catheter 200.
FIG. 9 shows a perspective cross-sectional view of the single lumen catheter 200 showing additional detail of the needle 230 and bell-shaped needle sleeve 220 when seated in the head unit 110 of the self-sealing valve system 100 of FIGS 5-7.
FIG. 10 is a top perspective view of a self-sealing valve system 400 with longitudinal axis B, in an exemplary embodiment.
FIG. 11 is a half-section of the self-sealing valve system 400 of FIG. 10 wherein the section is taken along lines 11-11 of FIG. 10.
FIG. 12 is a cross-section of the self-sealing valve system 400 FIG. 10, with the cross-section being taken along line 12-12 of FIG. 10.
FIG. 13 illustrates fluid connection between a bell-shaped needle sleeve 220 of FIG. 8 and the self-sealing valve system 400 of FIG. 10.

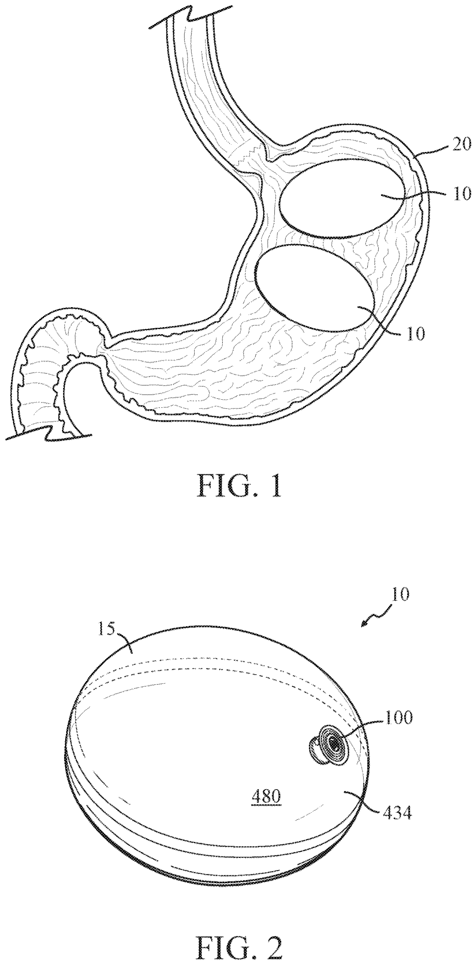

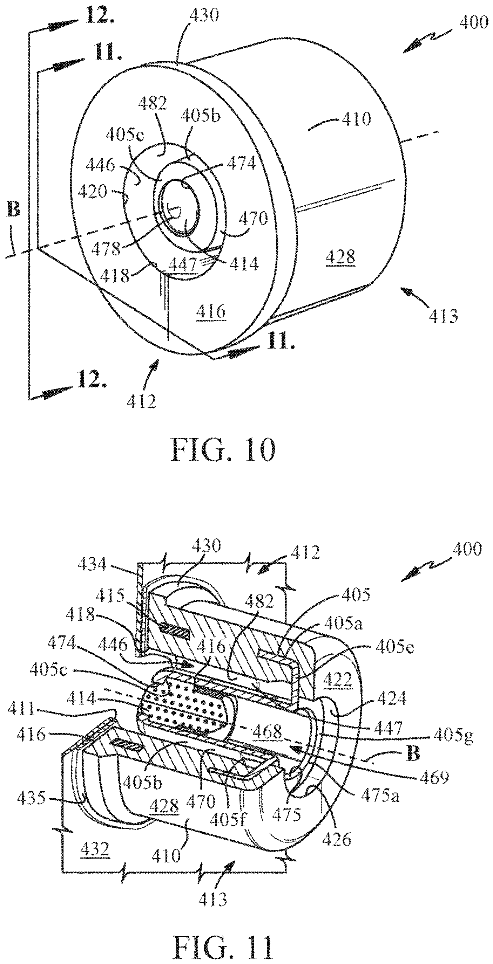

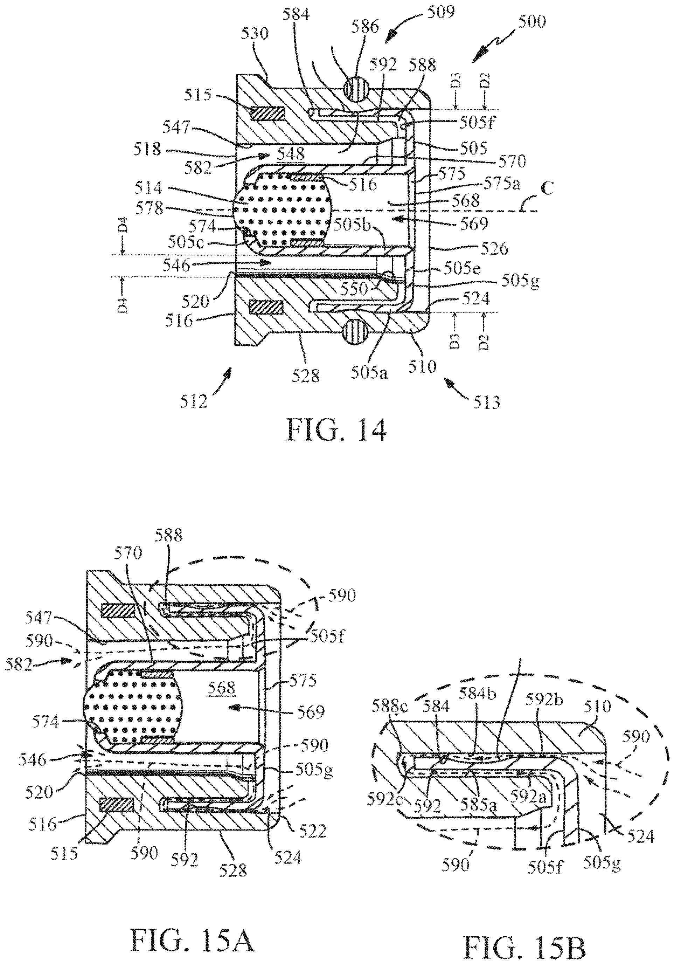

FIG. 14 is a cross-section of a self-sealing valve system 500, in an exemplary embodiment, the self-sealing valve system including a longitudinal axis C, a head unit 510 and an integrated deflation component 511, wherein the cross-section has been taken along a plane parallel and intersecting with the longitudinal axis C, similar to the line 12-12 of FIG. 10, and wherein the deflation component 511 is in an engaged configuration, the engaged configuration being adapted to maintain an intragastric balloon, such as the intragastric balloon of FIG. 2, in an inflated state for a period of time, such as but not limited to a treatment period.
FIGS. 15A and B illustrate the self-sealing valve system 500 of FIG. 14, with the deflation component 511 is in a released configuration, wherein the released configuration is adapted to allow release of inflation fluid from the intragastric device such that the intragastric balloon deflates, and wherein the deflation component 511 transitions from the engaged configuration of FIG. 14 to the released configuration of FIGS. 15A and B at about the end of the treatment period or upon activation of the transition.
FIG. 15A illustrates the self-sealing valve system 500 of FIG. 14 in another exemplary embodiment.
FIG. 15B illustrates an enlarged portion of the self-sealing valve system 500 of FIG. 15A, showing greater detail thereof.

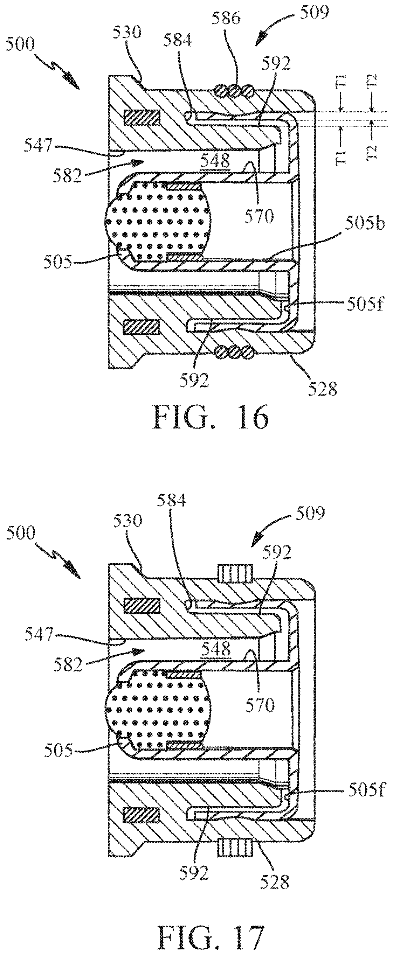

FIG. 16 illustrates the self-sealing valve system 500 of FIG. 14 in yet another exemplary embodiment.
FIG. 17 illustrates the self-sealing valve system 500 of FIG. 14 in a further embodiment.

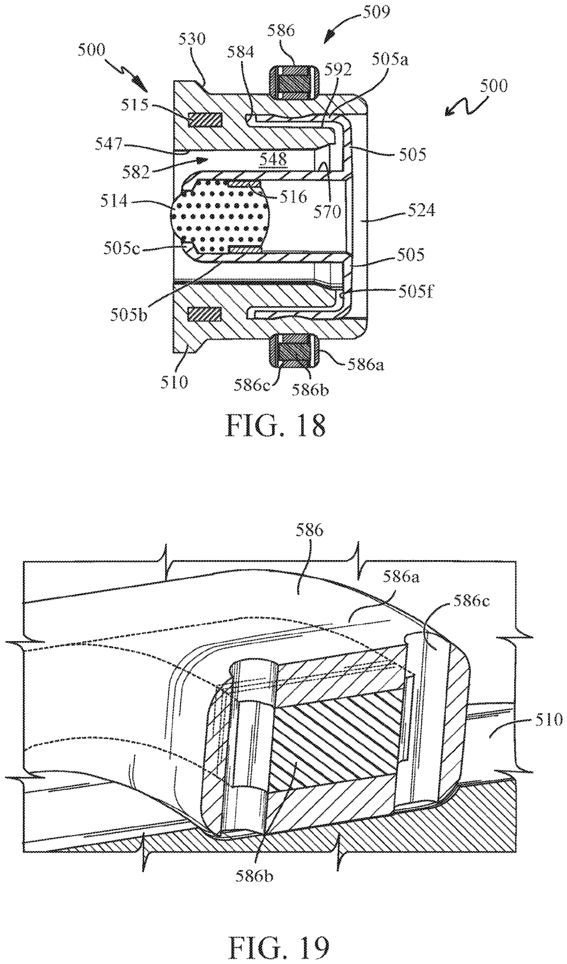

FIG. 18 17 illustrates the self-sealing valve system 500 of FIG. 14 in a further embodiment.
FIG. 19 is an enlarged view of a portion of the self-sealing valve system 500 of FIG. 18.

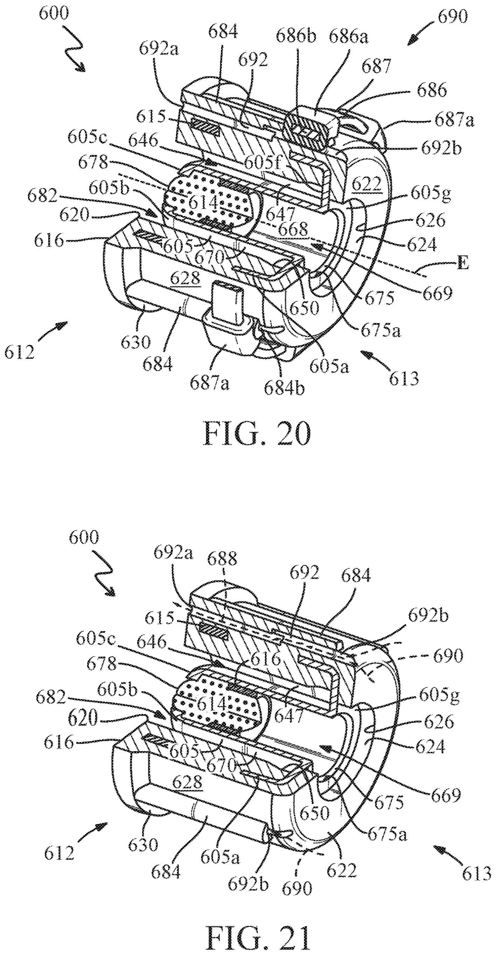

FIG. 20 is a perspective half-section view of another exemplary embodiment of a self-sealing valve system 600 with a longitudinal axis D and a deflation component 611 in an engaged configuration.
FIG. 21 illustrates the self-sealing valve system 600 of FIG. 20 in a released configuration.

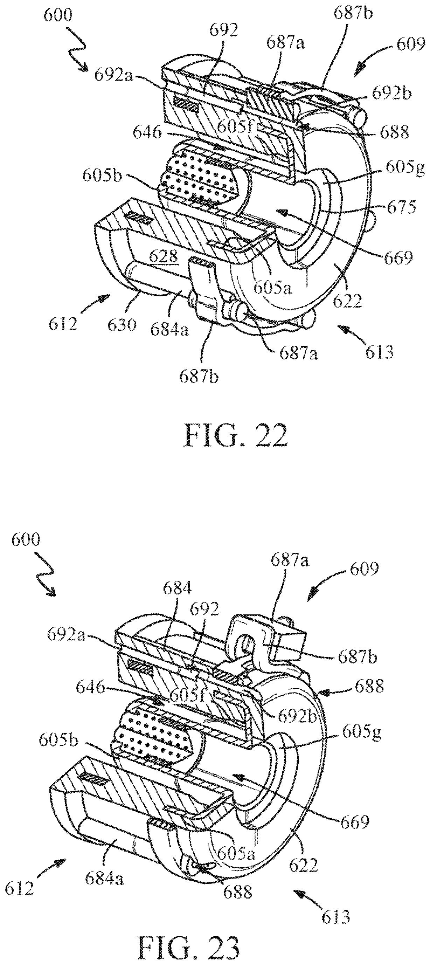

FIG. 22 illustrates the self-sealing valve system 600 of FIG. 20 in another exemplary embodiment.
FIG. 23 illustrates the self-sealing valve system 600 of FIG. 20 in yet another exemplary embodiment.

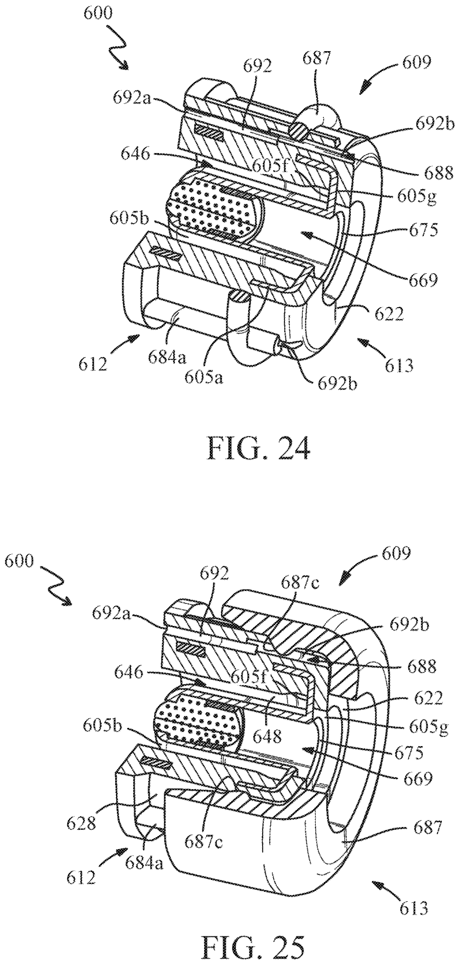

FIG. 24 illustrates the self-sealing valve system 600 of FIG. 20 in still another exemplary embodiment.
FIG. 25 illustrates the self-sealing valve system 600 of FIG. 20 in another exemplary embodiment.

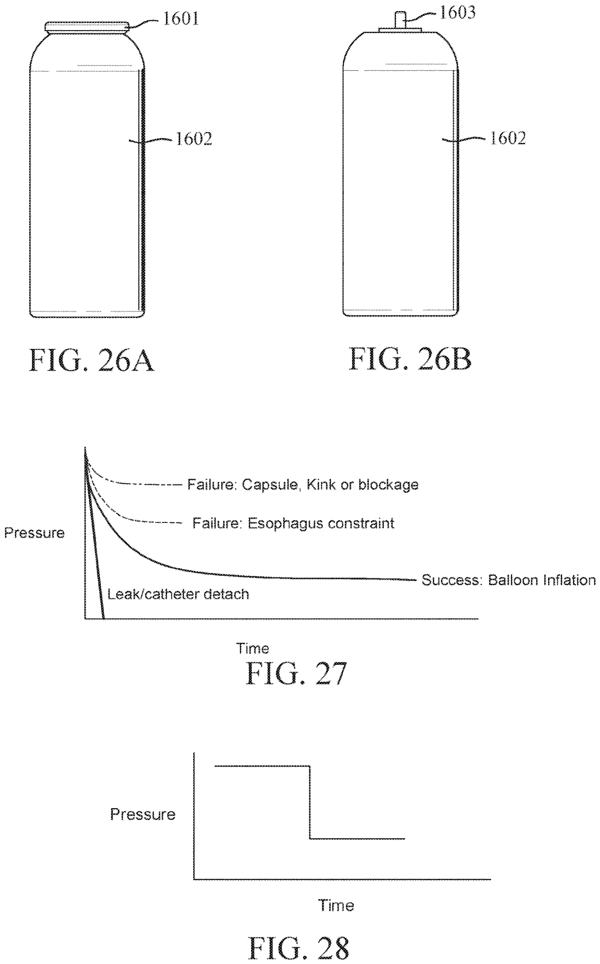

FIGS. 26A and 26B depict a disposable inflation fluid container 1602 with a cap 1601 and without a cap with a portion of a tube 1603 that extends to the bottom of the can exposed (FIG. 26A and FIG. 26B, respectively).
FIG. 27 is a graph depicting pressure as a function of time (pressure decay), obtained from feedback from an inflation source container.
FIG. 28 depicts the expected decay curve for pressure sources using a spring mechanism or a balloon-within-balloon mechanism.

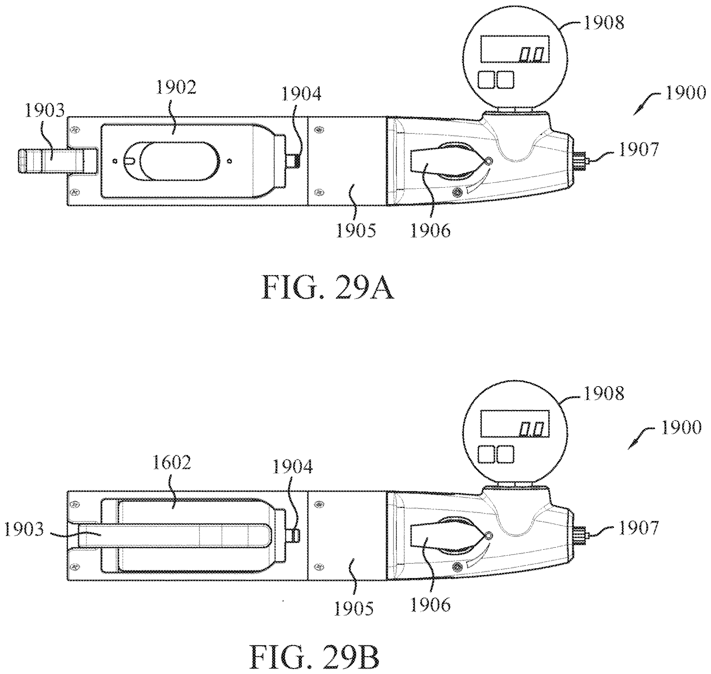

FIGS. 29A-29B depict an inflation fluid dispenser 1900 in isolation (FIG. 29A) and in connection with an inflation fluid container or canister 1602 (FIG. 29B). The inflation fluid dispenser ('dispenser') a securement latch 1901, a receiving space 1902 fitted to receive an inflation fluid container 1602, a spring 1904, a housing 1905 configured to couple to a canister, a lever 1906 for actuating a valve inflation fluid flow within the dispenser 1900, a port 1907, and a pressure gauge 1908.

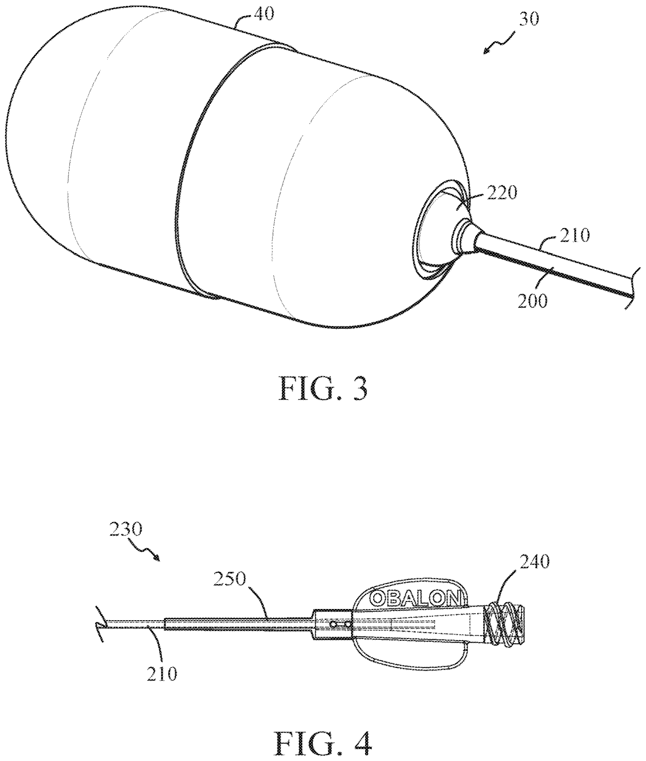

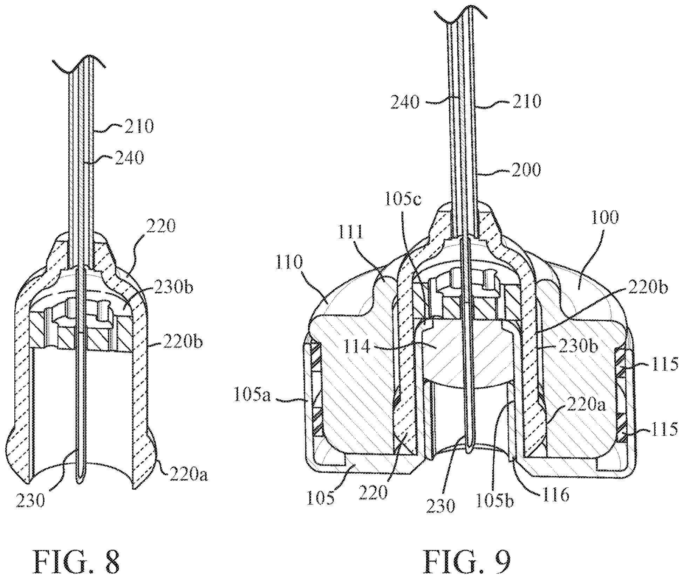

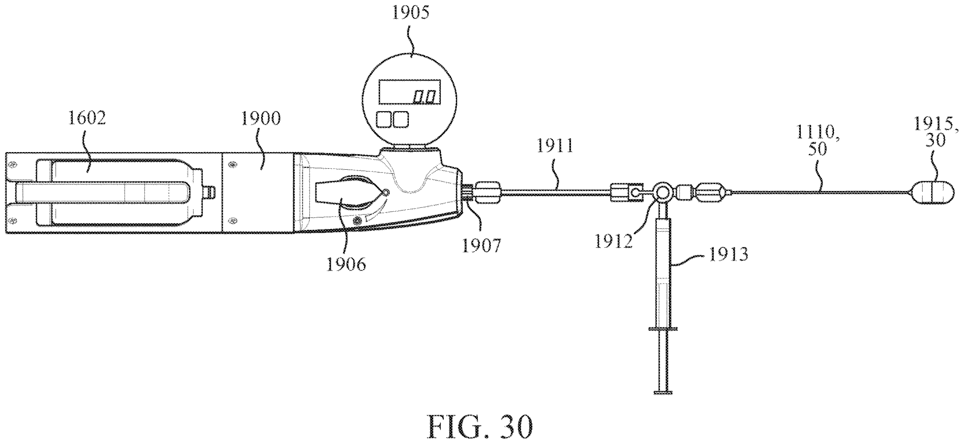

FIG. 30 depicts an inflation system for inflating a gastric balloon including an inflation fluid container 1602, an inflation fluid dispenser 1900, an extension tube 1911, a stopcock 1912 (`3-way valve'), a catheter 1110, 50 similar to those described with respect to FIGS. 2 and 9A-9C, an ejection syringe 1913, and a capsule encasing a balloon 1915, 30 similar to that described with respect to FIG. 3.

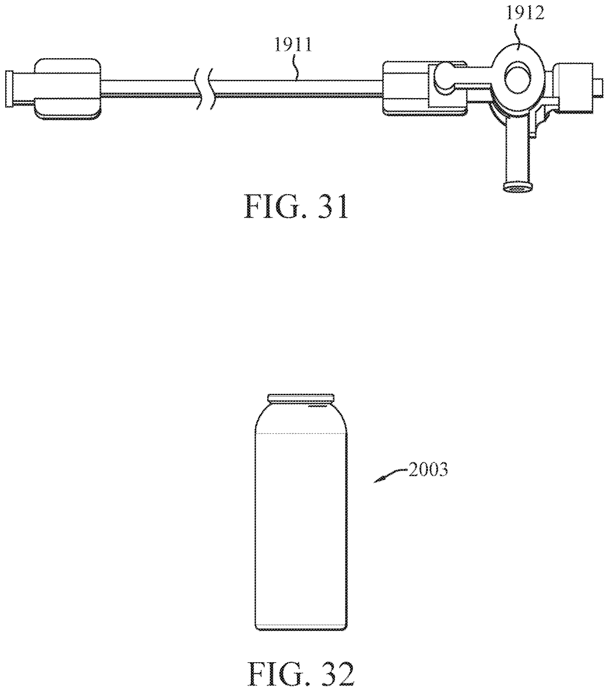

FIG. 31 depicts an extension tube 1911 and stopcock 1912 with a 3-way valve for use in conjunction with the Obalon Gastric Balloon Assembly of FIG. 3.

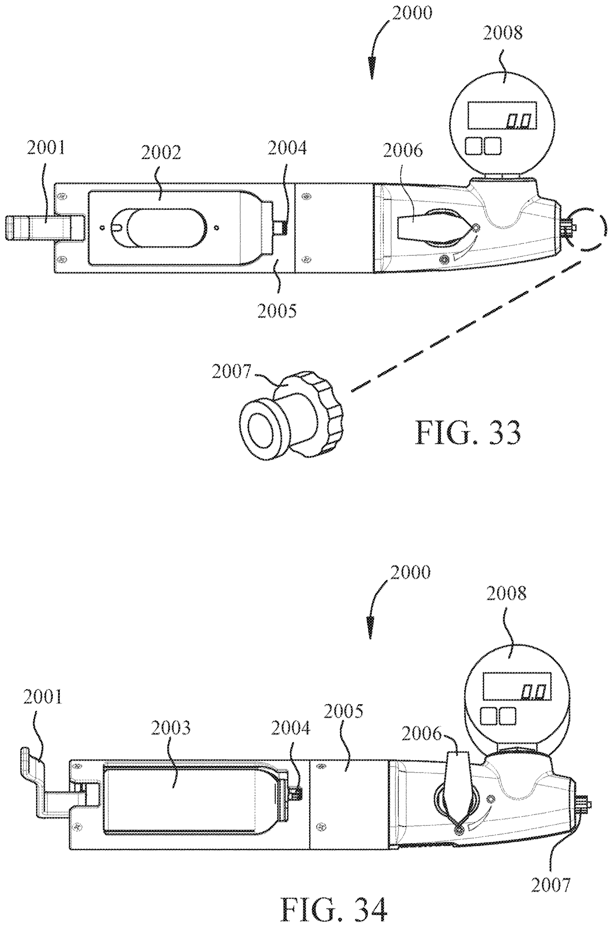

FIG. 32 depicts a disposable Procedure Canister ('can' or 'canister') 2003 for use with the Nitrogen Fill System ('dispenser') 2000 of FIG. 33 for use in conjunction with the Obalon Gastric Balloon Assembly 30 of FIG. 3.
FIG. 33 depicts a Nitrogen Fill System 2000 (or "dispenser," for use in conjunction with the Obalon Gastric Balloon Assembly 30 of FIG. 3) with a securement latch 2001, a receiving space 2002 fitted to receive the Procedure Canister 2003 of FIG. 32, a spring 2004, a housing 2005 configured to couple to a canister 2003, a valve 2006, a port 2007 with a luer lock plug 2007 configured to cover the end of the gauge of the Nitrogen Fill System 2000 and the valve 2006 in the 'open' position, and a pressure gauge 2008, wherein the valve 2006 in the 'open' position.
FIG. 34 depicts the Nitrogen Fill System 2000 with lever 2001 in the 'open' position and valve 3600 in the 'closed' position in advance of receiving the disposable Procedure Canister 2003.

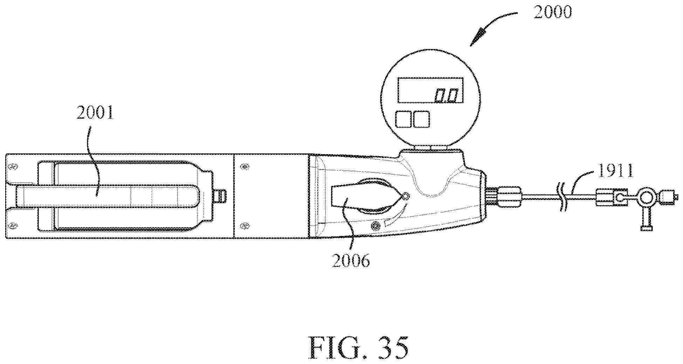

FIG. 35 depicts the Nitrogen Fill System 2000 of FIG. 34 with lever 2001 in the 'closed' position and valve 2006 in the 'open' position after receiving the disposable Procedure Canister 2003, and the Extension Tube 1911 attached to the port 2007.

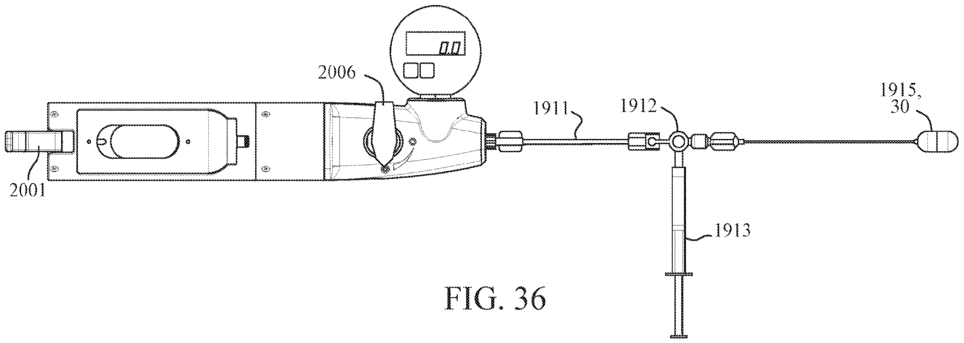

FIG. 36 depicts the Nitrogen Fill System 2000 with the valve 2006 in the 'closed' position, the Balloon Ejection Syringe 1913 and Obalon Gastric Balloon Assembly 1915, 30 attached to the Extension Tube 1911, and the 3-way valve 1912 (in the 'closed' position) attached to the catheter 1110, 50.

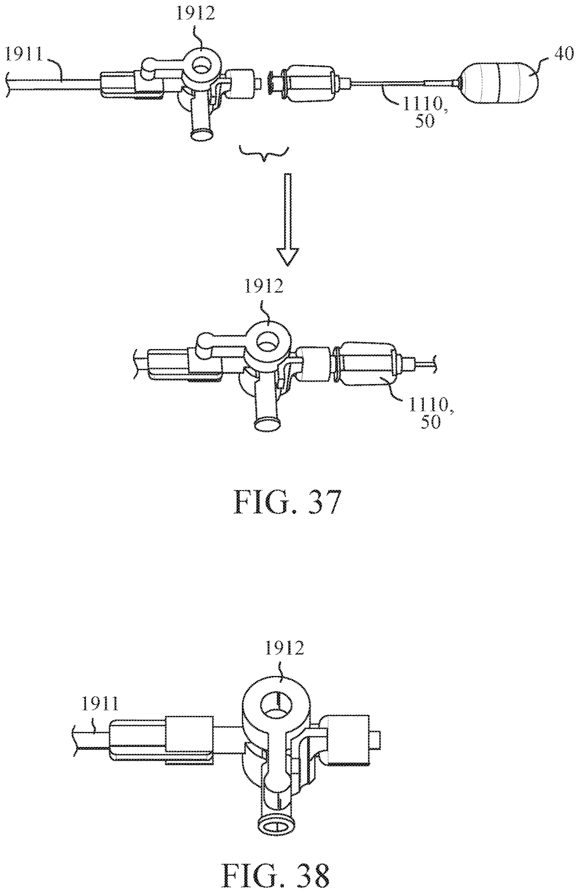

FIG. 37 depicts connection of the catheter 1110, 50 to the extension tube Extension Tube 1911 so as to attach the balloon (not shown) contained in the capsule 1915, 40 and with the 3-way valve in the 'closed' position.
FIG. 38 depicts detail of the stopcock of the Extension Tube 1911 with 3-way valve 1912 in the 'open' position.

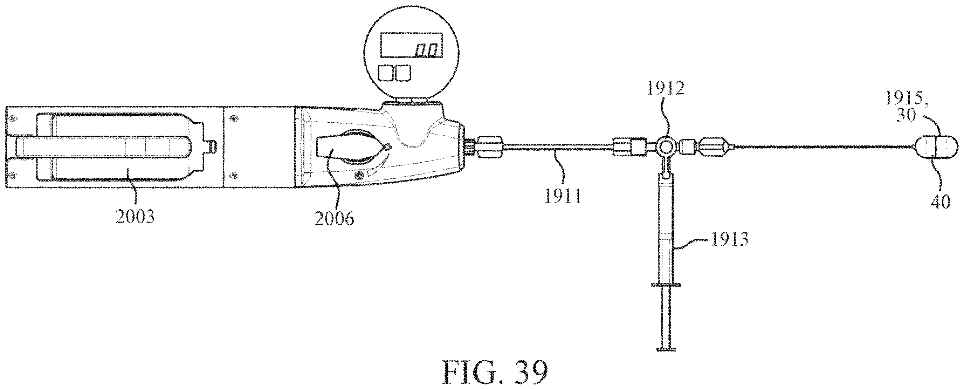

FIG. 39 depicts the Nitrogen Fill System with the valve 2006 in the 'open' position and the Balloon Ejection Syringe 1913 and Obalon Gastric Balloon Assembly 1915, 30 attached to the Extension Tube 1911 *via* the 3-way valve 1912, with the 3-way valve 1912 in the 'open' position, such that inflation fluid can flow from the canister 2003 to the balloon within the capsule 40.

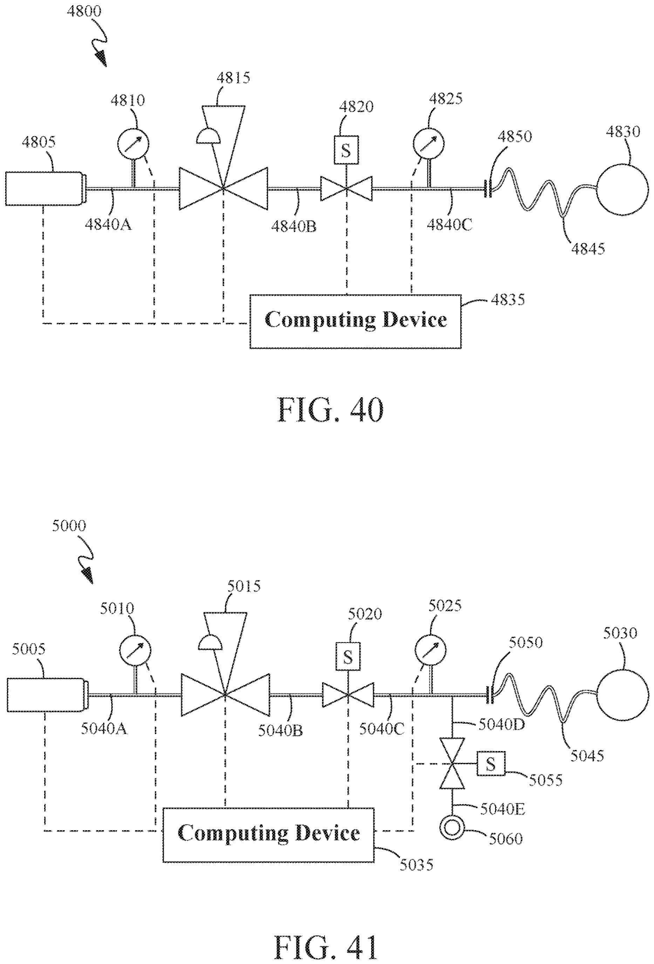

FIG. 40 is a schematic of an inflation system 4800 for inflating an intragastric balloon 10.
FIG. 41 is a schematic of another inflation system 5000 for inflating an intragastric balloon 10.

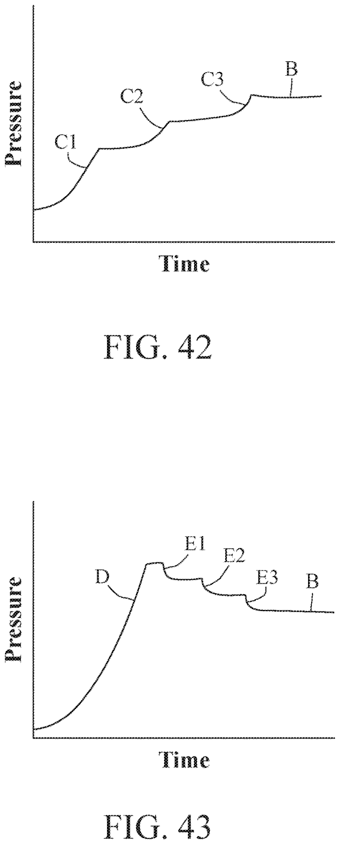

FIG. 42 depicts a graph illustrating the pressure in an intragastric balloon over time as the intragastric balloon is inflated in an exemplary operation of the inflation system 4800 of FIG. 40.
FIG. 43 depicts a graph illustrating the pressure in an intragastric balloon over time as the intragastric balloon is inflated in an exemplary operation of the inflation system 5000 of FIG. 41.

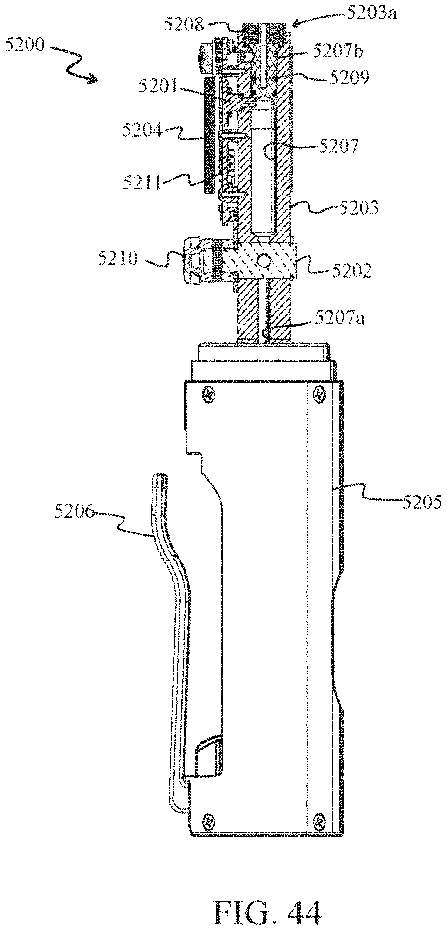

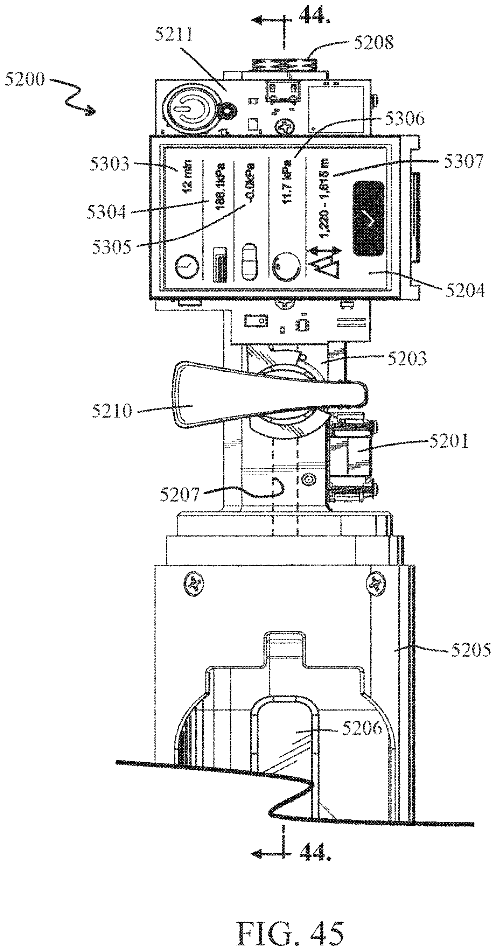

FIG. 44 is a side view of an exemplary embodiment of a dispenser 5200 for use with an inflatable intragastric device system 1915, 30, wherein portions of the dispenser are shown in cross-section, the cross-section being taken along line 44-44 of FIG. 45.
FIG. 45 is a front view of the dispenser 5200 of FIG. 44, with portions cut away, a touch screen housing 5201 not shown and a tunnel 5207 shown in phantom.

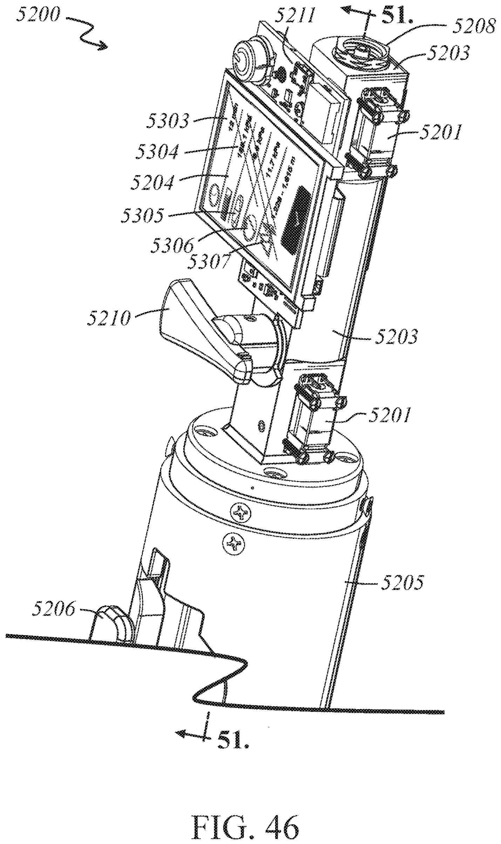

FIG. 46 is a front perspective view of the dispenser 5200 of FIG. 44, with portions broken away and the touch screen housing 5201 not shown.

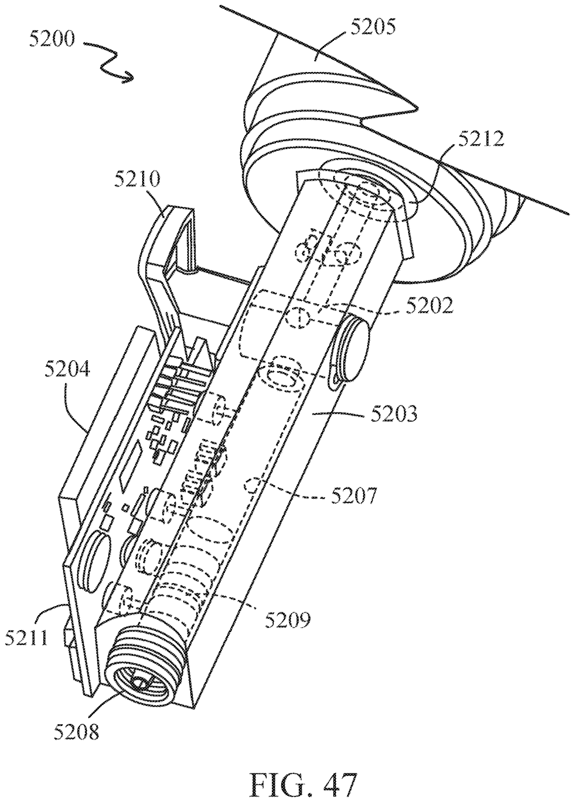

FIG. 47 is a rear perspective view of the dispenser 5200 of FIG. 44, with portions broken away, the touch screen housing 5201 not shown and the tunnel 5207 shown in phantom.

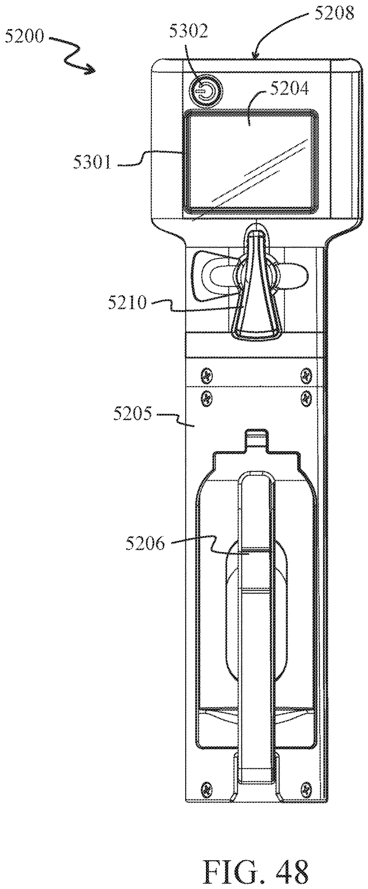

FIG. 48 is a front view of the dispenser 5200 of FIG. 44, including the touch screen housing 5201.

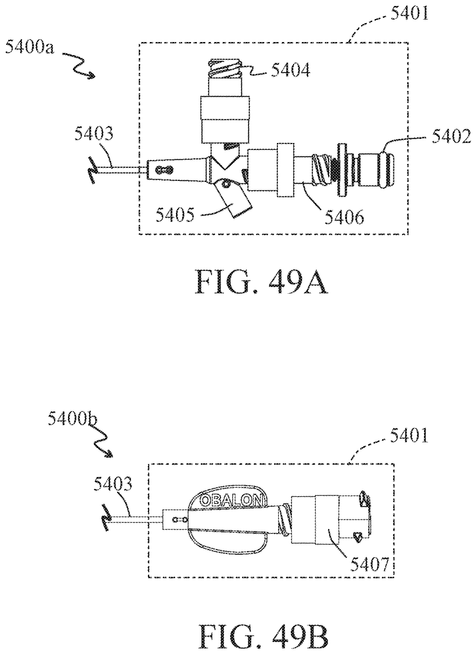

FIG. 49A is a side view of an exemplary catheter connection assembly 5400a for use with the dispenser 5200 of FIG. 44.
FIG. 49B is a side view of another exemplary catheter connection assembly 5400b for use with the dispenser 5200 of FIG. 4.

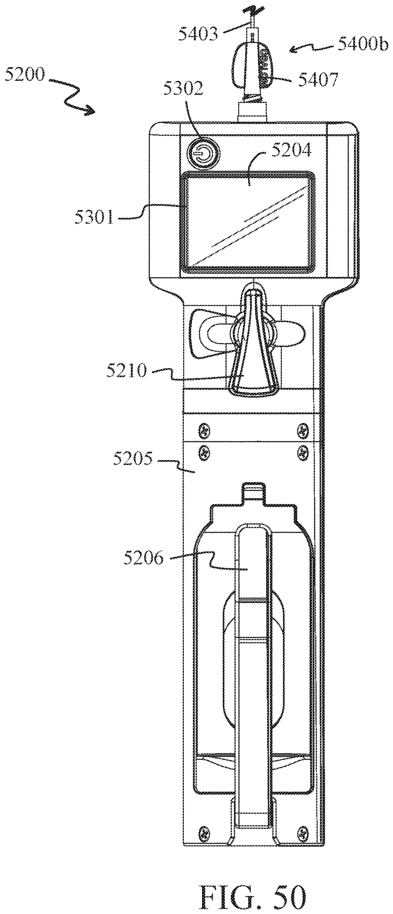

FIG. 50 is a front view of the dispenser 5200 of FIG. 44 with a catheter connection assembly 5400b of FIG. 36B attached to a dispenser quick disconnect valve 5208.

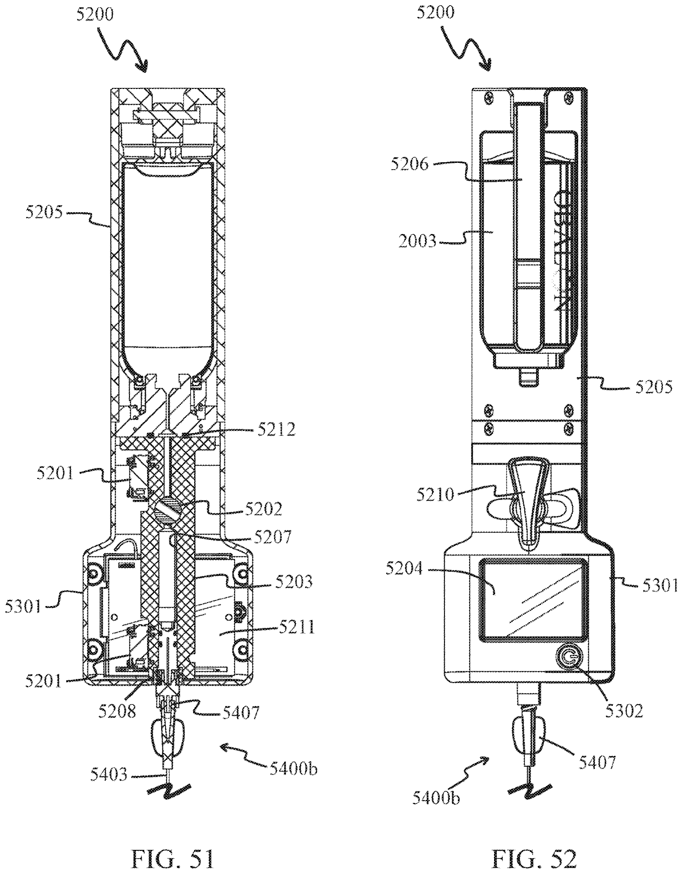

FIG. 51 is a cross-section of the dispenser 5200 of FIG. 44, the cross-section being taken along line 51-51 of FIG. 46.
FIG. 52 is a front view of the dispenser 5200 of FIG. 44 with a fill fluid canister 2003 engaged in a canister housing 5205 and locked in place by a locking mechanism 5206.

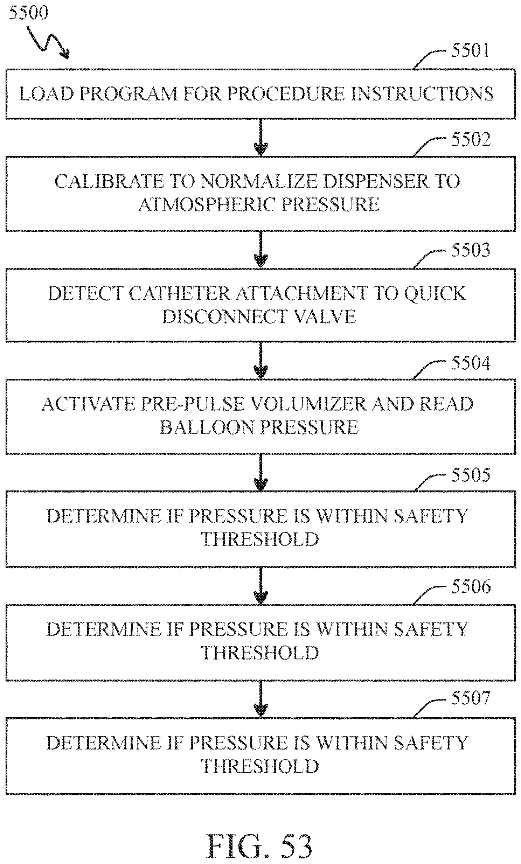

FIG. 53 is a flow chart 5500 illustrating steps in using the dispenser 5200 of FIG. 42 to inflate an inflatable intragastric device.

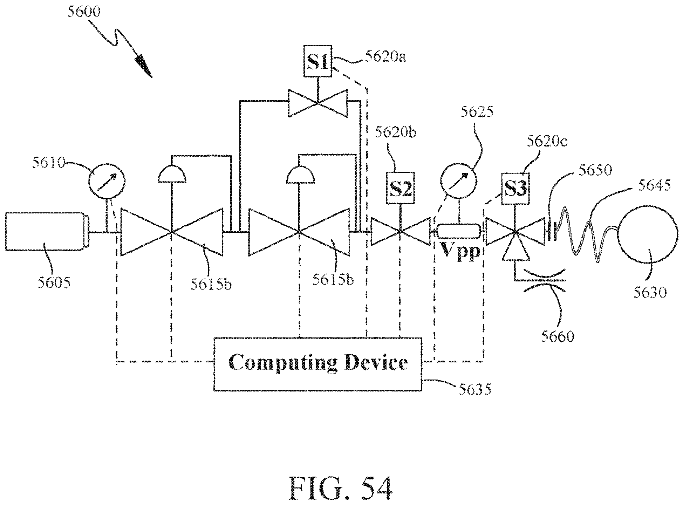

FIG. 54 is a schematic of the components of a Dispenser 5600 in an embodiment.

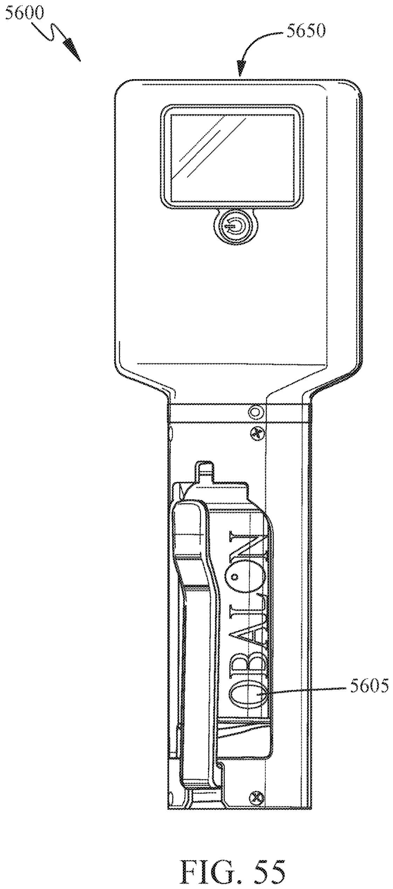

FIG. 55 is a top view of the Dispenser 5600 of FIG. 54.

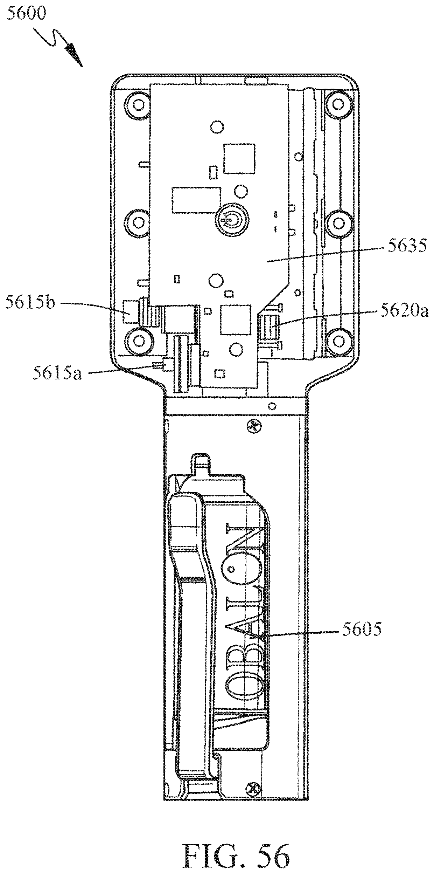

FIG. 56 is a top view of the Dispenser 5600 of FIG. 54, with the top shroud removed.

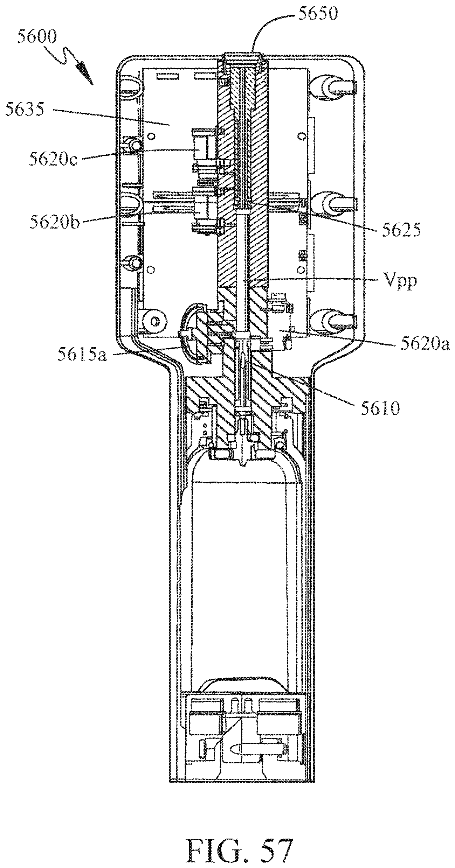

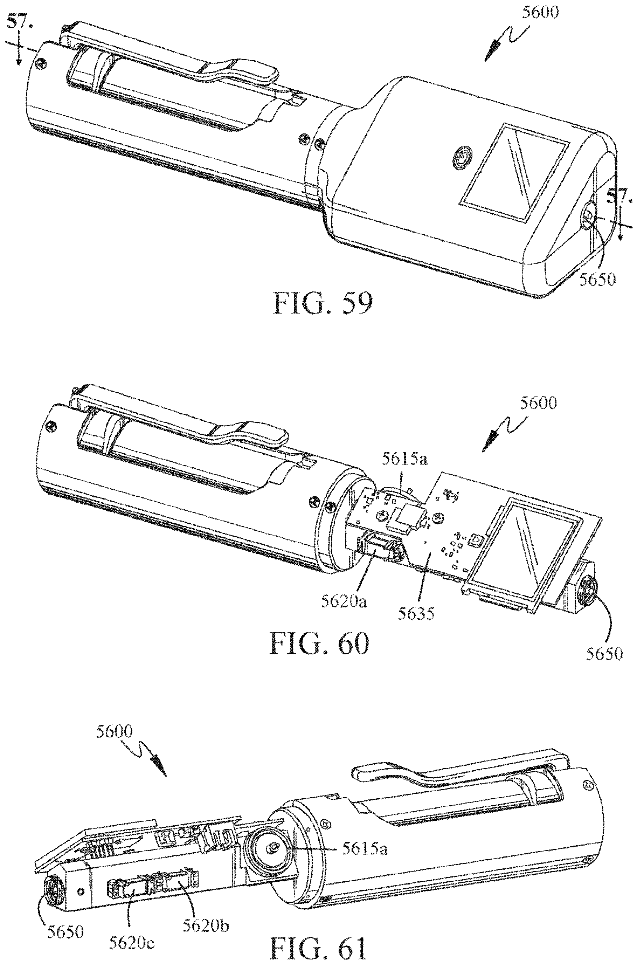

FIG. 57 is a bottom cross-sectional view of the Dispenser 5600 of FIG. 54 with the cross-section being taken along line 57-57 of FIG. 59.

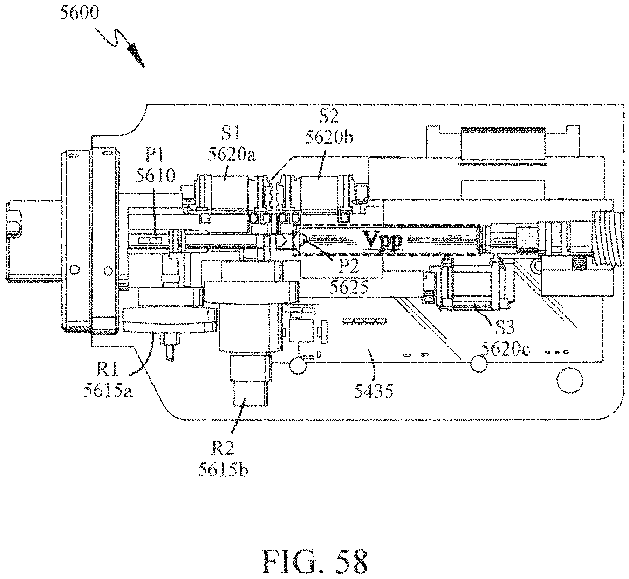

FIG. 58 is a schematic of the Dispenser 5600 of FIG. 54, illustrating the positions of various components.
FIG. 59 is a top perspective view of the Dispenser 5600 of FIG. 54.
FIG. 60 is a front perspective view of the Dispenser 5600 of FIG. 54 with the shroud removed to show portions of the computer system 5635.
FIG. 61 is a rear perspective view of the Dispenser 5600 of FIG. 54.

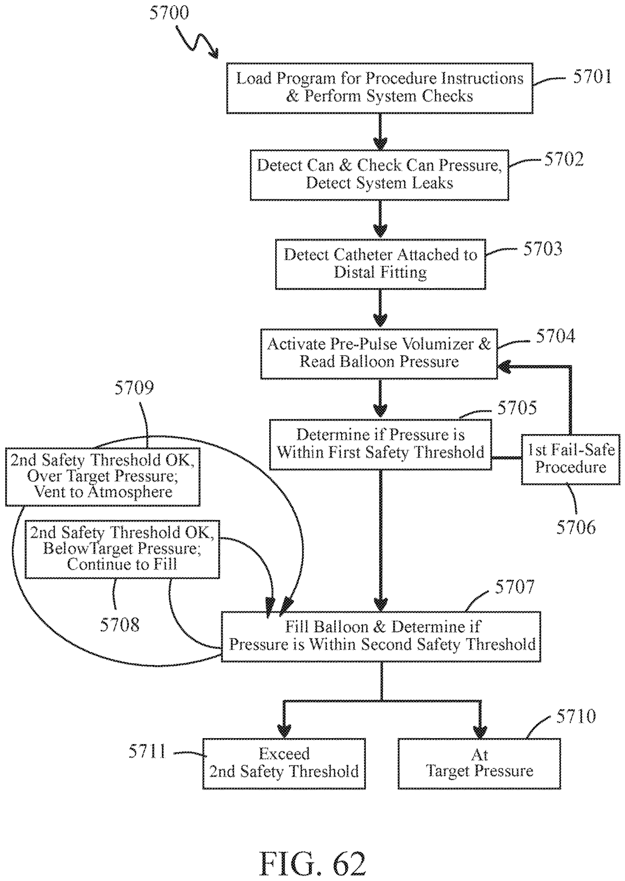

FIG. 62 is a flow chart 5700 illustrating steps in using the dispenser 5600 of FIG. 54 to inflate an inflatable intragastric device.

### DETAILED DESCRIPTION OF THE EMBODIMENT

The following description and examples illustrate a embodiment of the present invention in detail. Those of skill in the art will recognize that there are numerous variations and modifications of this invention that are encompassed by its scope. Accordingly, the description of a embodiment should not be deemed to limit the scope of the present invention.

The term "degradable" as used herein is a broad term, and is to be given its ordinary and customary meaning to a person of ordinary skill in the art (and is not to be limited to a special or customized meaning), and refers without limitation to a process by which structural integrity of the balloon is compromised (e.g., by chemical, mechanical, or other means (e.g., light, radiation, heat, etc.) such that deflation occurs. The degradation process can include erosion, dissolution, separation, digestion, disintegration, delamination, comminution, and other such processes.

The term "swallowable" as used herein is a broad term, and is to be given its ordinary and customary meaning to a person of ordinary skill in the art (and is not to be limited to a special or customized meaning), and refers without limitation to ingestion of a balloon by a patient such that the outer capsule and its constituents are delivered to the stomach 20 *via* normal peristalsis movement. While the systems of embodiments are swallowable, they are also configured for ingestion by methods other than swallowing. The swallowability of the system is derived, at least in part, by the outer container size for the self-inflating system and the catheter and outer container size for the manual inflation system. For the self-inflating system, the outer capsule is sufficient to contain the inner container and its constituents, an amount of activation agent injected prior to administration, the balloon size and the balloon material thickness. The system is preferably of a size less than the average normal esophagus diameter.

Described herein is an orally ingestible device. In embodiments, the device is able to traverse the alimentary canal. The device may be useful, for example, as an intragastric volume-occupying device. The device overcomes one or more of the above-described problems and shortcomings found in current intragastric volume-occupying devices.

In order to more clearly describe the subject matter of the embodiments, different embodiments of the same subcomponent will be described under a single relevantly-titled subheading. This organization is not intended to limit the manner in which embodiments of different subcomponents may be combined in accordance with the present invention.

### SWALLOWABLE INTRAGASTRIC BALLOON SYSTEM

A swallowable, inflatable intragastric balloon system according to selected embodiments includes the following components: self-sealing valve system for addition of fluid to the lumen of the balloon ("valve system"), an intragastric balloon in a deflated and compacted state ("balloon" or "volume-occupying subcomponent") and an outer capsule, container, or coating ("outer container") that contains the balloon. An inflation assembly, described in greater detail in the section entitled "Inflation Assembly," is provided for inflating the balloon after ingestion or placement in the stomach. The inflation assembly includes, but is not limited to, an inflation fluid source and a catheter and/or tubing. In some embodiments, the inflation fluid source comprises an inflation fluid dispenser ("dispenser") and a container of inflation fluid ("can" or "canister") for use with the dispenser. A valve providing releasable attachment of the inflation assembly catheter or tubing to the balloon is provided. Preferably, the self-sealing valve system attached to the balloon (e.g., on its inside surface) is "universal" or compatible with a swallowable catheter or a physician-assisted catheter. The valve system serves to allow for balloon inflation using a miniature catheter that includes a needle assembly, while the balloon is in the patient's stomach, and provides a mechanism for detachment of the catheter after balloon inflation has been completed.

The outer container preferably incorporates the balloon in a compacted state (e.g., folded and rolled).

Selected components of a swallowable intragastric balloon system of a embodiment can include a silicone head with radioopacity ring, trimmed 30 D silicone septum, Nylon 6 inoculation spacer, compacted balloon, and outer container as constituents of the system in unassembled form. A fully assembled outer container can include a vent hole aligned with a septum or a port for connection of tubing. As discussed further below, the components of certain systems possess the attributes described herein; however, in certain embodiments systems can be employed which utilize components having other attributes and/or values.

Devices according to the embodiments are intended for ingestion by a patient and deployment without the need to resort to invasive methods. It is therefore desirable that the device of the embodiments be operable to conform to a compact delivery state which can be swallowed by a patient using normal peristaltic motions and with minimal discomfort. Once in the stomach, it is desirable for the device to assume a substantially larger deployed state. In order to achieve the transition from a delivery state to a deployed state the device is subjected to inflation.

In order to treat obesity or assist individuals with their weight loss goals, various embodiments of the intragastric devices 10 (e.g., 'intragastric balloon' or 'balloon') described herein are preferably delivered to a patient's stomach 20 and maintained in the patient's stomach 20 in an inflated state, preferably for at least thirty days (see FIG. 1). In some embodiments, the inflated intragastric devices 10 are maintained in a patient's stomach 20 for a treatment duration of one to three months. In a more embodiment, the devices 10 are maintained in a patient's stomach 20 for at least six months., In an even more embodiment, the devices 10 are maintained in a patient's stomach 20 for at least 9 months, at least 12 months, or longer. A plurality of intragastric devices 10 may be delivered to a patient's stomach 20 during a treatment duration. For example, in some embodiments, up to two or three or more inflated intragastric devices 10 (of the same size or of two or more different sizes) may be present in a patient's stomach 20 at a point in time, or during the course of treatment. At the end of treatment, the devices 10 may be removed endoscopically. In other embodiments, the devices 10 may self-deflate, such as at the end of treatment, and pass through the lower gastrointestinal tract. In embodiments, this self-deflation of the devices 10 occurs at the end of a predetermined treatment time period, such as but not limited to a treatment period of about 3 months, about 6 months, about 9 months, about 12 months or longer. In order to maintain a proper degree of inflation and reduce discomfort and/or side effects for a patient, the patient may be prescribed one or more prescription drugs to take regularly while the inflated intragastric device 10 is in the patient's stomach 20. For example, in some embodiments, a proton pump inhibitor, an antiemetic, and/or a spasmolytic agent may be prescribed.

### OUTER CONTAINER

FIG. 2 illustrates an intragastric balloon assembly 30. The intragastric balloon 10 of the assembly 30 is preferably provided in a deflated and folded state in a capsule 40 or other retaining, containing or coating structure ("outer container"). The outer container 40 is preferably in the form of a standard push-fit gelatin capsule, with the push-fit relied upon for containing the deflated/folded balloon; however, a gelatin wrap can advantageously be employed in certain embodiments. Gelatin can advantageously be used as the outer container 40; however other materials can also be suitable for use, e.g., cellulose, collagen, and the like. Preferably, the outer container 40 has a length (longest dimension) of from about 0.95 inches (2.4 cm) to 2.5 inches (6.3 cm) and a diameter or width of from about 0.35 inches (0.9 cm) to about 0.9 inches (2.4 cm). The outer container 40 may be configured with one or more holes, slits, passageways or other egresses, preferably on each end. The process of the outer capsule 40 degrading (e.g., separates, dissolves, or otherwise opens) is expedited by pressure build up caused by inflation (inflation *via* catheter 200) of the balloon 10. The outer capsule 40 can be dipped in water for a brief time to soften the materials but not release the balloon 10 prior to swallowing to minimize the time lapse between swallowing and balloon inflation. The outer container is provided with a hole to house the inflation tube needle assembly (e.g., the bell-shaped needle sleeve 220, such as is shown in FIGS. 8-9), wherein the diameter of the catheter needle housing is mechanically compatible with the diameter of the outer container hole such that the needle 230 can be inserted into the self-sealing valve 100 (see FIGS. 5-7, 9) while maintaining therein the housed balloon 10 to facilitate pushing or swallowing of the balloon assembly. In a embodiment, the outer container 40 is a capsule 40. The distal half of the capsule 40 may be flared to prevent abrasion of the balloon 10 materials by the leading edge of the capsule 40 as the compacted balloon 10 is inserted into the capsule 40. The capsule 40 can also comprise two parts held together with a gel band and encompassing the folded balloon 10 that allows for quicker separation of the capsule 40 so that inflation can take place more expeditiously. The outer capsule 40 degrades (e.g., separates, dissolves, or otherwise opens) due to contact with ingested fluid ingestion (e.g., water intake) and preferably degrades within 5 minutes or less, more preferably within 2 minutes or less, so as not to cause discomfort to the patient while the balloon/catheter tube is in place.

In a embodiment, the device (e.g., intragastric balloon 10) is fitted into a standard sized gelatin capsule 40. The capsule 40 may be formed of a material that has a known rate of degradation such that the balloon 10 will not be released from the capsule 40 or otherwise deployed prior to entry into the stomach 20 (see FIG. 1). For example, the capsule 40 materials may include one or more polysaccharide and/or one or more polyhydric alcohols.

Alternatively, the device, in its delivery state (e.g., the balloon 10 folded and compacted inside the capsule 40), may be coated in a substance that confines the balloon 10 in its delivery state while also facilitating swallowing. The coating may be applied by a dipping, sputtering, vapor deposition, or spraying process which may be conducted at an ambient or positive pressure. The balloon 10 may also be encapsulated by wrapping gelatin tape around the balloon 10 and then placing the wrapped balloon 10 in a capsule 40, if so desired.

In certain embodiments, the encapsulated or coated device Obalon Gastric Balloon Assembly 30 is lubricated or otherwise treated so as to facilitate swallowing. For example, the encapsulated or coated device 30 may be wetted, heated, or cooled, prior to swallowing by the patient. Alternatively, the encapsulated or coated device 30 may be dipped in a viscous substance that will serve to lubricate the device's 30 passage through the esophagus. Examples of possible coatings can be any substances with lubricious and/or hydrophilic properties and include glycerine, polyvinylpyrrolidone (PVP), petroleum jelly, aloe vera, silicon-based materials (e.g. Dow 360) and tetrafluoroethylene (TFE). The coating may also be applied by a sputtering, vapor deposition or spraying process.

In additional embodiments, the coating or capsule 40 is impregnated or treated with one or more local anesthetics or analgesics to ease swallowing. Such anesthetics may include anesthetics in the amino amide group, such as articaine, lidocaine and trimecaine, and anesthetics in the amino ester group, such as benzocaine, procaine and tetracaine. Such analgesics may include chloraseptic.

In certain embodiments, the capsule 40 may be weighted at a certain end in order for it to be oriented appropriately when it is administered, as it travels down the esophagus, and/or when it is in the stomach 20. The weighting components may include polymer materials or inflation reactants.

### INTRAGASTRIC BALLOON

The intragastric balloon 10 of the embodiments, also referred to herein as a volume-occupying subcomponent, is generally formed of a flexible material forming a wall which defines an exterior surface and an interior cavity or lumen with an inner surface. The volume-occupying subcomponent 10 can vary in size and shape according to the patient's internal dimensions and the desired outcome. The volume-occupying subcomponent 10 may be engineered to be semi-compliant, allowing the volume-occupying subcomponent 10 to stretch or expand with increases in pressure and/or temperature. Alternatively, in some embodiments, a compliant wall offering little resistance to increases in volume may be desirable.

Spherical or ellipsoidal volume-occupying subcomponents 10 are advantageously employed in certain embodiments. Delivery, inflation and deflation of the volume-occupying subcomponent 10 may be accomplished by any of the methods described in U.S. Patent No. 8,647,358, issued February 11, 2014, or U.S. Patent Publication No. 2013/0226219, published August 29, 2013, each of which is incorporated herein in its entirety.

It is advantageous in certain embodiments for the volume-occupying subcomponent 10 wall to be both high in strength and thin, so as to minimize the compacted volume of the device as it travels the esophagus of the patient. In certain embodiments, the volume-occupying subcomponent 10 wall materials are manufactured with a biaxial orientation that imparts a high modulus value to the volume-occupying subcomponent 10.

In one embodiment, the volume-occupying subcomponent 10 is constructed of a polymeric substance such as polyurethane, polyethylene terephthalate, polyethylene naphthalate, polyvinyl chloride (PVC), Nylon 6, Nylon 12, or polyether block amide (PEBA). The volume-occupying subcomponent 10 may be coated with one or more layers of substances that modify (increase, reduce, or change over time) gas-barrier characteristics, such as a thermoplastic substance.

Preferably, the gas-barrier materials have a low permeability to carbon dioxide or other fluids that may be used to inflate the volume-occupying subcomponent 10. The barrier layers should have good adherence to the base material. Barrier coating materials include biocompatible poly(hydroxyamino ethers), polyethylene naphthalate, polyvinylidene chloride (PVDC), saran, ethylene vinyl alcohol copolymers, polyvinyl acetate, silicon oxide (SiOx), acrylonitrile copolymers or copolymers of terephthalic acid and isophthalic acid with ethylene glycol and at least one diol. Alternative gas-barrier materials may include polyamine-polyepoxides. These materials are commonly acquired as a solvent or aqueous based thermosetting composition and are generally spray-coated onto a preform and then heat-cured to form the finished barrier coating. Alternative gas-barrier materials which may be applied as coatings to the volume-occupying subcomponent 10 include metals such as silver or aluminum. Other materials that may be used to improve the gas impermeability of the volume-occupying subcomponent 10 include, but are not limited to, gold or any noble metal, PET coated with saran, conformal coatings and the like, as listed, for example, in Tables 1a-b, below.

In certain embodiments, the volume-occupying subcomponent 10 is injection, blow or rotational molded. Either immediately following such molding, or after a period of curing, the gas-barrier coating may be applied if not already applied within the composite wall.

In another embodiment, the intragastric volume-occupying subcomponent 10 is formed using a Mylar polyester film coating silver, aluminum or kelvalite as a metallicized surface, to improve the gas impermeability of the volume-occupying subcomponent 10.

In the event that the volume-occupying subcomponent's wall is composed of multiple layers of materials, it may be necessary to use certain substances or methods to connect, attach or hold together such multiple layers. Such substances can include a solvent or an ether-based adhesive. Such multiple layers may also be heat-bonded together. Once such layers are attached together to form (for example) a sheet of material to be made into a volume-occupying subcomponent, it may also be necessary to apply additional treatment steps to such material to allow it to seal together (for example, by application of a certain degree of heat and pressure) in order to be made into a volume-occupying subcomponent 10. Accordingly, it may be advantageous to include as an additional layer in the volume-occupying subcomponent 10 certain materials that seal. For example, a volume-occupying subcomponent 10 comprised of a combination of PET and SiOx layers, which impart favorable mechanical and gas impermeability characteristics to the volume-occupying subcomponent, may be sealed by including a layer of sealable polyethylene in such volume-occupying subcomponent 10.

According to another embodiment of the embodiments, the functionality of the volume-occupying subcomponent 10 and the deflation component is combined either in part or in whole. For example, the volume-occupying subcomponent 10 may be formed of a substance that is degraded within the stomach 20 over a desired period of time. Once the degradation process has formed a breach in the wall of the volume-occupying subcomponent, the volume-occupying subcomponent 10 deflates, continues to degrade and passes through the remainder of the digestive tract.

Preferably, an automated process is employed that takes a fully constructed volume-occupying subcomponent, evacuates all of the air within the interior cavity and folds or compresses the volume-occupying subcomponent 10 into the desired delivery state. For example, the evacuation of air from the volume-occupying subcomponent 10 may be actuated by vacuum or mechanical pressure (e.g. rolling the volume-occupying subcomponent). In certain embodiments, it is desirable to minimize the number of creases produced in the volume-occupying subcomponent 10 when in the delivery state.

In another embodiment, deflation of the volume-occupying subcomponent 10 may be achieved through one or more injection sites within the wall of the volume-occupying subcomponent 10. For example, two self-sealing injection sites can be incorporated at opposite sides of the volume-occupying subcomponent 10. The volume-occupying subcomponent 10 may be positioned within a fixture that employs two small-gauge needles to evacuate the air from the volume-occupying subcomponent 10.

In one embodiment, the self-sealing injection sites may further be used to insert chemical elements of the inflation subcomponent into the interior of the volume-occupying subcomponent 10. After injection of the chemical elements into the volume-occupying subcomponent, the same needles may be used to perform evacuation of the volume-occupying subcomponent 10.

It may be desirable that the volume-occupying subcomponent 10 is packed into the delivery state under, for example, a negative vacuum pressure or under a positive external pressure.

The volume-occupying subcomponent wall materials may also be engineered to, once they are initially punctured or torn, tear relatively easily from the point of such puncture or tear. Such properties can, for example, be advantageous if deflation of the volume-occupying subcomponent 10 were initiated by a tearing or puncturing of the volume-occupying subcomponent 10 wall, since such initial tear or puncture may then increase in scope, hastening and/or maximizing the deflation process.

The volume-occupying subcomponent 10 may also be coated by a lubricious substance that facilitates its passage out of the body following its deflation. Examples of possible coatings can be any substances with lubricious and/or hydrophilic properties and include glycerine, polyvinylpyrrolidone (PVP), petroleum jelly, aloe vera, silicon-based materials (e.g. Dow 360) and tetrafluoroethylene (TFE). The coating may be applied by a dipping, sputtering, vapor deposition or spraying process which may be conducted at an ambient or positive pressure.

The balloon composite wall materials can be of similar construction and composition as those described in U.S. Patent No. 9,072,583, the contents of which is hereby incorporated by reference in its entirety. The materials are able to contain a fluid, preferably in compressed or non-compressed gas form, such as, e.g., N₂, Ar, O₂, CO₂, SF₆ or mixture(s) thereof, or atmospheric air (composed of a mixture of N₂, O₂, Ar, CO₂, Ne, CH₄, He, Kr, H₂, and Xe) that simulate gastric space concentrations. In certain embodiments, the balloon is able to hold the fluid (gas) and maintain an acceptable volume for up to 6 months, preferably for at least 1 to 3 months after inflation. Certain fill gases include non-polar, large molecule gases that can be compressed for delivery.

Prior to placement in the outer container, the balloon 10 is deflated and folded. In the inverted configuration in a deflated state, the balloon 10 is flat, with the inverted seam extending around the perimeter of the balloon 10. A self-sealing valve system 100 is affixed to the inner wall of the lumen close to the center of the deflated balloon 10. The walls of the balloon 10 are then folded. As part of the balloon 10 design, the self-sealing valve system 100 is manufactured in a manner such that it can be and is preferably placed "off center" to minimize the number of folds upon themselves (e.g., doubling or tripling up) required to fit the balloon in the outer container. For example, the self-sealing valve system 100 can advantageously be placed ½ r ± ¼ r from the center of the balloon, wherein r is the radius of the balloon along a line extending from the center of the balloon through the septum.

Additional description of intragastric balloons 10 and the fabrication of such, for use with the embodiments herein, can be found in U.S. Patent No. 8,162,969, issued April 24, 2012, U.S. Patent No. 9,072,583, issued July 7, 2015, U.S. Patent No. 8,647,358, issued February 11, 2014, U.S. Patent No. 8,292,911, issued October 23, 2012, U.S. Patent No. 8,202,291, issued June 19, 2012, U.S. Publication No. 2013/0226219, published August 29, 2013, U.S. Publication No. 2016/0310306, published October 27, 2016, International Publication WO 2016/200612, published December 15, 2016, U.S. Publication No. 2017/0156909, published June 8, 2017, and US Patent Application No. US 15/623,175, filed June 14, 2017, each of which is incorporated herein in its entirety.

### TRACKING AND VISUALIZATION SUBCOMPONENT

It may also be beneficial to implement tracking and visualization functionality into devices according to the embodiments. Due to the non-invasive nature of the present device, physicians may desire to determine, or confirm, the location and orientation of the device prior to inflation or during the course of treatment.

A radiographic marker may be applied to the volume-occupying subcomponent 10 when the volume-occupying subcomponent 10 is in a creased or folded state such that when the volume-occupying subcomponent 10 is in its deflated state the marker appears concentrated when viewed on visualization equipment, and when the volume-occupying subcomponent 10 is inflated the marker appears less concentrated when viewed on visualization equipment. Alternatively, a marker may be applied or incorporated into the volume-occupying subcomponent 10 at multiple positions so as to facilitate identification and location of the various subcomponents of the device, such as a valve, head, or weight. The marker may be printed or painted onto a surface of the volume-occupying subcomponent 10 or between layers of the material forming the volume-occupying subcomponent 10. Alternatively, a metal coating as described below may be used as a marker to identify and/or locate the volume-occupying subcomponent 10. Metal coatings for visualizing the volume-occupying subcomponent 10 may include silver, gold, tantalum or any noble metal. Alternatively, the marker may be applied to an elastomeric sleeve that covers all or part of the volume-occupying subcomponent 10.

In another embodiment, the volume-occupying subcomponent 10 incorporates a subcomponent that changes mechanically upon inflation of the volume-occupying subcomponent, which mechanical change can be visualized using x-ray or other visualization equipment. For example, a mechanical portion of the volume-occupying subcomponent 10 containing a visualization marker may elongate upon an increase in pressure in the volume-occupying subcomponent 10.

Alternatively, a marker may be formed using a metallized mesh located between layers of the material from which the volume-occupying subcomponent 10 is constructed. The pattern or patterns formed by the imbedded marker will appear when the volume-occupying subcomponent 10 is in an inflated, deployed state.

It is envisioned that marker materials may be incorporated into the volume-occupying subcomponent 10 to facilitate various visualization techniques such as, for example, MRI, CT and ultrasound.

The volume-occupying subcomponent 10 may also contain a dye or marker that is released upon deflation to indicate that the volume-occupying subcomponent 10 cavity has been breached. Such dye or marker may, for example, be apparent in the patient's urine as an indication that the volume-occupying subcomponent 10 has begun to deflate.

In yet further embodiments, microchips and other components employing electronic modalities may be used to locate and identify a device. Microchips analogous to those utilized for the identification of pets may be used to communicate device specific information and its approximate location. For example, a Wheatstone or other bridge circuit may be incorporated into the device and, together with RF "ping and listen" technology may be used as part of a system to determine the device's approximate location and measure and communicate device specific information. Such device specific information can include internal volume-occupying subcomponent 10 pressure, which can indicate the degree of inflation of the volume-occupying subcomponent 10.

In yet further embodiments, mechanical, chemical, visual and other sensors may be included as part of the device to measure, record and/or transmit information relating to the device and/or the patient's internal environment. For example, the device may contain a camera or any of the other imaging and transmission components of a Pillcam device. As an additional example, the device may contain sensors that measure, record and/or transmit information relating to stomach pH, stomach pressure, hormone levels, organ health, and organ safety.

### SELF-SEALING VALVE SYSTEM

In embodiments, a self-sealing valve system 100 is attached to the balloon (e.g., on its inside surface) that is "universal" or compatible with the swallowable catheter and a physician-assisted catheter, such as is described elsewhere herein. The valve system 100 serves to allow for balloon inflation using a miniature catheter that includes a needle assembly and also provides a mechanism for detachment of the catheter after inflation has been completed.

FIGS. 5-7 and 9 depict views representing a design of a self-sealing valve system 100 which contains a self-sealing septum 114 coaxially housed within a metallic concentric cylinder 105 ("tube septum") along longitudinal axis A. The self-sealing valve system 100 is preferably adhered to the underside (e.g., inner surface) of the balloon material such that only a portion of the valve 100 protrudes slightly outside of the balloon surface to ensure a smooth outer surface. The septum 114 preferably consists of a material possessing a durometer of 20 Shore A to 60 Shore D. The septum 114 is inserted or otherwise fabricated into the smaller inner cylinder 105b of the concentric metallic retaining structure 105 that is preferably cylindrical in shape. As shown in FIG. 9, the inner cylinder 105b within the larger outer cylinder 105a controls alignment (e.g., longitudinal or coaxial) of the catheter needle sleeve/needle assembly 220/230 with the septum114, provides a hard barrier so that the catheter needle 230 does not pierce the balloon material (needle stop mechanism), and provides compression such that the valve/septum 100/114 re-seals after inflation and subsequent needle 230 withdrawal.

The concentric valve system 100 can also provide radio opacity during implantation and is preferably titanium, gold, stainless steel, MP35N (nonmagnetic, nickel-cobalt-chromium-molybdenum alloy) or the like. Non-metallic polymeric materials can also be used, e.g., an acrylic, epoxy, polycarbonate, nylon, polyethylene, PEEK, ABS, or PVC or any thermoplastic elastomer or thermoplastic polyurethane that is fabricated to be visible under x-ray (e.g., embedded with barium).

The septum 114 can be cone shaped, so that the compressive forces are maximized for self-sealing after inflation. The self-sealing septum 114 allows air to be evacuated from the balloon 10 for processing/compacting and insertion into the outer container 40, and allows for piercing by an inflation catheter needle 230, and then subsequent detachment of the inflation catheter 200 and withdrawal of the catheter needle 230 significantly limiting gas leakage outside of the balloon 10 during the inflation process and needle withdrawal/catheter detachment. The septum 114 is inserted into the tube septum inner cylinder 105b using a mechanical fit mechanism to provide compression. An additional ring 116 (FIG. 7) can be placed at the distal end of the inner cylinder 105b to provide additional compression to ensure the septum material is pre-loaded so as to re-seal itself. The ring 116 ("ring-stop") is preferably metallic in nature, but can also be a non-metallic polymeric material such as an acrylic, epoxy, or thermoplastic elastomer or thermoplastic polyurethane. In some embodiments, the ring-stop 116 material is preferably the same material as the inner cylinder 105b, titanium, but can also be gold, stainless steel, MP35N or the like.

Referring to FIGS. 5-7 and 9, the larger, outer cylinder 105a of the concentric valve housing 105 ("tube septum") contains a material 110 (e.g., the "head" 110) with a slightly harder durometer than the inner cylinder 105b (50 Shore A or greater), but is also preferably silicone. The purpose of using a harder durometer material is to ensure sealing when connected to the needle sleeve 220 for inflation. The silicone located in the outer cylinder 105a of the concentric retaining structure 105 is adhered to the balloon 10 from the balloon wall inside surface (e.g., element 432 of FIG. 11). The entire outer cylinder 105a is filled and a small circular lip 111 of this same material is provided that is slightly larger than the diameter of the inner cylinder 105b and extends to the balloon wall outside surface (e.g., element 480 of FIG. 2). The lip 111 is compatible with the bell-shaped needle sleeve 220 and provides sealing to enhance connection of the self-sealing valve 100 to the catheter 200 to withstand the inflation pressures applied and also increases the ejection distance or attachment force of the catheter 200. This silicone lip 111 preferably does not protrude past the balloon surface 480 more than 2 mm to ensure that the balloon outer surface 480 remains relatively smooth and does not cause abrasion or ulcerations of the stomach mucosa. The lip 111 is designed to provide compressive forces against the needle sleeve 220 of the catheter 200 for inflation and detachment whereby when connected to the needle sleeve 220 of the inflation catheter 200, the connection coupling can preferably withstand a pressure of 35 PSI during inflation. This seal is then broken during detachment using hydraulic pressure that is preferably more than 40 PSI but less than 200 PSI to separate the coupling. An additional retaining ring 115, preferably made of the same material as concentric valve 105, can be included in the valve system 100 to further enhance the seal between the metal and the valve silicone and provide additional mechanical support to ensure proper mechanical fit and are intended to disrupt slippage of the silicone material from the hard (metallic) valve system (causing an increase in tensile force).

The valve structure uses a mechanical fit mechanism to provide the functions of the self-sealable valve 100 for inflation by the catheter 200 and subsequent catheter 200 detachment; however, primer and/or adhesive may be used to provide additional support in construction of the self-sealing valve assembly 100. The configuration can be modified by modifying the surfaces of the metal components, making them more sticky or slippery e.g. more or less conducive to adhesion, to provide the desired mechanical/interference fit. The interference fit between the self-sealing valve 100 and the catheter 200 can be modified to change the pressure requirements for inflation and/or detachment. Additional assemblies can include overmolding the metallic portions or the concentric system in silicone such that additional support rings to ensure the mechanical fit and the tensile strength and forces required to sustain the assembly during catheter inflation and detachment can be omitted.

The total self-sealing valve diameter is designed to fit a miniature catheter system (e.g., FIG. 9) that does not exceed 8 French (2.7 mm, 0.105 inches) in diameter. The total diameter does not exceed 1 inch (2.54 cm) and is preferably less than 0.5 inches (1.27 cm), to facilitate swallowing. Additional valves can be added, if desired; however, it can be advantageous to employ a single valve 100 so as to maintain the volume of the deflated/folded balloon 10 (and thus the outer container 40 dimensions) as small as possible. The valve system 100 is preferably attached to the balloon 10 and bonded such that a shear force greater than 9 lbs. (40 N) is required to dislodge the valve system 100.

FIGS. 10-13 illustrate another self-sealing valve system 400, in an exemplary embodiment. The self-sealing valve 400 includes a head unit 410 with a longitudinal axis B, and which is preferably formed of a polymer such as described elsewhere herein. In the illustrated exemplary embodiment, the head unit 410 includes a top portion 412 and a bottom portion 413. The top portion 412 includes an annular top surface 416 with a top opening 418 defined by a top edge 420. The bottom portion 413 includes a bottom surface 422 with a bottom opening 424 defined by a bottom edge 426. The head unit 410 includes an outer surface 428 that joins the top surface 416 with the bottom surface 422. In the exemplary illustrated embodiment, the outer surface 428, or top portion 412, includes an optional shoulder portion 430. The shoulder portion 430 provides for a larger top surface 416 while simultaneously enabling minimization of the overall size of the valve system 400. The larger top surface 416 facilitates secure attachment of the head unit 410 to the inner surface 432 of the balloon wall 434, such as is shown in FIGS. 11-13. For example, the valve system 400 may be adhered to the balloon inner surface 432 with an adhesive 435. At the same time, the smaller size of the valve system 400 allows for tighter folding of the intragastric balloon during manufacture, such that the folded balloon can be inserted into a smaller outer container or capsule 40. Smaller capsule size reduces swallowing difficulties of the patient receiving the balloon. In other embodiments, the valve system 400 lacks a shoulder portion and therefore is substantially cylindrical along its total length.

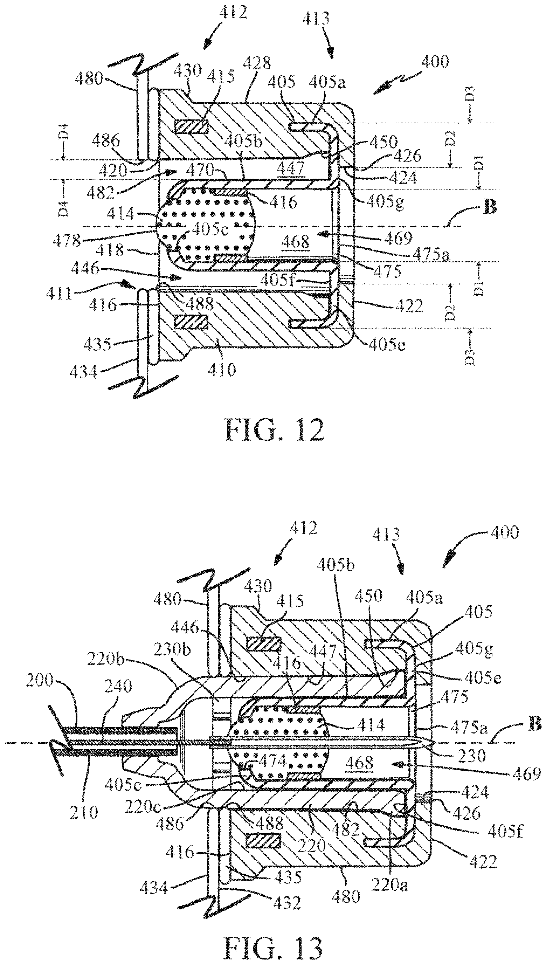

Still referring to FIGS. 11-14, the top and bottom edges, 420 and 426 respectively, are joined by a coaxial through-channel 446 with a cylindrical inner surface 447. In the illustrated embodiment, the through-channel 446 is coaxial with the head unit outer surface 428. The through-channel 446 is substantially cylindrical with a surface 447 configured to contact and engage the needle sleeve outer surface 220b (e.g., see FIG. 13). In some embodiments, the through-channel 446 includes a ring-channel 450that is shaped as a radial groove, cavity, duct, bulge or gap. The ring-channel 450 is sized and shaped to cooperatively receive the bottom rim 220a of the bell-shaped needle sleeve 220. In embodiments, the through-channel 446 is sized and shaped so as to center the bell-shaped needle sleeve 220 during engagement with the valve system 400, such that the needle-sleeve is coaxial with the valve system 400 and the hollow needle 230 pierces a self-sealing septum 414 substantially along the longitudinal axis A, such as is described below. An exemplary bell-shaped needle-sleeve 220 is shown in FIGS. 8-9 and described elsewhere herein. FIG. 13 illustrates another exemplary bell-shaped needle sleeve 220. Additional bell-shaped needle sleeves are foreseen.

Coaxial with the head unit 410 is a retaining structure 405, also referred to herein as a retainer or a tube septum 405. The retaining structure 405, or portions thereof, may be over-molded, or otherwise embedded, within the head unit 410, using techniques known in the art. The retaining structure 405 comprises concentric outer and inner metallic cylinders, 405a and 405b respectively. The outer cylinder 405a is concentric with the inner cylinder 405b and the head unit 410, and may be partially or wholly embedded within the head unit 410. The inner and outer metallic cylinders 405b and 405a are joined by lower wall 405e. The lower wall 405e includes an upwardly or inwardly-facing upper surface 405f and a downwardly our outwardly-facing lower surface 405g. The inner cylinder 405b includes an inner surface 468 defining an inner channel 469, an outer surface 470, and an inwardly-curing inner lip 405c that defines an upper opening 474. A lower opening 475, defined by edge 475a, joins the inner surface 468, of the inner cylinder 405b, with the lower wall lower surface 405g, whereby the inner channel 469 is fluidly joined, fluidly connected or in fluid communication with the lumen or interior of the balloon. In the illustrated embodiment, the retaining structure lower opening 475 includes a diameter D1 that is smaller than the diameter D2 of the head unit bottom opening 424 (see FIG. 12), such that a portion of the lower wall lower surface 405g is exposed to the lumen of the balloon. Additionally, the outer cylinder 405a includes a diameter D3 that is larger than the head unit bottom opening 424, such that the retaining structure 405 is secured within the head using 410. It is foreseen that the head unit bottom opening 424 may include a diameter D2 that is smaller than shown, such that less or none of the lower wall lower surface 405g is exposed. It is also foreseen that the diameter D2 of the head unit bottom opening 424 may be larger, such as to enable insertion of the retaining structure 405 into the head unit 410 during manufacturing, rather than over-molding the head unit 410 onto the retaining structure 405.

The self-sealing septum 414 is compressed against the lip inner surface 468 by a ring-stop 416, such that the upper opening 474 comprises a gas-tight seal and a portion of the self-sealing septum 414 extends or protrudes therethrough. In the illustrated embodiment, the inner cylinder 405b includes a length such that the self-sealing septum 414 is wholly within the balloon 10, or below the balloon outer surface 480 (e.g., see FIG. 12). However, it is foreseen that the length of the inner cylinder 405b may be greater, such that the top 478 of the self-sealing septum 414 is flush with the outer surface 480 of the balloon or extends slightly above the balloon outer surface 480. Advantageously, locating the self-sealing septum top 478 within the balloon, or at or adjacent to the balloon outer surface 480 ensures that the outer surface 480 is substantially smooth at the location of the self-sealing valve system 400, so as to prevent irritation of the stomach mucosa during residency of the balloon within the patient's stomach, thereby contributing to enablement of extended treatment periods and reduction of patient injury during such treatment.

As shown in FIGS. 11-13, the valve system 400 includes a connection channel 482 that is sized and shaped to receive and engage the bell-shaped needle sleeve 220. In the illustrated embodiment, the connection channel 482 is defined by the through-channel inner surface 447 of the head unit 410, the upper surface 405f of the retaining structure lower wall 405e, and the inner cylinder outer surface 470. As shown in FIG. 12, the distance D4 between the head unit inner surface 447 and the inner cylinder outer surface 470 is sized and shaped, or otherwise configured, so as to receive the bell-shaped needle sleeve 220 therein. For example, in the embodiment shown in FIG. 13, the needle-sleeve bottom rim 220a abuts the lower wall upper surface 405f and also engages the through-channel ring channel 450 so as to provide and/or contribute to an interference fit between the needle sleeve 220 and the self-sealing valve 400. Further, as discussed elsewhere herein, the needle-sleeve outer surface 220b frictionally engages the head using inner surface 447. Similarly, the needle sleeve inner surface 220c frictionally engages the outer surface of the inner cylinder.

In the illustrated embodiment, the self-sealing valve 400 includes a metallic retainer ring 415 embedded or housed within the head unit 410. The retainer-ring 415 is sized and shaped so as to provide and/or contribute to the interference fit with a bell-shaped needle sleeve 220 received into the connection channel 482. For example, the retainer-ring 415 may exert pressure or compress the polymeric head material located between the retainer-ring inner surface and a received needle-sleeve 220, so as to facilitate or strengthen an interference fit between the needle-sleeve 220 and the head 410. As described elsewhere herein, the interference fit provides for sealing of the self-sealing valve 400 to the inflation catheter 200. In embodiments, the interference fit is sufficient to maintain a gas-tight seal, as well as the engagement between the valve system 400 and the inflation catheter 200, during swallowing and subsequent inflation of the balloon, wherein the inflation catheter 200 is connected to the intragastric balloon 10 prior to inflation is of a size and shape configured for swallowing by a patient in need thereof.

In some embodiments, such as is shown in FIGS. 11-13, the edge 486 of the balloon wall opening and/or the adhesive 435 adhering the self-sealing valve system 400 to the wall inner surface 432 may provide a lip or protrusion 411 that extends over a portion of the connection channel 482, so as to press against the outer surface 220b of the needle sleeve 220 and thereby provide and/or contribute to the interference fit with a bell-shaped needle sleeve 220. It is foreseen that the balloon wall opening edge 486 and the adhesive 488 may not form a lip 411.

As shown in FIG. 13, the bell-shaped needle sleeve 220 includes a needle holder 230b that engages the hollow needle 230, so as to ensure that the hollow needle 230 is substantially coaxial with the self-sealing septum 414 when the bell-shaped needle sleeve 220 is engaged with the self-sealing valve system 400. The hollow needle 230 may extend through the self-sealing valve system 400, however, the hollow needle 230 can advantageously be sized and shaped to as to be retained within the inner through-channel 446 of the head unit 410, so as to reduce the possibility of damage to the balloon interior surface 432.

### BALLOON COMPOSITE WALL

The materials selected for the composite wall 434 of the balloon 10 may be optimized to maintain the original inflation gas without significant diffusion, or may also allow for diffusion of the gases located in the gastric environment, e.g., CO₂, O₂, argon, or N₂ to diffuse through the wall of the balloon 10 to inflate, partially or wholly, once the balloon 10 is placed in the stomach. A fluid (a liquid or gas) can also be added inside of the balloon 10 using the inflation catheter(s) described herein to change diffusion direction of the balloon 10 composite wall and when it reaches stasis based on the internal and external environment.

A gastric balloon 10 inflated by nitrogen, CO₂ gas, SF₆, a single fluid (gas) or a mixture of gasses employs a composite wall that provides barrier properties (fluid retention), properties imparting resistance to pH and moisture conditions in the gastric environment or the environment within the central lumen of the balloon 10, and structural properties to resist gastric motility forces, abrasion of the balloon 10 wall in vivo, and damage during manufacturing and folding of the balloon 10. Certain materials employed in the balloon 10 materials are able to withstand a hostile gastric environment designed to break down foreign objects (e.g., food particles). Some of the variables that the gastric environment encompasses are as follows: gastric liquid pH of from 1.5-5; temperature of approx. 37°C; a relative humidity of 90-100%; ingress of gastric space gas content; and constant gastric motility external pressures of from 0-4 psi at variable frequencies and cycle times based on the fed state of the stomach. The external pressure imparted by gastric motility can also cause abrasions on the surface of the balloon 10. The inside of the balloon lumen may contain moisture from a solution injected in the balloon 10 for timing of auto-deflation or any moisture that has transferred across the membrane due to the external humid environment. In addition to these environmental stresses the wall materials meet biocompatibility requirements and are constructed such that the total thickness of the wall (barrier material) is thin enough to be compacted and placed inside of a swallowable-sized container ("outer container") without considerable damage or lodging. The outer container is small enough to transcend the esophagus (which has a diameter of approximately 2.5 cm). The wall or barrier material is also heat formable and sealable for balloon 10 construct and maintains a bond strength that can contain internal gas pressures of up to 10 psi generated by the initial inflation pressure as well as pressure due to the ingress of gas molecules from the stomach cavity until the system's gas environment reaches stasis. The film properties that are evaluated to determine suitability for use in the composite wall of the balloon 10 include pH resistance, water vapor transmission rate, gas barrier properties, mechanical strength/abrasion properties, temperature resistance, formability, flex-crack (Gelbo) resistance, surface energy (wettability) compliance, and heat bond potential.

The various layers in the composite wall can impart one or more desirable properties to the balloon 10 (e.g., fluid retention, resistance to moisture, resistance to acidic environment, wettability for processing, and structural strength). A list of polymer resins and coatings that can be combined into a multi-layer preformed system ("composite wall") is provided in Tables 1a-b. These films can be adhesively bonded together, co-extruded, or adhered *via* tie layers or a combination thereof to obtain the desired combination of properties for the composite wall, as discussed below. The materials identified as film coatings in Tables 1a-b are provided as coatings applied to a base polymer film, e.g., PET, Nylon, or another structural layer.

**Table 1a.**

| Film Resins | | | |
|---|---|---|---|
| | Characteristics | | |
| | Good Structural/Behavior/ Mechanical Strength/Compliance | Good Fluid Retention Barrier Properties | Good Manufacturability/ Surface Energy Properties |
| FILM RESINS | | | |
| Polyethylene Terephthalate (PET) | X | X | |
| Polytrimethylene Terephthalate (PTT) | | | |
| Liquid Crystal Polymer (LCP) | X | X | |
| Polytrimethylene naphthalate (PTN) | X | X | |
| Polyethylene naphthalate (PEN) | X | X | |
| Polyimide (PI) | X | X | |
| Linear Low Density Polyethylene (LLDPE) | | | X |
| Ethylene Vinyl Alcohol (EVOH) | | X | |
| Polyamide: Nylon (PA) and Nylon-6 (PAG) /Nylon 12 | | X | X |
| High Density Polyethylene (HDPE) | | | X |
| Polypropylene (PP) | | | X |
| Polyurethane | | | X |
| PVDC (Saran) | | X | X |
| Polyether Block Amide (Pebax) | | | X |
| Polyvinyl Alcohol (PVOH) | | X | |
| Silicone | X | | X |

**Table 1b.**

| Film Coatings | | | |
|---|---|---|---|
| | Characteristics | | |
| | Good Structural/B ehavio r/ Mechanical Strength/Compliance | Good Fluid Retention Barrier Properties | Good Manufacturability/ Surface Energy Properties |
| FILM COATINGS | | | |
| Silicone Dioxide (SiO2) | | X | |
| Aluminum Oxide (Al₂O₃) | | X | |
| Nanopolymers (Nano/Clay) | | X | |
| External Organic Coatings (e.g., epoxy amine) | | X | |
| Inorganic Coatings (e.g., Amorphous Carbon) | | X | |
| Oxygen Scavengers | | X | |
| Parylene C | | X | |

### Fluid Retention Layers

In embodiments, a blended polymer resin using multiple layers is employed to maintain the inflated balloon's shape and volume by retaining the inflation fluid for the duration of the intended use. Certain barrier films, widely used in the food packaging and plastic bottling industries, can advantageously be employed for this purpose in the composite wall of the balloon 10. Preferably, the barrier materials have a low permeability to carbon dioxide (or other gases, liquids, or fluids that are alternatively or additionally used to inflate the volume-occupying subcomponent). These barrier layers preferably have good adherence to the base material. Barrier coating materials and films include polyethylene terephthalate (PET), linear low density polyethylene (LLDPE), ethylene vinyl alcohol (EVOH), polyamides such as Nylon (PA) and Nylon-6 (PA-6), polyimide (PI), liquid crystal polymer (LCP), high density polyethylene (HDPE), polypropylene (PP), biocompatible poly(hydroxyamino ethers), polyethylene naphthalate, polyvinylidene chloride (PVDC), saran, ethylene vinyl alcohol copolymers, polyvinyl acetate, silicon oxide (SiOx), silicon dioxide (SiO₂), aluminum oxide (Al₂O₃), polyvinyl alcohol (PVOH), nanopolymers (e.g., nanoclay), polyimide thermoset film, EVALCA EVAL EF-XL, Hostaphan GN, Hostaphan RHBY, RHB MI, Techbarrier HX (SiOx-coated PET), Triad Silver (silver metallized PET), Oxyshield 2454, Bicor 84 AOH, acrylonitrile copolymers, and copolymers of terephthalic acid and isophthalic acid with ethylene glycol and at least one diol. Alternative gas-barrier materials include polyamine-polyepoxides. These materials are typically provided as a solvent-based or aqueous-based thermosetting composition and are typically spray-coated onto a preform and then heat-cured to form the finished barrier coating. Alternative gas barrier materials that can be applied as coatings to the volume-occupying subcomponent include metals such as silver or aluminum. Other materials that may be used to improve the gas impermeability of the volume occupying subcomponent include, but are not limited to, gold or any noble metal, PET coated with saran, and conformal coatings.

One method that is used in the packaging industry to delay diffusion of the inflation fluid is to thicken the material. Thickening the material is generally not desired, as the total composite wall thickness preferably does not exceed 0.004 inches (0.010 cm) in order for the balloon 10 to be foldable into the desired delivery container size for swallowing by a patient.

A multilayer polymer film that is able to withstand the gastric environment over the course of the usable life of the balloon 10 includes linear low-density polyethylene (LLDPE) adhesively bonded to a Nylon 12 film. Alternatively, an additional film layer with barrier properties, such as PVDC can be added to the composite wall.

The layers providing gas barrier properties are preferably situated as inner layers in the composite wall as they are less mechanically robust than resins that are considered "structural" such as Nylon and the like.

### Structural Layers

Layers such as polyurethane, Nylon, or polyethylene terephthalate (PET) can be added to the composite wall for structural purposes, and are preferably placed as outermost (proximal to the gastric environment or proximal to the central lumen of the balloon 10) layers, provided that the pH resistance of such layers can withstand the acidic environment of the stomach 20 or the central lumen of the balloon 10.

### Fabrication of the Composite Wall

The various layers of the composite wall, including the gas barrier layers, need not be situated in any particular order, but those of superior resistance to acidity, temperature, mechanical abrasion, and superior biocompatibility profile are preferably employed as layers contacting the gastric environment. Those with superior resistance to, e.g., acidity and temperature, are preferably employed as layers contacting the central lumen of the balloon 10.

The various layers of the wall can include a single layer or up to 10 or more different monolayers; however, a film thickness of from 0.001 inches (0.0254 cm) to 0.004 inches (0.010 cm) thick is desirable such that the resulting balloon 10 compacted to fit into a swallowable capsule. The resulting composite wall preferably has good performance specifications with respect to each category listed in Tables 1a-b.

Films that are co-extruded are advantageously employed, as some adhesives may contain leachables that are undesirable from a biocompatibility perspective. In addition, coextrusion allows for better blending such that the materials maintain their original properties when combined in this fashion and are less likely to be subject to delamination when exposed to gastric motility forces.

Combining films with similar properties, e.g., two film layers with excellent gas barrier properties, in a composite wall is advantageous for use in a gastric balloon 10 containing nitrogen, oxygen, CO₂ or a mixture thereof as the inflation gas or where the external environment the product is to be placed in, contains a mixture of gases including CO₂, e.g., the stomach. A primary advantage of such composite films is that restrictions on film thickness can be observed without sacrifice of gas barrier properties. Such a configuration also contributes to reducing the effects of processing damage (e.g., manufacturing and compacting) and damage due to exposure to in vivo conditions (e.g., gastric motility forces).

In a particularly embodiment, the composite wall includes a plurality of layers. The first layer is an outer protective layer that is configured for exposure to the gastric environment. This layer is resistant to mechanical forces, exposure to water (vapor), abrasion, and high acidity levels. Nylon or more specifically, Nylon 12 can advantageously be employed for the layer exposed to the gastric environment, and is especially resistant to mechanical forces.

In an alternative embodiment, polyurethane is RF welded to saran to yield a 6-7 mil thick composite wall. In another embodiment, a five-layer system is provided comprising a layer of saran sandwiched between two polyurethane layers. Between the saran layer and each of the polyurethane layers is a tie layer. The layers can be welded together, co-extruded or adhered using an adhesive. This tri-layer is then co-extruded to Nylon on each side, and then a final sealing layer (polyethylene or the like) is added to one of the nylon layers for the total composite wall. A representative example of material combinations that are commercially available or manufacturable is provided in Table 2. The orientation of the layers (innermost - in contact with the central balloon 10 lumen, or outermost - in contact with the gastric environment) is also indicated if more than two layers are described to support a suggested composite wall.

Most of the film resins listed in Table 2 provide some degree of gas barrier properties. Therefore, many can be used solely to form the balloon 10 wall as a monolayer film; however, they can also be used in conjunction with other film resins to meet the desired gas retention and mechanical specifications for the useful life of the balloon 10 based on the inflation gas and external environment the balloon 10 is to be placed in. These film resins can also be coated with gas barrier coatings listed in Tables 1a-b. Additional film layers can be added to form the total composite wall. While such additional layers may not impart substantial barrier properties, they can provide structural and/or mechanical properties, protection for the other layers of the composite wall that are susceptible to water vapor, humidity, pH, or the like, or other desirable properties. The film layers can be assembled using various adhesives, *via* co-extrusion, *via* lamination, and/or using tie layers and such to create a composite wall that meets the requirements of an intragastric balloon 10 suitable for use for at least 25 days, or up to 90 days or more, with the specified gas retention properties. Table 2 provides a list of layers and layer combinations suitable for use in composite walls for an intragastric balloon 10. The composite description, resin abbreviation, configuration (single layer, bilayer, trilayer, or the like) and trade name of commercially available combinations are listed. The number of layers indicated does not include any adhesive layers or tie layers used to fabricate the composite wall, such that a 6-layer composite wall may, for example, have two or three adhesive layers and/or tie layers that make up the total composite wall, and therefore the total number of layers can be eight or nine in final form. The term "layer" as used herein is a broad term, and is to be given its ordinary and customary meaning to a person of ordinary skill in the art (and is not to be limited to a special or customized meaning), and refers without limitation to a single thickness of a homogenous substance (e.g., a coating such as SiOx, or a layer such as PET), as well as to a supporting layer having a coating thereon (wherein a "coating" is, e.g., a material typically employed in conjunction with substrate that provides structural support to the coating layer). For example, a PET-SiOx "layer" is referred to herein, wherein a layer of Si-Ox is provided on a supporting PET layer.

**Table 2.**

| **Example Film Composite Walls*** | **Abbre*via*tion** | **Trade name** |
|---|---|---|
| polyethylene terephthalate | PET | Mylar |
| metallized oriented polyethylene terephthalate | metallized OPET | Custom |
| polyvinyl alcohol coated oriented polypropylene | PVOH coated OPP | Bicor |
| metallized biaxially oriented nylon 6 | metallized OPA6 | Custom |
| Biaxally oriented Nylon/ethylene vinyl alcohol/biaxally oriented Nylon | OPA/EVOH/OPA | Honeywell Oxyshield Plus |
| Nylon/ethylene vinyl alcohol/Low Density Polyethylene | Nylon/EVOH/LDPE | Custom |
| polyvinylidene chloride coated oriented polyethylene terephthalate | PVDC/ OPET | Mylar |
| polyvinylidene chloride coated oriented polypropylene | PVCD/OPP | Custom |
| polyvinylidene chloride coated biaxally oriented Nylon 6 | PVCD/OPA6 | Honeywell Oxyshield |
| high density polyethylene/ethylene vinyl alcohol | HDPE/EVOH | Custom |
| polypropylene/ethylene vinyl alcohol laminate | PP/EVOH | Custom |
| polyethylene terephthalate/ethylene vinyl alcohol | PET/EVOH | Custom |
| metallized oriented polypropylene | metallized OPP | Custom |
| sealable PVDC coated oriented polypropylene | PVDC coated PP | Custom |
| polyvinylidene fluoride | PVDF | Custom |
| Polyvinyl chloride | PVC | Custom |
| polyvinyl fluoride | PVF | Tedlar |
| polychlorofluoroethylene | PCTFE | ACLAR UltRx, SupRx, Rx |
| amine-based epoxy coated Nylon | epoxy coated PA6 | Bairocade |
| polyvinyl chloride-polyvinylidene chloride copolymer | PVC-PVDC | Custom |
| medium density polyethylene | MDPE | Custom |
| Nylon/Polypropylene | Nylon/PP laminate | Custom |
| Nylon-High Density Polyethylene | Nylon-HDPE laminate | Custom |
| Nylon 12/Ethyl Methyl Acrylate/Polyvinylidene Chloride/ Ethyl Methyl Acrylate /Nylon 12/Linear Low Density Polyethylene+Low Density Polyethylene | Co-extruded Nylon 12-encapsulated PVDC-Nylon 12-LLDPE+LDPE | Custom Co-extruded blend |
| Multi-layer Nylon 12/ Linear Low Density Polyethylene+Low Density Polyethylene | Co-extruded multi-layer Nylon 12-LLDPE+LDPE | Custom Co-Extruded Blend |
| acetylene plasma coating on polyester | PET/A | Custom |
| difluoroethylene coating on polyethylene terephthalate | PET/DA | Custom |
| oriented polypropylene | OPP | Custom |
| cast propylene | CPP | Custom |
| high density polyethylene | HDPE | Custom |
| cyclic olefin copolymer | COC | Custom |
| oriented polystyrene | OPS | Custom |
| Fluorinated Ethylene Propylene | FEP | Custom |
| difluoroethylene coating on low density polyethylene | LDPE/D | Custom |
| difluoroethylene coating on polypropylene | PP/D | Custom |
| acetylene plasma coating on polypropylene | PP/A | Custom |
| acetylene plasma coating on low density polyethylene | LDPE/A | Custom |
| polybutylene terephthalate polyether glycol copolymer | TPC-ET | Hytrel |
| polyether block amide TPE | PEBA | Pebax |
| oxide coated biaxally oriented Nylon | oxide coated PA | Honeywell Oxyshield Ultra |
| Nanoclay/ nylon | MXD6/Nanoclay | Imperm/ Aegis OXCE |
| Polyethylene Terephthalate/Silicone Dioxide | PET/SiOx | BestPET/ TechBarrier |
| Polyethylene Terephthalate/Oxygen scavengers | PET+02 Scavengers | MonoxBar |
| Modified Polyethylene Terephthalate | Modified PET | DiamondClear |
| Polyethylene Terephthalate/Nylon 6 | PET/MXD6 | HP867 |
| Amorphous polyvinyl alcohol | Amorphous PVOH | Nichigo G-Polymer |
| Nylon 6/ Ethyl vinyl alcohol/Linear Low Density Polyethylene | Nylon 6/ EVOH/LLDPE | Custom |
| Ethyl vinyl alcohol/ Poly-Propylene/ Ethyl vinyl alcohol | EVOH/PP/EVOH | Custom |
| Ethyl vinyl alcohol/Nylon | EVOH/Nylon | Custom |
| Polyethylene/ Ethyl vinyl alcohol/Polyethylene | PE/EVOH/PE | Custom |
| Polyethylene/ Ethyl vinyl alcohol/ Polyethylene Terephthalate | PE/EVOH/PET | Custom |
| Silicon dioxide-coated Polyethylene Terephthalate/Linear Low Density Polyethylene/ Ethyl vinyl alcohol/Linear Low Density Polyethylene | PET-SiOx/LLDPE/EVOH/LLDP E | Custom |
| Aluminum Oxide-coated Polyethylene Terephthalate/Polyethylene | PET-Al₂O₃/LLDPE | Custom |
| Polyethylene/ Ethyl vinyl alcohol/Linear Low Density Polyethylene | PE/EVOH/LLDPE | Custom |
| Polyethylene Terephthalate/ Polyethylene/ Polyethylene/ Bi-axially oriented Ethyl vinyl alcohol | PET/PE/OEVOH/PE | Custom |
| Polyethylene Terephthalate/ Polyethylene/Ethyl vinyl alcohol/ Ethyl vinyl alcohol/ Ethyl vinyl alcohol/ Polyethylene | PET/PE/EVOH/EVOH/EV OH/PE | Custom |
| Polyethylene Terephthalate/ Polyethylene/Nylon 6/Ethyl vinyl alcohol/ Nylon 6/ Polyethylene | PET/PE/Nylon 6/EVOH/Nylon 6/PE | Custom |
| Silicone dioxide-coated Polyethylene Terephthalate/ Polyethylene/ Ethyl vinyl alcohol/ Polyethylene | PET-SiOx/ PE/EVOH/PE | Custom |
| Polyethylene/Ethyl vinyl alcohol/polyvinylchloride | PE/EVOH/PVDC | Custom |
| Polyethylene Terephthalate/ Linear Low Density | PET/LLDPE/EVOH/LLDP | Custom |
| Polyethylene/Ethyl vinyl alcohol/ Linear Low Density Polyethylene | E | |
| Kurrarister C-coated Polyethylene Terephthalate /Polyethylene/ Ethyl vinyl alcohol/Polyethylene | PET-Kurrarister-C /PE/EVOH/PE | Custom |
| Polyethylene Terephthalate/ Polyethylene/Nylon 6/Ethyl vinyl alcohol/Nylon 6/Polyethylene | PET/PE/Nylon 6/EVOH/Nylon 6/PE | Custom |
| Nylon 6/Ethyl vinyl alcohol/ Polyvinylchloride/Low Density Polyethylene | Nylon 6/EVOH/ PVDC/Nylon 6/LDPE | Custom |
| Polyimide | PI | Custom |
| Polyimide/Linear Low Density Polyethylene | PI/LLDPE | Custom |
| Polyimide/Polyvinylchloride | PI/PVdC | Custom |
| Polyimide/Polyvinylchloride/ Linear Low Density Polyethylene | PI/P VdC/LLDPE | Custom |

In particularly embodiments, the composite wall has a thickness of 0.005 inches or less (5.0 mil or less); however, in certain embodiments a thicker composite wall may be acceptable. Generally, it is desireable that the composite wall have a thickness of no more than 0.004 inches (4.0 mil); however, thicker walls can also be employed.

### FABRICATION OF THE BALLOON

To ensure good mechanical strength of the balloon 10, the balloon 10 is preferably formed and sealed such that the edges of the pieces used to form the balloon 10 are overlapping. This can be accomplished by any suitable method. For example, two flat sheets of material can be placed in a frame with magnetized edges to hold the two sheets in place. Slack can be added to the piece of film to orient the material such that it maintains its properties after the forming process. The frame can be placed over a mold that represents a hemisphere the balloon 10. The material, with slack put in it prior to pressure being applied, re-orients the material such that it is more evenly distributed around the hemisphere shape. The material is preferably thickest in the middle and is made thinner on the sides where it will be welded to a second piece to create a sphere or ellipsoid having a substantially uniform wall thickness. For example, starting with a 0.0295" film, the middle of the film or subsequent apex has an ending film thickness of 0.0045" and the edges have an ending thickness of 0.0265" for subsequent overlapping during the welding process.

The valve can be adhered to the (e.g., polyethylene, PE) side of one of the hemispheres and protrude out of the opposite (e.g., nylon) side. One hemisphere typically consists of Nylon as the outermost layer and the second hemisphere typically has polyethylene (sealing web) as the outermost layer. The edges of the two hemispheres are preferably aligned such that they overlap by at least 1 mm and no more than 5 mm. Alignment and overlay of the two hemispheres is done to compensate for the thinning at the edges during the thermoforming process, which in turn inhibits seam bursts *in vivo.* Each half of the spheroid is placed on a fixture and the excess from the forming process is trimmed. On a multi-layer film, the sealing layer, a PE or similar layer is bonded to the sealing layer of the second film half. To do this the film of the hemisphere that has the nylon exposed to the external environment is folded up along the edges of the sphere on one half such that it can be bonded to the hemisphere with the polyethylene on the outermost layer.

The two film pieces are then sealed using a roller bonder or a band heater. In the roller bonder, a pneumatic cylinder provides the compression, the heater provides the sealing heat, and a motor that moves the bonder around the area controls the time that is required to ensure proper sealing. In the band heater, there is a heating element, an expandable plug that provides the compression, and a timer. The band is a metal, preferably copper and a spool-like fixture provides the compression needed. Using film layers of different melt temperatures helps ensure integrity of the barrier layers of the final balloon 10 configuration. If two similar materials are welded, then an insulator can be employed. In a embodiment, one sphere is provided with the Nylon layer facing out and the second sphere has a PE layer facing out.

### BALLOONS WITH RESISTANCE TO SPONTANEOUS DEFLATION

The largest percentage of intragastric balloon malfunctions is due to spontaneous deflations. Spontaneous deflations can occur due to (1) external puncture of the intragastric balloon 10 due to gastric motility forces, (2) over inflation of the balloon 10 due to increased internal pressure of the balloon 10 from uptake of the gastric environment of the gasses and water vapor and (3) under inflation of the balloon 10 that leads to fatiguing of the excess material and subsequent puncture of the balloon 10. By managing these two variables and tuning these variables to withstand the dynamic gastric environment, the balloon system can be tailored to ensure it remains inflated throughout its useful life. Instances of spontaneous deflation in this intragastric balloon 10 can be minimized by selection of the starting inflation gas in conjunction with selection of the composite wall materials and construction. Selection of the permeability characteristics with respect to water vapor transmission and gas permeability of the composite wall so as to take advantage of the properties of the gastric space contents can enable the rate of diffusion of gases into and out of the balloon 10 to be controlled. This method allows for a tunable method for prevention of under inflation and over inflation.

Another phenomenon seen with gastric balloons 10 and obesity in general is stomach accommodation. In the process of stomach accommodation, the stomach 20 grows to accommodate the space occupying device or excess food that is ingested. In the process of stomach accommodation, the volume of a stomach 20 containing an intragastric balloon 10 grows over time, such that the patient becomes hungrier. However, by controlling gas diffusion and water vapor transmission across the balloon wall over time, the balloon size can also be increased over time by selecting the starting inflation gas(es) and water and other in vivo gas permeability characteristics of the film so as to maintain weight loss. In addition to spontaneous deflations, selecting the permeability characteristics of the composite wall in conjunction with the starting gases and utilizing the transfer of gases and water inside of the balloon 10 from the gastric environment, the balloon 10 can be designed to grow over its useful life in response to stomach accommodation.

Experiments were performed wherein various starting inflation gases were selected in conjunction with varying external gas environments that mimic the stomach gas and water environment *in vivo.* The stomach environment consists of water, acid (hydrochloric acid), a mixture of gases, and chyme (the semifluid mass of partly digested food expelled by the stomach 20 into the duodenum). Stomach gas usually arises from swallowing air during eating. The composition of air is nitrogen (N₂) 78.084%; oxygen (O₂) 20.9476%; argon (Ar) 0.934%; carbon dioxide (CO₂) 0.0314%; neon (Ne) 0.001818%; methane (CH₄) 0.0002%; helium (He) 0.000524%; krypton (Kr) 0.000114%; hydrogen (H₂) 0.00005%; and xenon (Xe) 0.0000087%.

Five gases constitute greater than 99% of the gases in gastrointestinal system: N₂, O₂, CO₂, H₂ and methane, with nitrogen predominating. Gastric pCO₂ closely parallels local (splanchnic) arterial and draining venous blood pCO₂ values. Neutralization of stomach acid can also generate gas. For example, when the stomach acid reacts with bicarbonates (e.g., as are present in certain antacids) in the digestive juices, the chemical process creates CO₂, which is normally absorbed into the blood stream. Digestion of food in the intestines, mainly through fermentation by colonic bacteria, generates CO₂, H₂, and methane. Microbes appear to be the sole source of all of the hydrogen and methane produced in the intestine. These arise from fermentation and digestion of nutrients (polysaccharides from fruits and vegetables are not digested in the small intestines). Small quantities of a few other gases, including hydrogen sulfide, indoles, and ammonia can also be generated.

Controlled self-inflation of the intragastric balloon 10 in the *in vivo* environment can be achieved by using a semi-permeable or permeable composite wall in the balloon 10 and initially filling the balloon 10 with a preselected single gas, such as N₂ or O₂. The balloon 10 utilizes differences in concentrations of gases and water concentration differences between the internal balloon 10 environment and the external environment *in vivo* (GI/stomach) to increase and/or decrease the volume and/or pressure over time. To achieve a controlled decrease in volume and/or pressure, a wall can be employed that has a relatively higher permeability to the single gas used to inflate the balloon 10 than to other gases present in the *in vivo* gastrointestinal environment. For example, if nitrogen gas is employed as the inflation gas, over time in the *in vivo* environment, the volume and/or pressure in the balloon 10 will decrease as nitrogen diffuses out into the *in vivo* environment through the oxygen permeable wall. Similarly, if oxygen gas is employed as the inflation gas, over time in the *in vivo* environment, the volume and/or pressure in the balloon 10 will decrease as oxygen diffuses out into the *in vivo* environment through the oxygen permeable wall. The differential in partial pressure of the single gas in the balloon 10 (higher) versus the *in vivo* environment (lower) will drive the process until equilibrium or homeostasis is reached. To achieve a controlled increase in volume and/or pressure, a wall can be employed that has a relatively lower permeability to the single gas used to inflate the balloon 10 than to other gases present in the *in vivo* gastrointestinal environment. For example, if nitrogen gas is employed as the inflation gas, over time in the *in vivo* environment, the volume and/or pressure in the balloon 10 will increase as CO₂, etc. diffuses into the balloon 10 through the CO₂ permeable wall. The differential in partial pressure of the permeable gas in the balloon 10 (lower) *versus* the *in vivo* environment (higher) will drive the process until equilibrium is reached.

In addition, maintaining and/or controlling inflation of the balloon 10 can also be done using the differences in concentrations between the internal balloon 10 environment and external gastric environment in which the balloon 10 volume/pressure can be increased or decreased as needed to extend the useful life of the product. One reason to decrease the pressure can be to first inflate the balloon 10 with a large, but highly diffusible/soluble gas molecule such as CO₂ in addition to a more inert gas like nitrogen to pre-stretch the balloon, with the soluble gas diffusing out of the balloon 10 and other gases not originally present in the balloon 10 migrating in to fill the balloon.

Inflation gases can be selected to start with the majority of the gas in the balloon 10 comprising a large, inert gas or a gas that has low diffusivity through the selected composite wall. An inert gas in conjunction with a less inert gas(es) that are more soluble in the gastric environment, can be combined to comprise the starting balloon 10 inflation gas composition. Patient diet and medications can also affect/control balloon 10 inflation status - primarily by CO₂ concentration effects produced in the gastric environment. In addition, gastric pH also affects CO₂ concentration. This particular method can also allow for a greater degree of tuning of the device's useful life based on the composite wall material, e.g., barrier/non-barrier and whether the gas that diffuses in is maintained longer in the balloon 10 if it has a barrier wall versus a non-barrier wall. This particular form of self-inflation can be employed using a self-inflating gastric balloon 10 (e.g., initially inflated by a gas generating reaction in the balloon 10 initiated after swallowing), or an inflatable gastric balloon 10 (e.g., inflated using a catheter, with or without endoscopic assistance, delivered nasogastrically or any other delivery method). The method can be used with any gastric balloon, including swallowable balloons and balloons placed in the stomach 20 by, e.g., endoscopic methods. The method can advantageously be employed for use in connection with intragastric devices; however, it can also be applied to use in, e.g., pulmonary wedge catheters and urinary incontinence balloon 10 devices. The advantages to this technology include the ability to compensate for stomach accommodation, allowing the balloon 10 to adapt to a stomach 20 that may increase in volume over time, thereby maintaining patient satiety. It also permits starting with a smaller amount of inflation gas constituents for a self-inflating balloon. It can prevent spontaneous deflations by utilizing diffusion gradients between gastric balloon systems and the *in vivo* gastric environment.

In a particularly embodiment, used in connection with N₂ (with or without CO₂) as the inflation agent, a multi-layer co-extruded blend for the wall layers is employed. One configuration is Nylon 12/Ethyl Methyl Acrylate/Polyvinylidene Chloride/ Ethyl Methyl Acrylate /Nylon 12/Linear Low-Density Polyethylene+Low Density Polyethylene (also referred to as co-extruded Nylon 12-encapsulated PVDC-Nylon 12-LLDPE+LDPE multilayer). Another configuration is a co-extruded multi-layer Nylon 12/Linear Low-Density Polyethylene+Low Density Polyethylene. Selection of the resins for the composite wall construction (as well as selection of using a coextrusion method or adhesives) can be varied to control compliance (stretchiness), puncture resistance, thickness, adhesion, sealing bond strength, orientation, acid resistance, and permeability characteristics to gasses and water vapor to achieve a particular effect.

### AUTOMATIC SELF-DEFLATION OF INTRAGASTRIC BALLOON SYSTEMS

In some embodiments, the intragastric balloon 10 is provided with mechanisms to reliably control timing of deflation, also referred to as auto-deflation or self-deflation. In embodiments, the balloon 10 auto-deflates and passes through the stomach, through the lower gastrointestinal tract, and out of the body at the end of its pre-determined useful life (non-spontaneous), such as, but not limited to, about 30 days, about 60 days or about 90 days. For example, in one embodiment, the balloon is configured to reside in the patient's stomach for about 3-months, and then to auto-deflate or self-deflate to a size and shape that passes through the patient's pylorus into the intestines; after passing through the intestines, the deflated balloon passes from the patient's digestive system *via* normal elimination processes. In some embodiments, the pre-determined useful life of the balloon is at least 90 days, at least 120 days or at least 180 days. In embodiments, the pre-determined useful life of the balloon is about 7-months, about 8-months, about 9-months, about 10-months, about 11-months or about 12-months. In more embodiments, the balloon's predetermined useful life is greater than 12-months. In the embodiments described below, the timing of deflation can be accomplished *via* the external gastric environment (by conditions of temperature, humidity, solubility, and/or pH, for example) or *via* the environment within the lumen of the inflated balloon. It is preferable for consistency to control the initiation of the self-deflation process by manipulating the internal balloon environment.

In some embodiments, the self-deflation mechanism includes an erodible material, such as one or more patches or other structures added to the balloon during construction. Such degradable or erodible patches are made out of an erodible, degradable, or dissolvable material (natural or synthetic) and are incorporated into the wall of the balloon. The patch(s) are of sufficient size to ensure opening of a sufficient surface area to cause rapid deflation, and to prevent re-inflation by seepage of stomach fluid into the balloon. The balloon patch(s) comprise materials that can be applied to the balloon such that a substantially smooth surface is maintained, and preferably comprise a single layer or multilayered material. The patch(s) are constructed using an erodible, disintegrable, degradable or other such material that is preferably tissue-compatible and degrades into non-toxic products or is a material that slowly hydrolyzes and/or dissolves over time (e.g., poly(lactic-*co-*glycolic acid) (PLGA), poly(lactide-co-glycolide) (PLG), polyglycolic acid (PGA), polycaprolactone (PCL), polyesteramide (PEA), polyhydroxyalkanoate (PHBV), polybutylene succinate adipate (PBSA), aromatic copolyesters (PBAT), poly(lactide-co-caprolactone) (PLCL), polyvinyl alcohol (PVOH), polylactic acid (PLA), poly-L-lactic acid PLAA, pullulan, polyethylene glycol (PEG), polyanhydrides, polyorthoesters, polyaryletherketones (PEEK), multi-block polyetheresters, poliglecaprone, polydioxanone, polytrimethylene carbonate, and other similar materials). These erodible, disintegrable, or degradable materials can be used alone, or in combination with other materials, or can be cast into/co-extruded, laminated, and/or dip coated in conjunction with non-erodible polymers (e.g., PET or the like) and employed in the construction of the balloon. Degradation/erosion occurs, is initiated by, and/or is controlled by the gastric environment (e.g., by conditions of temperature, humidity, solubility, and/or pH, for example), or is controlled within the lumen of the balloon (e.g., by conditions of humidity and/or derived pH, for example) based on what the patch is exposed to. Thickness of the polymer as well as environment which affects degradation and time of exposure can also facilitate degradation timing. Degradation/erosion are timed such that they occur once the pre-determined balloon useful life is completed (e.g., inflation is maintained for from 25 to 90 days *in vivo* in the stomach before degradation/erosion results in formation of an opening permitting deflation). As an alternative to (or in connection with) using a degradable material for the patch, the patch can comprise a similar fluid retention barrier film or the same film as the remaining wall of the balloon which is adhered to the balloon using a weak adhesive, or welded or adhered such that after a specified amount of time the patch delaminates from the applied area and allows for an opening for inflation fluid release for deflation. Or if deemed necessary for rapid deflation the entire balloon composite wall can be made of the erodible material. The mechanism of using an erodible material or a material that mechanically fails after a prespecified time is be similar for all embodiments for deflation mechanisms described below as well. The timing of degradation or erosion can be controlled using the external gastric environment (e.g., by conditions of temperature, humidity, solubility, and/or pH, for example) and/or can be controlled by conditions within the lumen of the balloon (e.g., by conditions of humidity and/or pH of residual liquid in the balloon).

In other embodiments, self-deflation mechanism includes a plug or plugs (optionally in conjunction another degradable retaining structure) incorporated into the balloon construction and can consist, all or in part, of an erodible, disintegrable, or otherwise degradable synthetic or natural polymer similar to those described above (e.g., PLGA, PLAA, PEG, or the like). The plug can be formed into various shapes (e.g., cylinder shape or radial shape) to achieve various surface-to-volume ratios so as to provide a preselected and predictable bulk degradation pattern for the erodible polymer. The plug can incorporate a releasing mechanism that can be chemically initiated after degradation/erosion begins, such that the septum or plug material pops out of the balloon or falls inside of the balloon, thereby creating a passageway for fluid release and subsequent deflation of the balloon. Mechanical additions that can be used in conjunction with a plug include a degradable/erodible/disintegrable material that holds a plug (e.g., of a non-degradable or degradable material) in place or a compressed spring housed within the retaining structure or plug structure. More specifically one embodiment to achieve deflation can comprise a housing, a radial seal, a solid eroding core, and a protective film attached to the external surface of the eroding core. The inside of the eroding core is exposed to the internal balloon liquid. The core creates a compressive force that holds the seal against the housing. As the core erodes, the compression between the housing and the radial seal is reduced until there is clearance between the housing and the seal. Once there is clearance, gas can move freely from the inside of the balloon to the outside environment. The seal can fall out of the housing and into the balloon. The diameter, length, and material types can be adjusted in order to create the deflation at a desired time point. Example materials for each component used to achieve this deflation mechanism can be as follows. Housing - biocompatible structural material, capable of withstanding enough radial force to form an air tight seal. Materials can include polyethylene, polypropylene, polyurethane, UHMWPE, titanium, stainless steel, cobalt chrome, PEEK, or nylon. Radial Seal - composed of a biocompatible elastic material, capable of providing liquid and gas barrier to acidic environments. Materials can include silicon, polyurethane, and latex. Eroding Core - a material capable of breaking down at a predictable rate at given environmental conditions. Materials can include PLGA, PLA, or other polyanhydrides that are capable of losing integrity over time or any materials listed above that provide erodible characteristics.

For the spring mechanism, once the material degrades, the spring is released and/or the plug/septum is pulled into the balloon or pushed out of the balloon, thus releasing fluid once an orifice has been created by release of the spring mechanism and pushing out or pulling in of the plug.

Deflation mechanisms utilizing a septum and moisture absorbing expansion material and a moisture eroding material. The eroding materials slowly erode away when exposed to moisture, eventually exposing the moisture absorbing expansion material. When the moisture-expanding material begins to absorb moisture, the expansion pulls the septum out of position in the head by pushing against a septum lip or a ring attached to the septum. Pulling the septum out of position causes an immediate deflation of the balloon. In order to protect the expanding material from moisture until a desired timepoint, the expanding material can be sheathed in water blocking materials, such as parylene, as well as slowly water degrading materials. The moisture contact can be controlled by small inlet ports. The inlet ports can be small holes, or a wick material that draws moisture in a controlled manner. The desired deflation time is achieved through a combination of eroding materials, blocking materials, and inlet port sizing.

In certain embodiments, the balloon can incorporate one or more plugs in the wall of the balloon that contain a compressed pellet or gas releasing pellet. The pellet can be comprised of any combination of constituents that, when activated, emit CO₂ gas (e.g., sodium bicarbonate and citric acid, or potassium bicarbonate and citric acid, or the like). The pellet can be in tablet or rod form protected by an erodible, disintegrable, or degradable material that is preferably tissue-compatible and degrades into non-toxic products or that slowly hydrolyzes and/or dissolves similarly to the plugs and patches described above (e.g., poly(lactic-co-glycolic acid) (PLGA), polyvinyl alcohol (PVOH), polylactic acid (PLA), poly-L-lactic acid PLAA, Pullulan, Polyethylene Glycol, polyanhydrides, polyorthoesters, polyaryletherketones (PEEK), multi-block polyetheresters, poliglecaprone, polydioxanone, polytrimethylene carbonate, and other like materials). Degradation/erosion of the plug initiates the reaction of the two chemicals in the pellet and subsequently leads to formation of gas (e.g., CO₂). As sufficient gas is trapped or built up, sufficient pressure is eventually generated to push out the softened polymer material and create a larger channel for the CO₂ gas in the balloon to escape. External pressure applied by the stomach to the balloon (e.g., squeezing) can contribute to the process of creating a larger channel. Dimensions and properties of the plug (diameter, thickness, composition, molecular weight, etc.) comprised of the polymer drives the timing of degradation.

In other embodiments, plugs or patches of different shapes or sizes similar to those of the plugs described above can be employed within the balloon lumen in a multi-layer configuration including a semi-permeable membrane to facilitate balloon deflation. The plug or patch is made of similar degradable/erodible/dissolvable material as described above (e.g., poly(lactic-co-glycolic acid) (PLGA), polyvinyl alcohol (PVOH), polylactic acid (PLA), PLAA, pullulan, and other like materials) and contains a compartment enclosed by a semi-permeable membrane (impermeable to an osmolyte) that contains a concentrated solution of a solute or osmolyte (such as glucose, sucrose, other sugars, salts, or combination thereof). Once the plug or patch begins to degrade or erode, the water molecules move by osmosis down the water gradient from the region of greater water concentration to the region of lower water concentration across the semi-permeable membrane into the hypertonic solution in the compartment. The compartment containing the osmolyte swells and eventually bursts, pushing the membranes and the degraded plug or patch out, thereby allowing rapid gas loss through the newly created channels or areas.

In certain embodiments, a balloon composed of a septum, moisture eroding material inside an inlet port, and moisture absorbing expansion material is employed. The eroding materials slowly erode away when exposed to moisture, eventually exposing the moisture absorbing expansion material. When the moisture-expanding material begins to absorb moisture, the expansion pulls the septum out of position in the head by pushing against a septum lip or a ring attached to the septum. Pulling the septum out of position causes an immediate deflation of the balloon. In order to protect the expanding material from moisture until a desired time point has been reached, the expanding material can be sheathed in water blocking materials, such as parylene, as well as slowly water degrading materials. The moisture contact can be controlled by small inlet ports. The inlet ports can be small holes, or a wick material that draws moisture in a controlled manner. The desired deflation time is achieved through a combination of eroding materials, blocking materials, and inlet port sizing.

Another mechanism for self-deflation is to create a forced de-lamination scheme, which can provide a larger surface area to ensure rapid deflation. In, e.g., a balloon having a tri-layer wall, the outermost layer is substantially strong enough to hold the inflation fluid (e.g., polyethylene terephthalate (PET) or the like), the middle layer is comprised entirely of an erodible material (e.g., PVOH or the like) while the inner layer is comprised of a weaker material (e.g., polyethylene (PE) or the like). The PET or outermost layer is "scored" or hatched with erodible material to create small channels that erode over time (FIG. 23). This creates channels such that the gastric fluid seeps into the balloon layers and starts degrading the fully erodible material. When the erodible layer degrades or dissolves, the material that composes the innermost layer also erodes, degrades or dissolves since it is not strong enough to withstand the gastric forces/environment on its own. The balloon then collapses on itself and eventually passes through the lower gastrointestinal tract. Having an erodible layer sandwiched between a strong and weak layer facilitates timing of erosion by creating a longer path length than an erodible plug or patch affected by the gastric environment. The distance between scores or openings can also be selected so as to provide a desired deflation rate.

In another embodiment providing abrupt deflation of the balloon after a desired period of time has elapsed, the composite wall of the entire balloon or a section of the composite wall (patch) includes several material layers that are slowly penetrated by water that has been injected inside the balloon during the manufacturing process or during the inflation process. This water penetrates through the layers, eventually reaching a material that substantially expands, rupturing a thin external protective later, and creating a large hole for gas to escape and the balloon to deflate. The water expanding material is protected from liquid via a coating or sheath, such as parylene, which allows a controllable amount of moisture exposure. Once water reaches the expansion material, it exerts a force on the protective outer layer, causing it to rupture. The outer layer may be created with a weakened bonding area, a partially scored area, or other methods of ensuring a desired rupture location and to facilitate desired timing for auto-deflation to take place. There can be any number of layers between the moist environment and the moisture expanding center. Each material layer can have different erosion rates (e.g., fast or slow) and can be selected by the predetermined time deflation is desired to occur (e.g., after 30 days, 60 days, 90 days, or more). By varying the number, thickness, and rate of each of the circumferential layers, the time to deflation can be accurately controlled.

Alternatively, a pressure sealing button that is adhesively bonded over a perforation in the balloon material can be provided for deflation. The adhesive bonding the button erodes over time when it comes into contact with moisture derived from the gastric fluid or that has been injected inside the balloon. Once the adhesive can no longer bond and create an airtight seal between the adhesive and the button, the balloon will rapidly deflate. By controlling the hole size and moisture exposure of the adhesive, the erosion time can be accurately predicted.

Deflation can also be facilitated by creating a series of connecting ports within the septum or on another similar structure attached to the balloon composite wall. The ports can be constructed using a water-dissolving or acid-dissolving, biologically compatible, low permeability substance, such as gelatin. The diameter of the hole, number of holes, channel width, and channel length can all be adjusted to control the dissolving parameters. Once the material in the ports and channel is dissolved, there is a clear path for gas trapped in the balloon to escape, eventually resulting in a deflated balloon. The water can be gastric fluid or controlled internally by including water inside the balloon at assembly or during the inflation process. There can be a plurality of port openings to guarantee gas transmits. Additionally, there are several variables that can be adjusted to control dissolution time: size of the port openings; number of port openings; the length of the internal channel; the width of the internal channel; and the rate of material dissolution. The port/channel layout design can ensure that only a small amount of surface area is exposed to moisture at any particular time, thereby controlling the rate of erosion and ultimately deflation. In an alternative embodiment, an expandable material is employed to displace a push out component so as to initiate deflation.

A embodiment of manually inflated balloon that also possesses a mechanism for self-deflation would be a port that encompasses an inflation and deflation mechanism in the same location. The device comprises a catheter needle sleeve, e.g., of nylon or plastic that seals to silicon parts, that is attached to inflation tube when filling. It further comprises a silicon head which seals to the needle sleeve, allowing for inflation and detachment from the catheter. The silicon head also seals to part # 6 until pushed out of position by expanding part #7. The needle, e.g., fabricated from stainless steel, inflates the balloon. A compression Seal between part #6 and #2 vents internal gas when displaced. An insert, e.g., of titanium, provides imaging visibility, and provides rigid support for parts #2 and #4, and interference locks, sliding fits, and press fits to part #6. A septum, e.g., of silicon, seals to part #3 during inflation. The casing, e.g., PEEK or hard plastic, bonds to the balloon outer film and provides a sealing surface to part #2. It contains vents from inside the balloon to outside the balloon after part #7 expands. The expanding device, e.g., polyacrylamide in a binder material surrounded by a controlled moisture vapor transmission rate material (assorted blends of polyurethanes in varying thicknesses) uses the moisture available inside the balloon to uptake and swell in size. The press fit between parts #5 and #6 holds the parts firmly in position until part #7 begins to expand from moisture uptake.

In embodiments, a self-sealing valve is included that is compatible with an inflation catheter that contains a needle and needle sleeve. The self-sealing valve is sealed to the needle sleeve during the inflation process. Distal to the self-sealing valve is a titanium, stainless steel, MP35N, or any other radio-opaque rigid material insert that provides imaging visibility as well as mechanical support during the inflation process. Beneath the insert is the deflation mechanism that consists of an expanding device. The expanding device consists of a solute material, i.e. polyacrylamide material or the like encased in a binder material surrounded by moisture limiting material that has a defined moisture vapor transmission rate (MVTR). Moisture rate limiting material examples include but are not limited to assorted blends of polyurethanes in varying thicknesses. A hard plastic casing, such as PEEK, encompasses the self-sealing valves, the radio-opaque insert, the expanding material, and the moisture rate limiting material. The hard plastic case contains vents that would allow fluid to flow between the inside and outside of the balloon if the outer seal were not in position. The radio-opaque insert is coupled to the hard plastic casing via mechanical means, such as a press fit, that allow for linear movement but do not allow it to expel from the hard plastic casing. A second outer sealing valve creates an airtight seal to the hard plastic casing, blocking the casing vents, and moves linearly as the expanding device gains volume. Moisture placed inside of the balloon is absorbed by the expanding device as well as contributing moisture from the external gastric environment. Once the moisture transfers, the expanding material develops enough pressure such that the outer sealing valve is pushed linearly past the lip of the casing. This opens a vent pathway that allows the internal inflation fluid to quickly decompress and deflate the balloon. A deflated balloon allows for passing through the pylorus and through the remainder of the alimentary canal. One or multiple inflation/deflation ports on the surface of the balloon can be employed.

An alternative embodiment where the inflation port and deflation port are separate entities may be provided. The device comprises a seal, e.g., of Buna rubber or similar sealing material, to provide an airtight seal between parts #1 and #3. It slides along the surface of part #3 until airtight seal fails and allows internal air out. The vent allows gas to flow from the balloon once the seals displaces. Also included are a titanium plunger, a water retainer (cotton or sponge-like material that is capable of retaining water and holding it against the surface of part #4 in order to maintain a constant moisture environment) and a casing of PEEK or other hard material that seals via adhesive to the balloon film and provides rigid containment for parts #1, 2, 4, and 5. The design also allows venting between internal and external balloon environment, and water ingress to part #4 which forces part #4 to expand in one direction. An expanding device, polyacrylamide in a binder material surrounded by a controlled moisture vapor transmission rate material (assorted blends of polyurethanes in varying thicknesses) uses the moisture available inside the balloon to uptake The device can include a hard outer casing made of hard plastic or metal, an expanding device consisting of a super absorbent core surrounded by a moisture vapor transmission rate limiting membrane, and an airtight seal that is able to move linearly while the moisture expanding device grows in volume. The expanding device expands at a given rate based on how much moisture is available to it. In order to control the rate of expansion, a membrane, such as polyurethane, is used to control the desired moisture vapor transmission rate that is available to the super absorbent device. The moisture vapor transmission rate can be tuned by material formulation or material thickness. In order to maintain constant moisture-contact to the moisture vapor limiting membrane, a sponge like material, such as cotton, can be used as a moisture reservoir for the expanding device. Once the expanding device pushes the seal past the lip of the hard outer casing, fluid can vent from inside the balloon to the external environment, causing the balloon to deflate and pass through the pylorus and the remainder of the alimentary canal. The balloon can have at least one deflation port but may have as many as deemed necessary to deflate the balloon such that it completely deflates and no residual inflation fluid remains that would cause a bowel obstruction (i.e., partial deflation).

A mechanism to facilitate passing involves an erosion mechanism that allows for the balloon to be broken down into a size that has a higher probability of predictably passing through the lower gastrointestinal system. Preferably, the size of the balloon as deflated is less than 5cm long and 2 cm thick (similar to various foreign objects of similar size that have been shown to pass predictably and easily through the pyloric sphincter). This can be accomplished by providing the balloon with "erodible seams." One seam that breaks the balloon open into (at a minimum) two halves, or more seams are provided so that a plurality of smaller balloon pieces is produced in the dissociation reaction. The number of seams used can be selected based on the original surface area of the balloon and what is required to dissociate the balloon into pieces that are of a size that can predictably pass through the gastrointestinal tract more easily. The rate of seam erosion can be controlled by using a material affected by, e.g., the external gastric environment pH, liquid, humidity, temperature, or a combination thereof. Seams can be single layer consisting of only erodible material, or multi-layer. The timing of self-deflation can be further controlled by the design of the seam layers, e.g., making the reaction and/or degradation of the seam material dependent on the internal environment of the balloon instead of the external environment. By manipulating the reaction such that erosion or degradation is initiated by the internal environment (e.g., the balloon's internal pH, humidity, or other factors), any impact of person-to-person gastric variability (pH, etc.) that can affect erosion timing is minimized. The internal balloon environment can be manipulated by adding excess water at injection to create a more humid internal environment, or the quantity of constituents added can be varied to manipulate the pH, etc.

FIGS. 14-18 illustrate exemplary embodiments of a self-sealing valve 500 with a head assembly 510 and a deflation subcomponent 509, for use with the intragastric balloon 10. The self-sealing valve 500 includes a head unit 510 with a longitudinal axis C, and is preferably formed of a polymer such as described elsewhere herein. In the illustrated exemplary embodiment, the head unit 510 includes a top portion 512 and a bottom portion 513. The top portion 512 includes an annular top surface 516 with a top opening 518 defined by a top edge 520. The bottom portion 513 includes a bottom surface 522 with a bottom opening 524 defined by a bottom edge 526. The head unit 510 includes an outer surface 528 that joins the top surface 516 with the bottom surface 522. In the exemplary illustrated embodiment, the outer surface 528, or top portion 512, includes an optional shoulder portion 530. The optional shoulder portion 530 provides for a larger top surface 516 while simultaneously enabling minimization of the overall size of the valve system 500. The larger top surface 516 facilitates secure attachment of the head unit 510 to the inner surface 432 of the balloon wall 434, such as described elsewhere herein. For example, the valve system 500 may be adhered to the balloon inner surface 432 with an adhesive 435. At the same time, the smaller size of the valve system 500 allows for tighter folding of the intragastric balloon during manufacture, such that the folded balloon can be inserted into a smaller outer container or capsule 40. Smaller capsule size reduces swallowing difficulties of the patient receiving the balloon.

Similar to the self-sealing valve 440 described above, the top and bottom openings, 520 and 526 respectively, are joined by a coaxial through-channel 546. The through-channel includes a cylindrical inner surface 548 and is sized and shaped so as to receive the inner cylinder 505b of the retaining structure 505 therein, such that the inner cylinder 505b and head unit 510 are coaxial. In some embodiments, the through-channel 546 includes a ring-channel 550, which may be shaped as a radial groove, cavity, duct, bulge or gap. In embodiments, the ring-channel 550 is sized and shaped to cooperatively receive therein the bottom rim 220a of the bell-shaped needle sleeve 220. Preferably, the through-channel 546 is sized and shaped so as to center the bell-shaped needle sleeve 220 during engagement with the valve system 500, such that the needle-sleeve 220 is coaxial received within the valve system 500 and the hollow needle 230 pierces a self-sealing septum 514 substantially along the longitudinal axis C.

A metallic retaining structure 505, which is substantially similar in structure and function to the retaining structure 405 described above, is received coaxially into the head unit 510. Accordingly, the retaining structure 505 includes concentric, coaxial outer and inner cylinders, 505a and 505b respectfully, joined near the rear 513 of the valve 500 by a lower wall 505e. The lower wall 505e includes an upper surface 505f that faces the top opening 520 of the valve 500. The lower surface 505g faces the bottom opening 524. The inner cylinder 505b includes an inner surface 568 that defines an inner channel 569. The inner channel 569 is in fluid communication with the balloon lumen, so that when the needle 230 of the catheter 200 pierces the septum 514, the inflation fluid flows into the balloon lumen. The inner cylinder 505b also includes an outer surface 570, and an inner lip 505c that curves toward the axis C and defines an upper opening 574. A lower opening 575 joins the inner cylinder inner surface 568 with the lower wall lower surface 505g, whereby the inner channel 569 is fluidly joined, fluidly connected or in fluid communication with the lumen or interior of the balloon 10. In the illustrated embodiment, the retaining structure outer cylinder 505a includes a diameter D3 that is equal to the diameter D2 of the head unit bottom opening 524 (see FIG. 14). This configuration enables the retaining structure 505 to be slipped into the head 510 during valve assembly. Additionally, the retaining structure 505 can be dislodged when the deflation component 509 is actuated. In some circumstances, the entire retaining structure 505 may fall into the balloon lumen, to create an enlarged hole through the balloon wall, as is described in greater detail below. However, it is foreseen that the balloon may be deflated by only partially dislodging the retaining structure 505 from the valve 500.

In some embodiments, the valve 500 includes a self-sealing septum 514 that is compressed against the inner surface 568 of the lip 505c by a ring-stop 516. Compressing the self-sealing septum 514 into the opening 574 ensures that, when the needle 230 is removed from the septum 514, a gas-tight seal is formed and leakage of inflation fluid out of the balloon is prevented. In some circumstances, a portion of the septum 514 may extend or protrude through the opening 574. In the illustrated embodiment, the inner cylinder 505b includes a length selected such that the self-sealing septum 514 is retained wholly within the balloon 10. However, it is foreseen that the length of the inner cylinder 505b may be greater, such that the top 578 of the self-sealing septum 514 is either flush with or extends above the balloon outer surface 480.

Similar to that described above, the valve system 500 includes a connection channel 582 that is sized and shaped to receive and engage the bell-shaped needle sleeve 220. The connection channel 582 is defined by the through-channel inner surface 548, the lower wall upper surface 505f, and the inner cylinder outer surface 570, such that the connection channel 582 is generally cylindrical bore that extends from the upper opening 518 to the bottom wall 505e of the retaining structure 505. The distance between D4 the head unit inner surface 548 and the inner cylinder outer surface 570 is selected such that the bell-shaped needle sleeve 220 may be received therein. For example, similar to that described above, the needle-sleeve bottom rim 220a can abut, contact of engage the lower wall upper surface 505e and engage the ring-channel 550 so as to provide and/or contribute to an interference fit between the needle sleeve 220 and the self-sealing valve 500. A metallic retainer-ring 515 may be embedded in the head 510, so as to facilitate frictional engagement and the interference fit, such as described with respect to the valve 400.

As noted above, the self-sealing valve system 500 includes a deflation subcomponent 509. The deflation subcomponent 509 is configured to enable evacuation or release inflation fluid from the balloon lumen after passage of a pre-determined or preselected time, and thereby deflate the intragastric balloon 10. In embodiments, upon actuation of the deflation subcomponent, a substantial portion of the inflation fluid is evacuated from the balloon lumen. For example, in some embodiments, at least 75%, at least 80%, at least 90% or at least 95% of the inflation fluid is evacuated from the balloon lumen. In embodiments, substantially all of the inflation fluid is evacuated from the balloon lumen when the deflation subcomponent 509 is actuated. In more embodiments, the amount of inflation fluid evacuated from the balloon lumen deflates the balloon 10 to a size and/or configuration that enables passage of the deflated balloon through the alimentary canal without substantial discomfort to the patient.

In the illustrated embodiment, actuation of the deflation subcomponent 509 occurs at a predetermined or preselected time after administration of the balloon to the patient. This time period is preferably the length of the treatment period. For example, in some embodiments, the preselected period of time, or treatment period, is at least 30-days, at least 60-days or at least 90-days. In a embodiment, the treatment period is at least 6-months long. In a more embodiment, the treatment period is at least 9-months long, at least 12-months long, or even longer.

Various exemplary embodiments of the deflation subcomponent 509 are illustrated in FIGS. 14-19. The deflation subcomponent 509 provides a releasable or breakable seal against release or leakage of inflation fluid through the valve 500. In some embodiments, wherein the inflation fluid is a gas, the seal is an air-tight or gas-tight seal. In other embodiments, wherein the inflation fluid is a liquid, the seal is liquid-tight. The deflation subcomponent 509 includes two configurations. In a first configuration, such as is shown in FIG. 14, the deflation subcomponent 509 is closed, sealed or substantially blocked against a flow of inflation fluid therethrough. In the sealed configuration (FIG. 14) the sealing member 586 fills, compresses or collapses the vent member 584, so as to provide at least a portion of the deflation subcomponent 509 that is closed to the flow of inflation fluid therethrough. In a second configuration, such as is shown in FIGS. 15A and 15B, the deflation subcomponent 509 is unsealed, open, unblocked or released so as to enable a flow of inflation fluid (e.g., arrows 590). The deflation subcomponent 509 is configured so as to be able to transition from the sealed configuration (FIG. 14) to the open configuration (FIGS. 15A-15B) upon actuation of the deflation subcomponent 509. When the deflation subcomponent 509 is in the open configuration, inflation fluid can flow, such as is illustrated by the dashed arrows 590 of FIGS. 15A-15B. As discussed elsewhere herein, this actuation occurs after a preselected or desired period of time, such as the length of the treatment period. Such treatment periods may last at least 3-months. Accordingly, the balloon 10 would stay inflated within the stomach 20 for at least 3-months. At or near the end of that time, the deflation component 509 could transition from the closed configuration (FIG. 14) to the open configuration (FIGS. 15A-15B), followed by deflation of the balloon 10 and subsequent passage through and out of the patient's gut.

The deflation subcomponent 509 includes a vent member 584 and a sealing member 586, which cooperate to provide a fluid pathway 588, which fluidly joins or connects the balloon central lumen with the balloon wall outer surface 480. In some embodiments, the fluid pathway 588 is defined or formed by the interaction of vent member 584 with the outer cylinder 505a of the retaining member 505. In the embodiments illustrated in FIGS. 14-19, the outer cylinder 505a is coaxially received into the vent member 584, so as to provide the fluid pathway 588. As shown in FIG. 16, since the thickness T1 of the vent member 584 is greater than the thickness T2 of the outer cylinder 505a, the space between the vent member 584 and outer cylinder 505a provide a tortuous pathway therebetween.

As is most easily seen in FIG. 15B, the vent member 584 includes an inner cylindrical surface 584a and an outer cylindrical surface 584b. These inner and outer surfaces, 584a and 584b respectively, are coaxial with the longitudinal axis C. As shown in FIG. 16, the inner and outer surfaces, 584a and 584b respectively, are spaced apart by a distance or thickness T1. The outer cylinder 505a also includes coaxial cylindrical inner and outer surfaces, 592a and 592b respectively, which are joined at the end 592c. The inner and outer surfaces, 592a and 592b respectively, are spaced apart such that the outer cylinder 505a has a thickness of T2. Accordingly, a fluid pathway 588 is comprises (1) a first space 588a defined by the outer surface 584b of the vent member 584 and the outer surface 592b of the outer cylinder 505a, (2) a second space defined by the inner surface 584a of the vent member 584 and the inner surface 592a of the outer cylinder 505a, and (3) a third joining space 588c located at the end 592c of the outer cylinder 505a. When the fluid pathway 588 is in the open configuration, inflation fluid (represented by dashed arrows 590) can flow from the bottom opening 524, through the first, second and third spaces, 588a, 588b and 588c respectively, and out the top opening 518.

In a further embodiment, the vent member 584 includes a vent channel 592 sized, shaped and otherwise configured to receive the larger outer cylinder 505a of the retaining structure 505 therein. For example, in the illustrated embodiments of FIGS. 14-19, the vent channel 592 is cylindrical and coaxial with axis C. For example, the vent channel appears circular in a cross-section taken perpendicular to the axis C (not shown). The vent channel 592 is fluidly connected with the through-channel 546. As shown in FIG. 16, in embodiments, the vent channel 592 includes a thickness T1 that is as least slightly larger than the thickness T2 of the retaining structure outer cylinder 505a. When the vent member 584 is in the open configuration of FIGS. 15A-15B, inflation fluid 590 can flow around the outer cylinder 505a, through the vent channel 592, into and through the through-channel 546, and out the top opening 518. Alternative vent channel 592 configurations are foreseen.

As noted above, a releasable sealing member 586 cooperates with the vent member 584 so as to bias the fluid pathway 588 in a sealed configuration. For example, the releasable sealing member 586 biases the vent channel 592 against the larger outer cylinder 505a, so as to seal the pathway 588.

The sealing member 586 may take various forms. For example, as shown in FIG. 14, the sealing member 586 is a suture, at least a portion of which comprises dissolvable or degradable material, such as is described elsewhere herein. In another example, the sealing member 586 is a plurality of smaller biodegradable sutures, which may optionally degrade at different rates. The sealing member 586 is tightened or cinched about the head 510 so as to compress the vent channel 592, whereby blocking the flow of inflation fluid therethrough. The dissolvable suture 586 is exposed to a liquid within the balloon lumen, which causes the suture to deteriorate throughout the treatment period. By the termination or end of the treatment period, the suture 586 has sufficiently degraded so that is breaks away, or is otherwise released, from the head 510. Upon release of the sealing member 586, the vent member 584 transitions the fluid pathway 588 from the sealed configuration to the open configuration, such as described above.

In another embodiment, illustrated in FIGS. 17-19, the sealing member 586 is a band having at least a dissolvable or degradable portion. The thickness and structure of the band is configurable, so as to control the rate or timing of the release of the sealing member 586 from the head 510. For example, as shown in FIG. 17, the band may be a single thickness. In another example, shown in FIGS. 18-19, the band may comprise two or more portions, each of which has a different degradation rate or profile. For example, a first portion 586a of the releasable sealing member 586 may be configured to degrade at a faster rate than a second portion 586b of the sealing member 586. For example, the band may include core 586a formed of a rapidly dissolvable material. The core 586a can be coated in a layer 586b of a second material that dissolves at a slower rate. Thus, during a first portion of the treatment period, preferably a longer portion thereof, the outer layer 586b may degrade, and then when the core 586a is reached, the core 586a quickly degrades and is released from the head 510. Accordingly, during a first, extended portion of the treatment period, the band 586 tightly holds the vent member 584 closed, and then when near the end of the treatment period (e.g., a second, shorter period) the core 586a degrades very rapidly, for example within a few minutes or hours, thus causing a catastrophic failure of the self-sealing inflation vent 100, and rapidly deflating the balloon 10. Rapid deflation of the balloon 10 can be desirable, so as to prevent or avoid blockage of the patient's intestinal tract.

In certain embodiments, the rate of degradation of the components of the band 586 can be further controlled by adding one or more channels 586c therethrough. For example, the rate of degradation can be increased by adding more channels 586c. Alternatively, the rate of degradation can be adjusted by modifying the size of the channels 586c. For example, larger channels 586c provide a greater surface area, and therefore a more rapid rate of degradation, than smaller or more narrow channels 586c. In another embodiment, a first portion 586b of the releasable sealing member is configured to stabilize a second portion 586a of the releasable sealing member 586 such that, when the first portion 586b of the releasable sealing member degrades, the second portion of the releasable sealing member destabilizes and is released from the deflation subcomponent, thereby accelerating a process of deflation of the volume occupying subcomponent. Additional configurations are foreseen.

As described above, upon release of the releasable sealing member 586, the retaining structure is configured to be dislodged, thereby opening a larger deflation channel fluidly joining the central lumen with the wall outer surface, and thereby accelerating a process of deflation of the volume occupying subcomponent.

FIGS. 20-25 illustrate alternative embodiments of the self-sealing valve system 600, which are similar to the self-sealing valve 500 described above, the description of which is incorporated herein by reference. Briefly, the self-sealing valve 600 includes a head assembly 610 and a deflation subcomponent 609, for use with the intragastric balloon 10. The self-sealing valve 600 includes a head unit 610 with a longitudinal axis **E**, and is preferably formed of a polymer such as described elsewhere herein. The head 6 includes top and bottom portions, 612 and 613 respectively. The top portion 612 includes an annular top surface 616 with a top opening 618 defined by a top edge 620. The bottom portion 613 includes a bottom surface 622 with a bottom opening 624 defined by a bottom edge 626. An outer surface 628 that joins the top and bottom surfaces 616, 622. The top portion 612 includes shoulder portion 630 in some embodiments.

The top and bottom openings 520, 526 are joined by a coaxial through-channel 646. The through-channel 646 includes a cylindrical inner surface 648 and receives the inner cylinder 605b of the retaining structure 605 therein, such that the inner cylinder 605b and head unit 610 are coaxial along the longitudinal axis E. The through-channel 646 may include a ring-channel 650 for receipt of the needle sleeve bottom rim 220a. Additionally, the through-channel 646 centers the bell-shaped needle sleeve 220 so that the hollow needle 230 pierces a self-sealing septum 614 substantially along the longitudinal axis E.

A metallic retaining structure 605 is received coaxially into the head unit 610. Accordingly, the retaining structure 605 includes outer and inner cylinders, 605a and 605b respectfully, joined by a lower wall 605e. The lower wall 605e includes upper and lower facing surfaces, 605f and 605g respectively. The inner cylinder 605b includes an inner surface 668 defining an inner channel 669 that is in fluid communication with the balloon lumen. The inner cylinder 605b also includes an outer surface 670, and an inner lip 605c with an upper opening 674. A lower opening 675 joins the inner cylinder inner surface 668 with the lower wall lower surface 605g, whereby the inner channel 669 is fluidly joined with the balloon.

In some embodiments, the valve 600 includes a self-sealing septum 614 that is compressed against the inner surface 668 of the lip 605c by a ring-stop 616. In some embodiments, a portion of the septum 614 extends or protrudes through the opening 674.

The valve system 600 includes a connection channel 682 that receives and engages the bell-shaped needle sleeve 220. The connection channel 682 comprises the through-channel inner surface 648, the lower wall upper surface 605f, and the inner cylinder outer surface 670, such that the connection channel 682 is generally cylindrical bore that extends from the retaining structure upper opening 618 to the bottom wall 605e. A metallic retainer-ring 615 may be embedded in the head 610, so as to facilitate frictional engagement and the interference fit, such as described elsewhere herein.

As shown in FIGS. 20-25, the self-sealing valve system 600 includes a deflation subcomponent 609 configured to release inflation fluid from the balloon lumen after passage of a pre-determined time, and thereby deflate the intragastric balloon 10. In embodiments, upon actuation of the deflation subcomponent 609, a substantial portion of the inflation fluid is evacuated from the balloon lumen. For example, in some embodiments, at least 75%, at least 80%, at least 90% or at least 95% of the inflation fluid is evacuated from the balloon lumen. In embodiments, substantially all of the inflation fluid is evacuated from the balloon lumen when the deflation subcomponent 609 is actuated. In more embodiments, the amount of inflation fluid evacuated from the balloon lumen deflates the balloon 10 to a size and/or configuration that enables passage of the deflated balloon through the alimentary canal without substantial discomfort to the patient.

Referring now to FIGS. 20 and 21, the deflation subassembly 609 includes a vent member 684 and a releasable sealing member 686, and is configured to transition from a first, closed configuration (shown in FIG. 20) to an open configuration (shown in FIG. 21) after passage of a predetermined period of time, such as is described above. In the closed configuration, the vent member 684 and the releasable sealing member 686 cooperate to form a gas-tight seal to block the flow of inflation fluid from the lumen of the balloon and into the stomach 20, thereby preventing deflation of the balloon. For example, the releasable sealing member is tightened or cinched around the head using 610, so as to elastically deform the vent member 684 and create the seal. In another example, the releasable sealing member 686 may secure a plug member that either biases the vent member 684 closed or plugs or stops up a portion of the vent member 684.

In the illustrated embodiment, the vent member 684 includes at least one vent channel 692 that extends longitudinally through the head unit 610, with upper and lower openings, 692a and 692b respectively. The upper opening 692a is located in the upper surface 656, while the lower opening 692b is located in or adjacent to the lower surface 622. When the releasable sealing member 686 has been released and the vent member 684 has transitioned from the closed configuration to the open configuration, the vent channel 692 provides a longitudinally extending fluid pathway 688 that fluidly joins or connects the upper and lower openings 692a, 692b. When the fluid pathway 688 is open, inflation fluid 690 enters the lower opening 692b from the balloon lumen, passes through the vent channel 692 and exits the deflation subassembly 609 *via* the upper opening 692a, and thereby deflates the balloon 10. In some embodiments, the vent member 684 includes a longitudinally extending rib 684a, bulge, flange or shoulder that extends radially from the head outer surface 628. The rib 684a may be contiguous or unitary with the outer surface 628, or it may be attached. In some embodiments, the rib 684a houses the entirety of the fluid pathway 688. In other embodiments, the rib 684a houses a longitudinally extending first portion of the fluid pathway 688 while the second portion of the fluid pathway 688 is housed by the head unit 610. In an alternative embodiment, the vent member 684 is housed wholly within the head unit 610 and there is no rib 684a. Additional configurations are foreseen.

The releasable sealing member 686 comprises a tension member 687 that is attached to or secured around the outer surface 628 of the head 610 so as to maintain the vent member 684 in the closed configuration. The tension member 687 is at least one of a spring, a tension band, a suture, a cap and a lock. For example, as shown in FIG. 20, the tension member 687 compresses, collapses or elastically deforms vent member 684, thereby blocking the vent channel 692. In some embodiments, the tension member 687 is a polymeric or metallic band or strip closed or secured around the head unit 610 (e.g., shown in FIGS. 22-23). In other embodiments, the tension member 687 is a suture (e.g., shown in FIG. 24). In still other embodiments, the tension member 687 is a cap (e.g., shown in FIG. 25).

In some embodiments, the tension member 687 includes a dissolvable portion 687a configured to dissolve or degrade upon exposure to conditions within the central lumen of the volume-occupying subcomponent for a predetermined period of time, such as is described above. In an exemplary embodiment shown in FIG. 20, the tension member 687 secures a plurality of dissolvable rings 687a surrounding the tension member 687 such that the tension member 687 is spaced from the head unit 610 and the fluid pathway 688 is simultaneously blocked to fluid flow at or adjacent to the lower openings 692b. In another embodiment shown in FIG. 22, the tension member 687 secures a plurality of plugs 687a at or adjacent to the lower openings 692b. In other embodiments, the plugs 687a fill the openings 682b with a dissolvable material that degrades during the treatment period, and then releases the tension member 687, thereby opening the fluid pathways 688. In another embodiment shown in FIG. 23, the tension member 687 includes a releasable lock 687b secured and/or biased in a locked position by a block or plug of dissolvable material 687a. The block 687a degrades during the treatment period, and when it is sufficiently degraded, the lock 687b opens, (e.g., springs or breaks open) such that the tension member 687 falls away from the device 600, thereby allowing the vent member 684 to relax into the second configuration wherein the fluid pathway 688 is open. In the embodiment shown in FIG. 24, the tension member 687 is a suture that is at least partially degradable over the treatment period. The suture may be constructed such as is described above with respect to device 500. In the embodiment shown in FIG. 25, the tension member 687 is a cap, lid or similar closure that is as least partially degradable and blocks the lower openings 692b of the vent member 684. In the illustrated embodiment, the cap 687 includes an integrally formed, radial, inwardly extending ring portion 687c that biases the lower openings 692b closed. At least a portion of the cap 687, such as but not limited to the ring portion 687c, may be formed of degradable material, such that the material dissolves and thereby releases the cap from the head unit 610. In some embodiments, the entire cap 687 is formed of a degradable material and is pushed over the bottom portion 613 of the device 600, to bias the fluid pathways 688 into the closed configuration.

In embodiments, the sealing properties of the deflation subcomponent 609 is substantially unaffected by mechanical forces during processing or compacting the balloon, such as during insertion of the collapsed balloon into the capsule and attachment of the inflation catheter. In further embodiments, the deflation subcomponent 509 is no substantially affected by application of external gastric pressures, such as while the balloon remains within residence in the stomach. Preferably, the intragastric balloon system is configured to retain more than 75% of an original nominal volume in the gastric environment for at least 60 days.

In the illustrated embodiments, the balloon 10 includes only one deflation subcomponent 609. However, it is foreseen that the balloon 10 may comprise at least one additional deflation subcomponent. For example, the rate of balloon deflation can be increased by providing two, three, four or more devices 600.

It is foreseen that the device 600 may lack any components associated with connecting the balloon 10 to the inflation catheter 200 and/or inflation of the balloon 10. For example, the tension member 605 may lack an inner cylinder 605b, with the lower wall 605e completely sealing or closing the lower opening 675.

In an exemplary embodiment, an intragastric balloon system includes a volume-occupying subcomponent 10 with inner and outer surfaces, the inner surface defining a central lumen, and a deflation subcomponent 600 attached to the inner surface. The deflation subcomponent 600 is configured to deflate the volume-occupying subcomponent10 after a preselected period of time has lapsed since deployment of the system *in vivo,* such as is described above. The deflation subcomponent 600 includes a vent member 684 with a fluid pathway 688 that fluidly joins the central lumen with the outer surface of the volume-occupying subcomponent 10. The fluid pathway 688 is configured to transition between a sealed configuration and an open configuration. The deflation subcomponent 600 also includes a releasable sealing member 686 that cooperates with the vent member 684 so as to bias the fluid pathway 688 in the sealed configuration. Upon release of the sealing member 686, the fluid pathway 688 transitions from the sealed configuration to the open configuration, thereby allowing inflation fluid to flow from the lumen of the volume-occupying component 10 to the stomach 20. In some embodiments, the intragastric balloon system includes at least one device 600. In other embodiments, the intragastric balloon system includes at least two devices 600.

In embodiments, when the fluid pathway 688 is in the sealed configuration, a gas-tight seal is provided between the central lumen and the outer surface of the volume-occupying subcomponent 10. Accordingly, a flow of an inflation fluid 690 through the fluid pathway 688 is substantially blocked. When the fluid pathway 688 is in the open configuration, the sealing member 686 is released, thereby enabling a flow of the inflation fluid 690 through the fluid pathway 688, whereby the volume-occupying component 10 is deflated.

In another embodiment, a method of deflating an intragastric balloon system 10 is provided. After the intragastric balloon system 10, which includes a deflation component such as described elsewhere herein, has been administered to the patient, and ejected from the inflation catheter 200, the deflation component is configured to block flow of the inflation fluid out of the balloon for a preselected period of time. At some time after initiation of the treatment period, when the balloon was administered to the patient, the deflation subcomponent may be actuated. Depending upon the configuration of the deflation subcomponent, the actuation may be either a short, rapid process or along, slower process. In some embodiments, actuation of the deflation component is initiated soon after ejection of the catheter, when the ejection liquid comes into contact with a degradable portion of the deflation subcomponent. In other embodiments, such degradation may not be initiated for a period of weeks or months after balloon implantation. Preferably, a pre-determined period of time is allowed to pass before the dissolvable or degradable subcomponent begins and/or finished dissolving.

When the releasable sealing member is released from the deflation subcomponent 609, the fluid pathway 688 transitions from the sealed configuration to the open configuration, thereby fluidly joining the central lumen with the outer surface of the volume-occupying component. For example, when the fluid pathway transitions to the open configuration, the fluid pathway is allowed to recover from the elastic deformation created by the tension member. In some embodiments, upon release of the releasable sealing member, the retaining structure dislodges from the head structure, thereby opening a larger deflation channel fluidly joining the central lumen with the wall outer surface. Dislodging the retaining structure from the head structure may include allowing the retaining structure to fall into the central lumen. Further, releasing the releasable sealing member may include releasing a tension member secured around an outer side surface of the head. Additionally, releasing the tension member may include exposing the dissolvable or degradable material to conditions within the central lumen of the volume-occupying subcomponent for a predetermined period of time, such as is described above.

Once the fluid pathway 688 has transitioned to the open configuration, inflation fluid flows through the fluid pathway and out of the volume-occupying subcomponent, thereby deflating the volume-occupying component. In some embodiments, releasing the releasable sealing member includes degrading a dissolvable or biodegradable portion of the releasable sealing member, such as is described above. In some embodiments, different portions of the releasable sealing member degrade at different rates. In other embodiments, a portion of the releasable sealing member destabilizes another portion thereof, and accelerates the deflation process. Once the releasable sealing member has been released, at least a portion of the deflation subcomponent may fall into or out of the volume-occupying device, thereby accelerating the deflation process. Additional deflation subcomponents may be provided to further accelerate the deflation process. Preferably, transition of the deflation subcomponent from the closed to open position occurs rapidly and substantially at the end of the treatment period, so as to maximize the effect of the balloon on the patient's appetite and to minimize the possibility of intestinal blockage by the deflated balloon.

### INTRAGASTIC BALLOON INFLATION SYSTEMS

In certain embodiments, the volume-occupying subcomponent is filled or inflated with a fluid using tubing which is subsequently detached and pulled away from the volume-occupying subcomponent. One end of the volume-occupying subcomponent has a port connected to tubing of sufficient length that when unwound can span the entire length of the esophagus, from mouth to stomach. This tubing is connected to the volume-occupying subcomponent with a self-sealable valve or septum that can tear away from the volume-occupying subcomponent and self-seal once the volume-occupying subcomponent is inflated. A physician or other health care professional secures one end of the tubing as the patient swallows the device. Once the device is residing within the stomach, the physician uses the tube to transmit a fluid, such as air, nitrogen, other gas(es), saline solution, pure water, or the like, into the volume-occupying subcomponent and thereby inflate it. After the volume-occupying subcomponent is fully inflated, the tubing is released and can be pulled out from inside the patient.

The tube may be released in a number of manners. For example, the tubing may be detached by applying a gentle force, or tug, on the tubing. Alternatively, the tubing may be detached by actuating a remote release, such as a magnetic or electronic release. Additionally, the tubing may be released from the volume-occupying subcomponent by an automatic ejection mechanism. Such an ejection mechanism may be actuated by the internal pressure of the inflated volume-occupying subcomponent. For example, the ejection mechanism may be sensitive to a specific pressure beyond which it will open so as to release any excess pressure and simultaneously release the tube. This embodiment provides a desirable feature through combining release of the tubing with a safety valve that serves to avert accidental over inflation of the volume-occupying subcomponent in the patient's stomach.

In certain embodiments, it may also be advantageous for the volume-occupying subcomponent to inflate gradually or in several steps over time. For example, if gas escapes the volume-occupying subcomponent prior to the desired deflation time, it can be beneficial for the device to re-inflate in order to preserve it in its expanded state.

### INFLATION TUBING

Referring to FIGS. 3-4, 8-9 and 30, an intragastric balloon system 1915, 30 that is manually inflated by a miniature catheter 50, 1110 can be employed in certain embodiments. The system preferably remains "swallowable." The balloon 10 for delivery is in a compacted state and is attached to a flexible, miniature catheter 50, 1110, preferably no larger than 4 French (1.35 mm) in diameter. The catheter 50, 1110 is designed such that a portion of the catheter 50, 1110 can be bundled or wrapped upon itself for delivery with the encapsulated balloon 10, allowing the patient to swallow both catheter 50, 1110 and balloon 10 for delivery to the stomach 20 (see FIG. 1). The balloon 10 can contain a self-sealable valve system 100 (see FIGS. 5-7, 9-13) for attachment of the catheter 50, 1110 (see FIGS. 9, 13) and inflation of the balloon 10 once it reaches the stomach cavity. The proximal end of the catheter 50, 1110 can be left just outside of the patient's mouth, permitting connection to an inflation fluid dispenser that can house the inflation fluid (gas or liquid). After inflation, the catheter 50, 1110 can be detached from the balloon valve 100 and pulled back through the mouth. This method allows for the intragastric balloon 10 to maintain its swallowability but allow for inflation by a fluid source or a mixture of fluid sources *via* the catheter 50, 1110. Alternatively, a more rigid, pushable system can be employed wherein the balloon valve 100 is compatible with either the swallowable, flexible catheter 50, 1110 or the pushable, rigid catheter assembly.

The inflation catheters 50, 1110 (swallowable or administrator-assisted pushable) described herein are configured to deliver the balloon device 1915, 30 orally and without any additional tools. The administration procedure does not require conscious sedation or other similar sedation procedures or require endoscopy tools for delivery. However, other versions of the device 1915, 30 can be used in conjunction with endoscopy tools for visualization or can be adapted such that the balloon device 30 can be delivered nasogastrically as well.

In operation, the proximal end of the inflation catheter 50, 1110 is connected to a valve or connector that allows for connection to the inflation source or the inflation fluid dispenser. The connector materials may consist of polycarbonate or the like and can connect to a single or multi-lumen catheter tube 1100, such as is known in the art. The distal end of the inflation catheter 50, 1110 is connected to the self-sealing inflation valve system 100 of the balloon 10 that has been compacted and housed within a gelatin capsule 40 or compacted using gelatin bands (FIG. 3). The catheter tube 1100 is preferably from 1 French (0.33 mm) to 6 French (2 mm) in diameter. The catheter 50, 1110 is preferably long enough to extend out past the mouth (connected to the inflation connector or valve) and transverse the esophagus down to at least the middle of the stomach 20 - approximately 50-60 cm. Measurement ticks can be added to the tubing or catheter 50, 1110 to aid in identifying where the end of the tube 50 is located. Timing for inflation can be initiated by having the tube 1100 contain a pH sensor that determines a location difference between the esophagus (pH 5-7) and the stomach 20 (pH 1-4) based on the different pH between the two anatomical sources, or can be derived or verified from the expected pressure in a contained (i.e., esophagus) *versus* a less-constrained space (i.e., stomach 20). The catheter 50, 1110 can also contain nitinol that has a tunable transmission to the body temperature, considering the timing for swallowing. The catheter 50, 1110 can also be connected to a series of encapsulated or compacted balloons 10 on a single catheter 50, 1110, each of which can be inflated and released separately. The number of balloons 10 released can be tune-able to the patient's needs and desired weight loss.

The catheter 50, 1110 may be coated to enhance swallowability or is impregnated or treated with a flavored version and/or one or more local anesthetics or analgesics to ease swallowing. Such anesthetics may include anesthetics in the amino amide group, such as articaine, lidocaine and trimecaine, and anesthetics in the amino ester group, such as benzocaine, procaine and tetracaine. Such analgesics may include chloraseptic.

In another embodiment, a catheter 5400a or 5400b and tubing 5403 ("inflation assembly") are provided for inflating the balloon 10 after ingestion or placement in the stomach 20 (see FIGS. 49A and 49B). As shown in FIG.494A, the catheter 5400a may comprise a two-way luer activated valve 5404 coupled to the dispenser connection assembly 5401 to allow for automatic pressure normalization between the intragastric device and pressure relative to the catheter 5400. Such a configuration makes the catheter 5400 safer for use on patients by automating the valve system as opposed to a manual configuration, and provides a "plug-n-play" functionality. Further illustrated in FIG. 49A, the dispenser connection assembly 5401 may also comprise an O-ring seal 5402 at the disconnect valve 5208 connection point. The O-ring 5402 can provide a seal to prevent pressure escape at the connection point, and may alternatively be constructed of any adhesive or molding sufficient to prevent release of pressure. The connection assembly 5401 may also comprise a one-way valve 5406 for back-flow prevention. For example, the valve 5406 may prevent backflow of ejection fluid. The valve 5406 may allow an operator to release the pressurized contents from the canister 410 into the intragastric device, but prevent the device from re-pressurizing the canister 410 or dispenser after emptying. It may further allow for the catheter 5400 to be removed from the dispenser and attached to another dispenser or other compatible device without releasing pressure from the intragastric device. The connection assembly 5401 may further comprise a sealed navigation port 5405 to allow the operator to navigate and control the catheter tube in areas that prevent the operator from directly handling it.

Additional description of catheters 50, 1110 for use with the intragastric balloon system 30, 1915, of FIG. 3 can be found in U.S. Patent No. 8,647,358, issued February 11, 2014, or U.S. Patent Publication No. 2013/0226219, published August 29, 2013, each of which is incorporated herein in its entirety.

### INFLATION FLUID CONTAINER

The inflation fluid container or canister 1602 or 2003 is employed to control the amount or volume of fluid placed inside of the balloon. This can be in the form of a canister of, e.g., PVC, stainless steel, or other suitable material. The container can also be in syringe form. The materials employed are able to contain a fluid (e.g., a liquid, a gas, or a vapor), preferably in gas form, e.g., compressed or non-compressed N₂, O₂, Ar, CO₂, Ne, CH₄, He, Kr, H₂, and Xe, or mixture(s) thereof, or compressed or non-compressed atmospheric air (a mixture of N₂, O₂, Ar, CO₂, Ne, CH₄, He, Kr, H₂, and Xe), or inert gases including but are not limited to SF₆, C₂F₆, C₃F₈, C₄F₁₀, C₄F₈, C₄F₈, C₃F₆, CF₄, and CClF₂-CF₃, or combinations of one or more gases, e.g., a mixture of N₂ and SF₆. In selected embodiments, the following gases can be employed as inflation fluids, alone or in combination with other gases: hexafluoroethane, sulfur hexafluoride, perfluoropropane, perfluorobutane, perfluoropentane, perfluorohexane, perfluoroheptane, octafluorocyclobutane, perfluorocyclobutane, hexafluoropropylene, tetrafluoromethane, monochloropentafluoroethane, 1,2-dichlorotetrafluoroethane, Trichlorotrifluoroethane, trifluoroethane, chlorotrifluoroethylene, bromotrifluoromethane, monochlorotrifluoromethane, nitrogen, argon, air, xenon, and octafluoropropane.

The balloon composite wall materials and respective diffusion gradients and gas permeability characteristics are used to select a fluid for inflation of the intragastric balloon, so as to provide a desired volume profile over time for the inflated balloon. The inflation fluid container materials are selected to ensure no or minimal diffusion or leakage of the fluid before it is connected to the connector or valve of the inflation catheter. The inflation fluid container system includes a connector to the catheter and a pressure gauge. The inflation fluid container can be fabricated from any suitable material, e.g., stainless steel. It can also contain a smart chip that notifies the healthcare professional of whether inflation is successful or if the balloon 10 should be detached due to an error in the system.

In some embodiments, the inflation fluid container is in the form of a canister or container (flexible or stiff-bodied), squeezable bag, compliant balloon-like tube or the like. As with other embodiments described above, the canister or container has walls formed of stainless steel, pure aluminum, aluminum alloy, brass, or another suitable barrier material. The walls surround and define an inner reservoir or cavity. The canister may include an actuator which enables a delivery system to be activated to provide the gas, a valve cup configured to retain valve components in an arrangement, a spring, a stem connecting the actuator and the spring, a stem gasket that seals the opening around the valve stem, a tube or straw which extends from the valve to the bottom of the can, allowing the gas under pressure to flow out of the canister, and a housing which holds the spring and connects the tube or straw to the valve assembly. An aperture is typically disposed within one of the walls, providing an opening into the inner reservoir. Referring to FIGS. 26A-26B, the canister includes a cap configured to seal the aperture to prevent fluid from escaping the inner reservoir during storage. When the cap is removed, a straw is visible. A portion of the straw protrudes externally from the canister; the remainder of the straw extends through the aperture and into the inner reservoir. The straw may be formed of polypropylene, polyethylene, nylon, or other suitable polymeric material. The canister of some embodiments is configured to release an inflation fluid from the inner reservoir *via* the straw when a low-pressure gradient fluid path is opened toward the balloon.

In some embodiments, it is desirable to inflate the intragastric balloon 10 using an inflation fluid container having a known internal pressure or quantity of gas. For example, in some embodiments, it is desirable to begin with an inflation fluid container configured to have a volume of, e.g., 50 cm³ or less to 400 cm³ or more, or from 100 cm³ to 200 cm³ or 300 cm³, or from 125 cm³ to 175 cm³, or approximately 150 cm³. In other embodiments, other volumes of gas may be selected. In some embodiments, the inflation fluid container is filled at the site of the procedure, for example, by connecting the inflation fluid container to a tank of compressed gas prior to the procedure and filling the inflation fluid container until a desired volume or pressure is reached. The pressure can be monitored using a pressure gauge. In other embodiments, the inflation fluid container comes prefilled with the desired amount of fluid. In such embodiments, a location's altitude (and resultant atmospheric pressure) should be taken into account in order to provide an inflation fluid container having the desired volume of gas. Inflation fluid containers may be designed with different sizes, colors, or other distinctive marking indicating the altitude range for which each is tailored.

To maintain "swallowability" of the balloon 10 and to ensure comfort of the patient during the procedure, the amount of time the catheter is placed in the mouth/esophagus may be minimized. Timing of inflation can be selected so as to minimize time in place. The outer container-catheter assembly, once swallowed, takes approximately 3 to 120 seconds to reach the stomach. Once in the stomach, the inflation fluid container can be attached to a valve or port of the catheter system. Inflation timing can be controlled by selecting the length of catheter, diameter of the catheter tube, the starting temperature, and the starting pressure. Using the Ideal Gas Law for nitrogen and Boyle's Law (PiVi = P₂V₂) the amount of starting volume/pressure can be derived, where starting pressure takes into account the final target pressure at body temperature. It is desired to have an inflation time after swallow of less than 5 minutes, and preferably 2-3 minutes, before balloon 10 detachment and catheter withdrawal. The inputs use to derive inflation of the balloon 10 (preferably in less than 3 minutes) include inflation container volume, type of inflation fluid (preferably a compressed gas or compressed gas mixture), starting pressure, catheter length and diameter, and desired end volume and pressure of the balloon. Thus, due to differences in diameter, a smaller 1 French or 2 French diameter catheter system requires a lower starting pressure to achieve the same target balloon 10 volume and pressure in the same time frame as a larger diameter catheter (e.g., 4- or 5-French), assuming use of the same compressed gas formulation. In general, it is understood that starting with a lower pressure with the same flow rate/volume can increase the inflation time.

The inflation source container can provide feedback to the end user based on a pressure decay system. Where there is an expected starting pressure and expected ending pressure to indicate whether the balloon 10 is inflated properly, there is no need for endoscopic visualization. Each scenario of expected pressure outputs depicted in FIG. 12 can have its own tolerances around it to reduce possibilities of false positives, and the inflation fluid container can provide feedback based on these tolerances as to the status of balloon 10 inflation and detachment. The inflation container contains additional low volume bolus of pressure that is released to determine device location and then is followed by a second, larger bolus intended to inflate the balloon. The gas release could be done in 1, 2, 3 or a plurality of boluses based on the events intended to be detected. This is derived based on the Ideal Gas Law, where there is an expected end pressure based on the fixed volume of the balloon. If the pressure remains high and doesn't decay as expected, this can indicate a failure in the system (e.g., the balloon container 40 did not dissolve, the balloon 10 is expanding in the esophagus because there is, e.g., a kink in the tube or other failure in the catheter system). For example, for a successful decay using nitrogen only as the inflation fluid, the starting pressure is 22-30 PSI to inflate a balloon 10 to 250 cm³ and 1-2.5 psi (0.120 kg/cm²) for a nylon-based material. To indicate successful balloon 10 inflation, a math chip can be added to the inflation source container that provides at least one of a visual, audible, or tactile notification, or otherwise transmits a notification to a healthcare professional or administrator of whether inflation is successful or if there is an error in the system based on the pressure curve and a set of predetermined pressure tolerances and expected timing of inflation.

Alternatively, the balloon 10 can be filled based on a starting pressure by using a spring mechanism, a balloon-within-balloon mechanism, or other pressure source. These mechanisms can potentially result in more predictable/consistent pressure decay curves, and again can have accompanying, predetermined tolerances for feedback back to the end user. FIG. 13 depicts the expected decay curve for these methods of pressure sources, and again would have accompanying, predetermined tolerances for feedback back to the end user.

### INFLATION FLUID DISPENERS

FIGS. 29A-29B and 30 illustrate an exemplary embodiment of an inflation system, including an inflation fluid container 1602, an inflation fluid dispenser 1900 and an optional connector assembly 1911. In some embodiments, a canister, such as the canister of FIGS. 26A and 26B, is coupled to an inflation fluid dispenser 1900 in order to deliver an inflation fluid to the intragastric balloon 10 or other inflatable intragastric device. The inflation fluid dispenser 1900 includes a housing 1905 configured to couple to the canister 1602. Some embodiments have a securement latch 1903, a receiving space fitted to receive a canister 1602, a threaded engagement feature, and/or other features configured to securely couple the housing to the aerosol canister 1602.

The housing 1905 may also include a spring 1904 positioned to contact the straw 1603 of the aerosol canister 1602 when the aerosol canister 1602 is in its secured position within the housing 1905. In such embodiments, as the canister 1602 is brought into the secured position, the spring 1904 applies a force onto the straw 1603 in the direction of the canister 1602, causing a preliminary quantity of inflation fluid to be released from the canister 1602. In some embodiments, the release of a preliminary quantity of inflation fluid is designed to lower the inflation fluid within the aerosol canister 1602 to a desired starting volume or pressure. In some embodiments, the material, size, and strength of the spring 1904 are selected such that the amount of force exerted by the spring 1904, and the resultant preliminary quantity of inflation fluid released, are predetermined. The material, size, and strength of the spring 1904 can be selected according to the elevation and average atmospheric pressure of the location in which the inflation fluid dispenser will be used. In this manner, the inflation fluid dispenser can be calibrated for use in particular regions or elevation ranges, and a canister 1602 of a standard size and fill volume can be used regardless of location.

The housing 1905 may also have an inner wall defining a channel (e.g., similar to channel 5207 of FIG. 44), which extends at least partially through the housing 1905. In such embodiments, a proximal inlet of the channel is positioned around the straw of the canister when the canister is securely coupled to the housing 1905. The spring 1904, if present, may be disposed within the proximal inlet. A distal outlet 1907 of the channel is positioned at a distal end of the inflation fluid dispenser 1900 and is configured as a port 1907 to which a catheter 50, 1110 or other flexible tubing can be connected. In various embodiments, a valve (e.g., mechanically coupled to lever 1906) is disposed in the channel between the proximal inlet 1904 and the distal outlet 1907 and is configured to transition between a 'closed' position (e.g., see lever 2006 in FIG. 36) and an 'open' position (e.g., see lever 1906 in FIG. 29A). In the closed position, the valve blocks the flow of fluid between the proximal inlet 1904 and the distal outlet 1907. In the open position, the flow of fluid is not blocked. Accordingly, as shown in FIG. 30, in the open position, an inflation fluid can flow from an inner reservoir of the aerosol canister 1602, 2003, through the straw 1603 of the canister 1602, 2003 and the channel of the inflation fluid dispenser 1900, and out the distal outlet 1907 to a catheter 50, 1110, and then an intragastric device 30, 1915, and/or other component coupled to the port 1907.

In some embodiments, the inflation fluid dispenser 1900 includes a lever 1906 disposed on an outer surface of the housing 1905 and mechanically coupled to the valve. A medical professional can move the lever 1906 between a first and a second position to transition the valve between the open and closed positions. In other embodiments, the inflation fluid dispenser 1900 may include a button, key, toggle, or switch disposed on an outer surface of the housing 1905 and electrically coupled to the valve. When manipulated by a medical professional, the button, key, toggle, or switch may send an electrical signal to the valve to open or close. In still other embodiments, any suitable valve control mechanism configured for manipulation by a user may be present in the inflation fluid dispenser 1900.

In some embodiments, a pressure gauge 1908 is coupled to the housing 1905 and configured to detect the gauge pressure within the channel between the valve and the distal outlet 1907. In some such embodiments, the pressure gauge 1908 includes a digital display; in other embodiments, the pressure gauge 1908 has a card and mechanical dial indicator.

In some embodiments, the inflation fluid dispenser 1900 and canister 1602, 2003 (or other inflation fluid container) form part of a larger inflation system 2000 used to deliver the inflation fluid to the inflatable intragastric device 10. One example of an inflation system is provided in FIGS. 33-39. In some such embodiments, flexible tubing and a substantially encapsulated intragastric device are coupled to the inflation fluid dispenser 2000 and inflation fluid container 2003. The flexible tubing may include a catheter 50, 1110 a synthetic rubber extension tube 1911, or other bendable, elongated device having an inner lumen. In some embodiments, the flexible tubing includes both an extension tube 1911 and a catheter 50, 1110 connected *via* spokes of a stopcock 1912 having a three-way valve 1912 (e.g., see FIGS. 36-39. A syringe 1913 may be connected to a third spoke of the three-way valve 1912. Each of these components of the system is described in detail above.

In one method of delivering an inflatable intragastric device 10 into a patient's stomach 20 illustrated in FIGS. 33-39, the inflation fluid dispenser 2000 is first coupled to the inflation fluid container 2003. In some embodiments, this coupling is performed with the dispenser's valve in an open position. The inflation fluid dispenser 2000 may contain a barometric pressure compensation valve that accounts for temperature, altitude, starting pressure; where based on the current altitude settings adjusts for the final starting gas number of moles required to inflate a balloon 10 to its target pressure (constant, but can range from 1-5 psi). This compensation is done at coupling and may cause a preliminary amount of inflation fluid to be vented from the distal outlet 2007 of the channel when the inflation fluid container 2003 comes into contact with, and experiences a force from, a spring 2004 positioned in the proximal inlet of the channel based on differences in the environment of the manufacturing settings and the current environment the device 2000 is being utilized in (e.g., altitude). In some embodiments having a pressure gauge 2008, when the valve is open, the pressure of the inflation fluid container can be monitored. In some embodiments, it is desired for inflation fluid to be vented from the inflation fluid container 2003 until the pressure within the container 2003 reaches 250kPa, 300kPa, or any value therebetween. In other embodiments, the desired pressure within the inflation fluid container 2003 is between 257 and 297 kPa, inclusive of said values. Once the inflation fluid container 2003 is securely coupled to the dispenser 2000 and the desired pressure has been reached, the valve may be closed.

In some embodiments, the extension tube 1911, stopcock 1912, catheter 1110, 50, ejection syringe 1913, and the at least partially encapsulated intragastric device 1915, 30 are attached to each other, and a proximal end of the extension tube 1911 is attached to the port 2007 on the distal end of the dispenser 2000. With the three-way valve of the stopcock 1912 in a position which allows for fluid connection between the extension tube 1911 and the catheter, fluid will flow between the channel of the dispenser 2000 (downstream from the dispenser's valve), the extension tube 1911, the catheter 50, 1110, and the encapsulated intragastric device 30, 1915 until an equilibrium is reached. The reading of the pressure gauge 2008 should be generally reflective of the pressure within the intragastric device 10.

In some methods of delivering the inflatable intragastric device 10 into a patient's stomach 20, the patient swallows the substantially encapsulated intragastric device 30 in order to deliver it to the stomach 20. In various embodiments, the catheter 50, 1110 remains connected to the intragastric device 30 and extends from the stomach 20, through the esophagus, and at least into the patient's mouth. In some embodiments, the swallowing and positioning of the intragastric device 30 is monitored using a radiographic imaging method, such as, for example, fluoroscopy.

Once in the stomach 20, the capsule 40 surrounding the intragastric device 10 of various embodiments begins to dissolve. In some embodiments, the capsule 40 dissolves in less than ten minutes. In some embodiments, the capsule 40 dissolves within thirty seconds to four minutes. In some embodiments, the detected pressure will drop to below 10 kPa when the capsule dissolves. In some embodiments, the detected pressure will drop below 7kPa upon dissolution. In various embodiments, the process of filling the inflatable intragastric device 10 can begin upon dissolution of the capsule 40. In order to fill the inflatable intragastric device 10 with an inflation fluid, the valve of the dispenser 2000 is transitioned to an open position. Inflation fluid will flow substantially from the relatively high-pressured inner reservoir of the inflation fluid container 2003 to the relatively low-pressured inner cavity of the inflatable intragastric device 10 until an equilibrium pressure is achieved in the system. In some embodiments, approximately 150 cm³ of a gas in unconstrained atmosphere that is constrained to 28 psi or approximately 450 cc at atmospheric pressure. For example, N₂, will be transferred into the intragastric device 10. In some embodiments, the intragastric device 10 will achieve a final volume within the stomach 20 of approximately 250cc. In other embodiments, the intragastric device 10 will achieve a final volume within the stomach 20 of 50 to 400cc, and preferably 90 to 300cc, or any individual value or range therebetween. The intragastric device 10 of some embodiments will achieve a final pressure of 5kPa, 20 kPa, or any value therebetween. In a embodiment, the final pressure within the intragastric device 10 is between 8.3kPa and 17.2kPa, and more preferably, the final pressure of the intragastric device 10 is 13.8kPa. The intragastric device 10 of some embodiments reaches a desired final pressure in less than five minutes. In some embodiments, the final pressure is reached in approximately two minutes.

In some embodiments of the method, once the pressure of the system has stabilized and reached an equilibrium, the catheter can be separated from the intragastric device 10 and removed from the patient. In some embodiments, the syringe 1913 is used to separate the catheter 50, 1110 from the intragastric device 10. The three-way valve 1912 is moved into a position in which the syringe is in fluid connection with the catheter 50, 1110 and intragastric device 10. The syringe's plunger is pushed rapidly and expel a fluid from the syringe 1913 into the inner cavity of the balloon 10 at a force (e.g., hydraulic force) sufficient to dislodge the catheter 50, 1110 from the intragastric device 10.

### AUTOMATED INFLATION SYSTEMS

FIGS. 40-62 illustrate several embodiments of an automated inflation system for use with the Obalon Gastric Balloon Assembly 30. In some embodiments, an automated inflation system is employed to deliver inflation fluid to an inflatable gastric device similar to that illustrated in FIGS. 2 and 3. An automated inflation system can include a computing device, such as a computer, having a processing unit, such as a processor, microprocessor, or microchip configured to perform logical operations to cause one or more components of the inflation system to perform functions to facilitate the inflation of an intragastric balloon to a final target volume. The inflation fluid container or the inflation fluid dispenser can be configured to dispense an inflation fluid for delivery to an intragastric balloon. The computing device can further include a memory, which may be read only memory (ROM) and random-access memory (RAM). The memory can store one or more modules that store data values defining instructions to configure the processing unit to perform the logical operations of the inflation fluid system. In some embodiments, the computing device can include a user interface having a user input configured to allow a user to program one or more parameters of the inflation system. The user interface can further include a display configured to display data regarding the operation of the inflation system.

The computing device can further include a communications module for transmitting and/or receiving data from one or more components of the automated inflation system. The communications module can include a receiver, a transmitter, and or a transceiver. The communications module can be configured to communicate *via* any suitable wired or wireless communications medium. In some embodiments, the communications module can be configured to communicate with an external device *via* a medium capable of transmission through the body of a patient.

In some embodiments, the communications module of the computing device can be configured to transmit instructions to an inflation fluid container or inflation fluid dispenser. The memory of the computing device can store instructions that configure the processing unit to cause the inflation fluid container or the inflation fluid dispenser to dispense an initial volume of inflation fluid estimated to cause the intragastric balloon to inflate to a final target pressure of the intragastric balloon or an initial bolus target pressure. The initial bolus target pressure can be a pressure less than the desired final target pressure of the intragastric balloon. After an intragastric balloon receives an initial volume of inflation fluid causing the intragastric balloon to fill to an initial bolus target pressure less than the final target pressure of the intragastric balloon, the inflation system can be configured to provide one or more additional incremental volumes of inflation fluid to raise the pressure in the intragastric balloon to the final target pressure, as described further herein. In some embodiments, the initial bolus target pressure can be a pressure greater than the desired final target pressure of the intragastric balloon. After an intragastric balloon receives a volume of inflation fluid causing the intragastric balloon to fill to an initial bolus target pressure greater than the final target pressure of the intragastric balloon, the inflation system can be configured to cause the incremental release of one or more volumes of inflation fluid from the intragastric balloon in order to cause the pressure in the intragastric balloon to decrease to the final target pressure, as described further herein.

The inflation fluid container or inflation fluid dispenser can include a communications module configured to communicate with the computing device and/or one or more other components of the inflation system. The inflation fluid container or inflation fluid dispenser may further include a processing unit and/or memory storing data values defining instructions to configure the processing unit to perform the logical operations of the inflation fluid container or inflation fluid dispenser.

The inflation fluid system can further include one or more pressure sensors configured to provide data to the computing device. The pressure sensors can include a communications module for transmitting data to the computing device. The processing unit of the inflation fluid container can be configured to determine based on the data from the one or more pressure sensors, one or more additional volumes of inflation fluid to dispense in order to cause the intragastric balloon to inflate to a final target pressure. In some embodiments, the inflation system can include at least one pressure sensor configured to detect a pressure within the balloon. In some embodiments, the inflation system can include at least one pressure sensor configured to detect a pressure within the inflation fluid container or inflation fluid dispenser.

In some embodiments, the inflation fluid system can include a pressure regulator configured to reduce the pressure of the inflation fluid received from the inflation fluid container to a lower desired value at an output of the pressure regulator. Inflation fluid output from the pressure regulator can be direct to the intragastric balloon. Reduction of the pressure of the inflation fluid by the pressure regulator can allow the inflation fluid to enter the intragastric balloon at a safe pressure to reduce the risk of harm to patient. Further, reduction of pressure of the inflation fluid by the pressure regulator can allow for delivery of inflation fluid at a consistent pressure, which can improve the accuracy of the processing unit of the inflation fluid container or inflation fluid dispenser in determining additional volumes of inflation fluid to dispense in order to cause the intragastric balloon 10 to inflate to a final target pressure. In some embodiments, the pressure regulator can be configured to communicate with the computing device and/or one or more additional components of the inflation system. The pressure regulator may further include a processing unit and/or memory storing data values defining instructions to configure the processing unit to perform the logical operations of the pressure regulator. For example, in some embodiments, the pressure regulator can be configured to change the pressure of the inflation fluid output from the pressure regulator in response to data, such as pressure sensor data, received from the computing device and/or one or more additional components of the inflation system. In some embodiments, the pressure regulator can be configured to transmit data to the computing device for use in determining additional volumes of inflation fluid to dispense in order to cause the intragastric balloon to inflate to a final target pressure. The pressure regulator can be a diaphragm style pressure regulator, a piston style pressure regulator, or any other suitable pressure regulator.

The inflation fluid system can further include one or more valves configured to control the flow of the inflation fluid through the inflation system. In some embodiments, the inflation fluid system can include a valve positioned to control the flow of inflation fluid between the pressure regulator and the intragastric balloon. A pressure sensor can be positioned between the valve and the intragastric balloon such that, when the valve is closed, the reading of the pressure sensor is indicative of the pressure within the intragastric balloon. In some embodiments, a valve can be positioned between the intragastric balloon 10 and an outflow opening. A valve positioned between the intragastric balloon 10 and the outflow opening can be opened to control the flow of inflation fluid from the intragastric balloon 10 to the outflow opening to facilitate the reduction of pressure within the intragastric balloon. Each of the valves of the inflation system can be configured to communicate with the computing device and/or one or more additional components of the inflation system. Each valve can further include a processing unit and/or memory storing data values defining instructions to configure the processing unit to perform the logical operations of the pressure the valve. For example, each valve can be configured to open or close in response to instructions received from the computing device or one or more additional components of the inflation system. In some embodiments, each valve can transmit data to the computing device or one or more other components of the inflation system. For example, each valve can transmit data regarding the status of the valve as open or closed.

An automated inflation system can further include one or more sections of flexible tubing configured to facilitate the flow of the inflation fluid between various components of the inflation system.

One example of an automated inflation system is provided in FIG. 40. FIG. 25 depicts an inflation system 4800 including an inflation fluid container 4805, a first pressure gauge 4810, a pressure regulator 4815, a valve 4820, a second pressure gauge 4825, an intragastric balloon 4830, and a computing device 4835. The inflation system 4800 can further including a plurality of sections of flexible tubing 4840A-C (or a rigid channel) to facilitate the flow of an inflation fluid from throughout the inflation fluid system 4800. The intragastric balloon 4830 can be configured to receive the inflation fluid *via* a catheter 4845 connected to the flexible tubing at a connector 4850.

The inflation fluid container 4805 can contain an inflation fluid for delivery to the intragastric balloon 4830. When full, the inflation fluid container 4805 can have a pressure of 60 psi or about 60 psi. In response to instructions from the computing device 4835, the inflation fluid container 4805 can be configured to dispense a bolus of inflation fluid into the flexible tubing 4840A. The pressure regulator 4815 can be positioned to receive the inflation fluid from the flexible tubing 4840A. The pressure regulator 4815 can be configured to reduce the pressure of the inflation fluid. In some embodiments, the pressure regulator 4815 reduces the pressure of the inflation fluid to 5 psi, about 5 psi, 4 psi, about 4 psi, 3 psi, about 3 psi, 2 psi, about 2 psi, 1 psi or about 1 psi. In some embodiments, the pressure regulator can receive instructions from the computing device 4835 to adjust the pressure of the inflation fluid output by the pressure regulator. The pressure regulator can be configured to output the inflation fluid into the flexible tubing 4840B. The valve 4820 can be positioned to receive inflation fluid from the flexible tubing 4840B. The valve 4820 can be opened to permit inflation fluid to flow through the valve or closed to prevent inflation fluid from flowing through the valve. In some embodiments, the valve 4820 can be configured to open or close in response to instructions received from the computing device 4835. In some embodiments, the valve 4820 can be a solenoid valve. Inflation fluid flowing through the valve 4820 can be output into the flexible tubing 4840C. Inflation fluid flowing through the flexible tubing 4840C can flow through the catheter 4845 and into the intragastric balloon 4830. In some embodiments, a final target pressure of the intragastric balloon 4830 is 2 psi or about 2 psi. In some embodiments, a final target pressure of the intragastric balloon 4830 is between 2 psi to 5 psi or between 2 psi to 8 psi. Inflating an intragastric balloon to a pressure above 8 psi may risk injury to a patient.

The pressure gauge 4810 can be positioned between the inflation fluid container 4805 and the pressure regulator 4815. The pressure gauge 4810 can allow the pressure in the inflation fluid container 4805 to be monitored. The pressure gauge 4810 can further be configured to transmit pressure data to the computing device 4835 for processing. The computing device 4835 can monitor the pressure data received from the pressure gauge 4810 in the event of failure of the inflation system 4800, for example, as described herein with respect to FIG. 12.

The pressure gauge 4825 can be positioned between the valve 4820 and the intragastric balloon 4830. When the valve 4820 is closed, the reading of the pressure gauge 4825 should be generally reflective of the pressure within the intragastric balloon 4830 after an equilibrium is reached. The pressure gauge 4825 can be configured to transmit pressure data to the computing device 4835 for processing.

Referring now to FIG. 42, in one example of operating the inflation system 4800, the computing device 4835 can provide instructions to dispense an initial bolus volume C1 of inflation fluid from the inflation fluid container 4805 into the inflation fluid system 4800 (e.g., into tubing 4840A). The initial bolus C1 volume of the inflation fluid may be a volume estimated to produce a final target pressure B in the intragastric balloon 4830 or an initial bolus target pressure that is less than the final target pressure B. The valve 4820 can be in the open position prior to dispensing of the initial bolus C1 volume of inflation fluid from the inflation fluid container 4805 or may be opened after dispensing of the initial bolus C1 volume of inflation fluid from the inflation fluid container 4805 to allow the initial bolus C1 volume of the inflation fluid container to flow through the inflation fluid system 4800 and into the balloon 4830.

After a defined period of time sufficient to allow the initial bolus C1 volume of inflation fluid to flow into the intragastric balloon 4830, the computing device 4835 can be configured to provide instructions to the valve 4820 to close. The defined period of time can be determined by the computing device 4835. After the valve 4820 is closed, the computing device 4835 can be configured to determine an additional bolus (e.g., C2, C3) or volume of inflation fluid to be dispensed by the inflation fluid container 4805 when it is determined that the pressure in the intragastric balloon 4830 is lower than the final target pressure B based on data received from the pressure gauge 4825 after sufficient time is allowed for equilibrium to be achieved. As explained above, when the valve 4820 is closed and equilibrium is achieved (e.g., as indicated by a flat portion of the graph line of FIG. 27), the pressure measured by the pressure gauge 4825 is generally indicative of the pressure within the intragastric balloon 4830. In some embodiments, the computing system 4835 is configured to determine that equilibrium has been achieved after a determined period of time. In other embodiments, the computing system 4835 is configured to determine that equilibrium is achieved based on trends in the data received from the pressure gauge 4825.

After the computing device 4835 determines an additional volume or bolus (e.g., C2, C3) of inflation fluid to be dispensed from the inflation fluid container 4805, the computing device can provide instructions to the inflation fluid container 4805 to dispense the determined additional volume of inflation fluid. The computing device 4835 can further provide instructions to the valve 4820 to open to allow the determined additional volume (e.g., C2, C3) of the inflation fluid to flow into the balloon 4830. Measurement of the pressure within the intragastric balloon 4830, determination of an additional volume of inflation fluid to be dispensed by the inflation fluid dispenser 4800, and dispensing of additional volumes of inflation fluid can be repeated until a final target pressure of the intragastric balloon 4830 is achieved.

FIG. 42 depicts a graph showing an example of pressure measurements measured by the pressure gauge 4825 during inflation of an intragastric balloon inflated by dispensing incremental volumes of inflation fluid, wherein the target final balloon pressure B is approached incrementally by sequential boluses (e.g., C1, C2, C3) of inflation fluid into the balloon.

The computing device 4835 can further be configured to determine a status of the inflation system 4800 based on data from the first pressure gauge 4810 and the second pressure gauge 4825. For example, if the pressure measured at the first pressure gauge 4810 and the pressure measured at the second pressure gauge 4825 are each above certain values, a determination can be made that the intragastric balloon 4830 may be constrained, such as but not limited to in the esophagus or in a constrained space associated with the gastric system. If the pressure measured at the first pressure gauge 4810 and the pressure measured at the second pressure gauge 4825 are both below certain values, a determination can be made that there may be a leak in the inflation system 4800. Further, a certain range of pressures measured at each of the first pressure gauge 4810 and the second pressure gauge 4825 can be indicative of a successful inflation of the balloon 4830.

Although a separate computing device 4835 is described, one of skill in the art would recognize that the computing device may be part of one of the other components of the inflation system 4800, such as for example, the inflation container 4805 or a dispenser. In some embodiments, the functions described with respect to the computing device 4835 may instead be performed multiple separate components of the inflation system 4800. In some embodiments, one or more functions described with respect to the computing device 4835 can be performed at one or more processing units in connection with one or more of the inflation fluid container 4805, the first pressure gauge 4810, the pressure regulator 4815, the valve 4820, and the second pressure gauge 4825.

Another example of an automated inflation system is provided in FIG. 41, which depicts an inflation system 5000 including an inflation fluid container 5005, a first pressure gauge 5010, a pressure regulator 5015, a first valve 5020, a second pressure gauge 5025, a second valve 5055, an outflow opening 5060 or vent, and an intragastric balloon 5030, and a computing device 5035. The inflation system 5000 can further including one or more sections of flexible tubing 5040A-E (or a rigid channel) to facilitate the flow of an inflation fluid from throughout the inflation fluid system 5000. The intragastric balloon 5030 can be configured to receive the inflation fluid *via* a catheter 5045 connected to the flexible tubing at a connector 5050, such as is described elsewhere herein.

The inflation fluid container 5005 can contain an inflation fluid for delivery to the intragastric balloon 5030. When full, the inflation fluid container 5005 can have a pressure of 60 psi or about 60 psi. In response to instructions from the computing device 5035, the system 5000 can be configured to dispense an initial bolus of inflation fluid from the inflation fluid container 5005 into the flexible tubing 5040A. The pressure regulator 5015 can be positioned to receive the inflation fluid from the flexible tubing 5040A. The pressure regulator 5015 can be configured to reduce the pressure of the inflation fluid. In some embodiments, the pressure regulator 5015 reduces the pressure of the inflation fluid to 5 psi, about 5 psi, 4 psi, about 4 psi, 3 psi, about 3 psi, 2 psi, about 2 psi, 1 psi or about 1 psi. In some embodiments, the pressure regulator can receive instructions from the computing device 5035 to adjust the pressure of the inflation fluid output by the pressure regulator. The pressure regulator can be configured to output the inflation fluid into the flexible tubing 5040B. The first valve 5020 can be positioned to receive inflation fluid from the flexible tubing 5040B. The first valve 5020 can be opened to permit inflation fluid to flow through the valve or closed to prevent inflation fluid from flowing through the valve, respectively. In some embodiments, the first valve 5020 can be configured to open or close in response to instructions received from the computing device 5035. In some embodiments, the first valve 5020 can be a solenoid valve. Inflation fluid flowing through the first valve 5020 can be output into the flexible tubing 5040C. Inflation fluid flowing through the flexible tubing 5040C can flow through the catheter 5045 and into the intragastric balloon 5030. In some embodiments, a final target pressure of the intragastric balloon 5030 is 2 psi or about 2 psi. In some embodiments, a final target pressure of the intragastric balloon 5030 is between 2 psi to 5 psi or between 2 psi to 8 psi. Inflating an intragastric balloon to a pressure above 8 psi may risk injury to a patient.

The pressure gauge 5010 can be positioned between the inflation fluid container 5005 and the pressure regulator 5015. The pressure gauge 5010 can allow the pressure in the inflation fluid container 5005 to be monitored. The pressure gauge 5010 can further be configured to transmit pressure data to the computing device 5035 for processing. The computing device 5035 can monitor the pressure data received from the pressure gauge 5010 to failure of the inflation system 5000, for example, as described herein with respect to FIG. 41.

The pressure gauge 5025 can be positioned between the first valve 5020 and the intragastric balloon 5030. When the first valve 5020 and second valve 5055 are closed, the reading of the pressure gauge 5025 should be generally reflective of the pressure within the intragastric balloon 5030 after an equilibrium is reached. The pressure gauge 5025 can be configured to transmit pressure data to the computing device 5035 for processing.

The second valve 5055 can be positioned to receive inflation fluid flowing through the flexible tubing 5040C from the valve 5020 or from the intragastric balloon 5030. The second valve 5055 can be opened to permit inflation fluid to flow through the second valve 5055 or can be closed to prevent inflation fluid from flowing through the valve 5055. Inflation fluid flowing through the valve 5055 can flow out of the output opening 5060, and consequently, out of the inflation system 5000. If the first valve 5020 is open and the second valve 5055 is open, inflation fluid flowing through the first valve 5020 can flow out of the inflation system 5000 *via* the outflow opening 5060. If the first valve 5020 is closed and the second valve 5055 is open, inflation fluid within the intragastric balloon can flow out of the inflation system 5000 *via* the outflow opening 5060. In some embodiments, the second valve 5055 can be configured to open or close in response to instructions received from the computing device 5035. In some embodiments, the second valve 5055 can be a solenoid valve.

If the second valve 5055 is closed, the inflation system 5000 can operate in a similar manner as discussed herein with respect to the inflation system 4800 to inflate the intragastric balloon 5030 to a final target pressure.

In one example of operating the inflation system 5000, the inflation system 5000 can be configured to fill the intragastric balloon 5030 to an initial bolus target pressure (e.g., D of FIG. 43) that is greater than the final target pressure B and incrementally reduce the pressure in the balloon 5030 by opening the second valve 5055 (e.g., E1, E2, E3 of FIG. 28). In some embodiments, the initial bolus target pressure is 5 psi or about 5 psi, is between 2 psi to 5 psi, or is between 2 psi to 8psi. The computing device 5035 can provide instructions to the inflation fluid container 5005 to dispense the initial bolus D volume of inflation fluid into the inflation fluid system 5000. The initial bolus D volume of the inflation fluid may be a volume estimated to produce a final target pressure in the intragastric balloon 5030 or an initial bolus target pressure D that is greater than the final target pressure B. The first valve 5020 can be in the open position prior to dispensing of the initial bolus volume of inflation fluid from the inflation fluid container 5005 or may be opened after dispensing of the initial bolus volume of inflation fluid from the inflation fluid container 5005 to allow the initial bolus D volume of the inflation fluid container to flow through the inflation fluid system 5000 and into the balloon 5030. The second valve 5055 can be closed prior to dispensing of the initial bolus volume of inflation fluid to prevent the inflation fluid from flowing out of the outflow opening 5060.

After a defined period of time sufficient to allow the initial bolus D volume of inflation fluid to flow into the intragastric balloon 5030, the computing device 5035 can be configured to provide instructions to the first valve 5020 to close. The defined period of time can be determined by the computing device 5035. After the first valve 5020 is closed, the computing device 5035 can be configured to determine a volume (e.g., E1, E2, E3) of inflation fluid to be removed, vented or released from the intragastric balloon 5030 when the pressure in the intragastric balloon 5030 is determined to be greater than the final target pressure B based on data received from the pressure gauge 5025 after sufficient time is allowed for equilibrium to be achieved. When the first valve 5020 and the second valve 5055 are closed and equilibrium is achieved, the pressure measured by the pressure gauge 5025 is generally indicative of the pressure within the intragastric balloon 5030. In some embodiments, the computing system 5035 is configured to determine that equilibrium has been achieved after a determined period of time. In other embodiments, the computing system 5035 is configured to determine that equilibrium is achieved based on trends in the data received from the pressure gauge 5025.

After the computing device 5035 determines a volume of inflation fluid to be removed from the intragastric balloon 5030, the computing device 5035 can provide instructions to the valve 5055 to open for a determined length of time (while valve 5020 is closed) in order to allow the determined volume of inflation fluid to flow from the intragastric balloon 5030 out of the outflow opening 5060. After the valve 5055 allows the determined volume of inflation fluid to be removed from the intragastric balloon 5030, the computing device can provide instructions to the valve 5055 to close. Measurement of the pressure within the intragastric balloon 5030, determination of an additional volume of inflation fluid to be removed from the intragastric balloon 5030, and dispensing of additional volumes of inflation fluid from the intragastric balloon 5030 through the outflow opening can be repeated until a final target pressure of the intragastric balloon is achieved.

FIG. 43 depicts a graph showing an example of pressure measurements measured by the pressure gauge 5025 during inflation of an intragastric balloon inflated by dispensing an initial bolus volume D of inflation fluid and then reducing the internal pressure of the intragastric device 10 as by sequentially releasing small volumes (e.g., E1, E2, E3) of inflation fluid until the target internal pressure B is achieved, such as is described herein.

In some operations of the inflation system 5000, the inflation system 5000 can be configured to both incrementally increase the volume of the inflation fluid within the intragastric balloon 5030 and incrementally decrease the volume of the inflation fluid within the intragastric balloon 5030 following the dispensing of an initial bolus D volume of the inflation fluid by the inflation fluid container 5005. This can be desirable, for example, if the pressure in the intragastric balloon 5030 falls below a final target pressure B during an incremental decrease of the pressure within the intragastric balloon 5030 or rises above a final target pressure B during an incremental increase of the pressure within the intragastric balloon 5030. As described herein, the pressure within the intragastric balloon 5030 can be increased by dispensing an additional volume of inflation fluid from the inflation fluid container 5005 when the first valve 5020 is opened and the second valve 5055 is closed. The pressure within the intragastric balloon 5030 can be reduced when the first valve 5020 is closed and the second valve 5055 is open.

Although a separate computing device 5035 is described, one of skill in the art would recognize that the computing device 5035 may be part of one of the other components of the inflation system 5000, such as for example, the inflation container 5005. In some embodiments, the functions described with respect to the computing device 5035 may instead be performed multiple separate components of the inflation system 5000. In some embodiments, one or more functions described with respect to the computing device 5035 can be performed at one or more processing units in connection with one or more of the inflation fluid container 5005, the first pressure gauge 5010, the pressure regulator 5015, the first valve 5020, and the second pressure gauge 5025, and the second valve 5055.

FIGS. 44-52 illustrates an automated dispenser 5200 that may be configured similar to system 4800 or system 5000, for use with the intragastric device system described elsewhere herein. The dispenser 5200 may comprise a housing 5205 to surround a pressurized canister 2003 in order to lock and hold the canister 2003 in place. The housing 5205 may surround the canister 2003, in part or in full, and may provide a visual indicator (not shown in the FIG.) to enable an operator to gauge the remaining pressure in the canister 2003. The canister housing 5205 may comprise a locking mechanism 5206 to allow an operator to install, and lock into place, a canister 2003 of pressurized matter. The locking mechanism 5206 may also allow the operator to safely release and remove the pressurized canister 2003. Attached to the canister housing 5205 is a tunnel housing 5203 comprising in part a tunnel 5207 providing a trajectory into which the contents of the canister 2003 may be released. The tunnel 5207 can allow for the directional flow of the content of the canister 2003, and may also allow for manipulation of the flow through a plug valve 5202. The plug valve 5202 may be situated in an orthogonal orientation relative to the tunnel 5207 to allow interruption or prevention of the flow of the canister contents within the tunnel 5207. The tunnel 5207 may include a vent valve 5201. For example, a vent valve 5201 located between the plug valve 5202 and the canister 410 may allow for atmospheric normalization between the s system and the surrounding pressure. The tunnel housing 5203 may provide sufficient structural strength as to prevent damage to, or compromise the structural integrity of, the tunnel 5207 caused by the pressurized contents of the canister or other potential damage caused by shock, vibration, etc. The tunnel housing 5203 may also provide a structure for which additional components may be installed.

As further illustrated in FIG. 45, the tunnel housing 5203 may be connected to the canister housing 5205 in a manner which allows for the tunnel 5207 to direct the flow of the pressurized contents of the canister 2033. In one embodiment, the tunnel housing 5203 is connected with the canister housing 5205 using an O-ring seal 5209 and a number of screws to fasten the two components together. The connection may also comprise gaskets, gasket sealing adhesives, or any other fastener or sealer sufficient to prevent damage of the components from pressure.

As previously stated, the tunnel housing 5203 may provide a structure for support of additional components. For example, FIG. 45 provides an exemplary embodiment wherein the tunnel housing 5203 provides structure for a plug valve handle 5210, a touch sensitive display 5204 and related circuitry 5211, a quick disconnect valve 5208, and a solenoid vent valve 5201.

The plug valve 5202 may be manually operated using a plug valve handle 5210 attached to the plug valve 5202 outside of the tunnel housing 5203. Such a configuration may allow for the operator to manually adjust the pressure seen by the tunnel 5207. In one example embodiment, the plug valve 5202 may be adjustable by a handle 5210 that allows the operator to twist the valve 5202 open and shut, such as in response to instructions provided by the computer system. In another example embodiment, the valve 5202 may be adjustable by pushing or pulling the valve 5202 open and shut, such as in response to instructions provided by the computer system. Another exemplary embodiment may include a plug valve 5202 that can be electronically activated by either (1) operator feedback or instruction, or (2) electronically adjusted through automated feedback from a processor. In another exemplary embodiment, the plug valve 5202 may include a plug valve channel 5202a that is fluidly engageable with the tunnel 5207, such that when the channel 5202a is longitudinally aligned with the tunnel 5207, the pressurized fill fluid may flow therethrough, and when the channel 5202a is misaligned with the tunnel 5207, the flow therethrough is substantially blocked. Accordingly, the operator may control flow of the pressurized fluid by rotating the valve handle 5210, to move the valve channel 5202a to either an aligned position or a non-aligned position, relative to the tunnel 5207. Alternatively, positioning of the valve channel 5202a can be controlled electronically *via* the circuitry 5211.

The dispenser 5200 may also comprise a quick disconnect valve 5208 affixed to a distal end 5203a of the tunnel housing 5203 in order to allow an operator of the system to attach a catheter 5400a or 5400b to an exit point of the tunnel housing 5203. The tunnel 5207 may direct the flow of the pressurized matter into the catheter 5400a or 5400b in this manner. The disconnect valve 5208 may be fastened to the tunnel housing 5203 using an adhesive or mechanical fastening means. In one exemplary embodiment, the connection between the housing 5203 and the disconnect valve may be sealed with an O-ring 5209 or other type of sealant (e.g., adhesive, gasket, weld). The disconnect valve 5208 may provide a port for connection to the catheter connection assembly 5400a or 5400b. The disconnect valve 5208 may further comprise an electromechanical means for alerting the system to when a catheter assembly 5400a or 5400b is connected to the valve, or when there is no peripheral connection.

As further illustrated in FIGS. 46-47, the tunnel housing 5203 may provide structure to support a solenoid vent valve 5201. The solenoid vent valve 5201 may allow for atmospheric normalization between the pressure in the intragastric device 10 and the pressure in the surrounding atmosphere. The vent valve 5201 may be controlled by an electric current through a solenoid 5201 that is activated when a pressure difference is detected between the intragastric device 10 and the surrounding atmosphere. In one exemplary embodiment, the solenoid 5201 may be activated automatically upon connection of the catheter connection assembly 5400 to the disconnect valve 5208 when the catheter assembly 5401 triggers the electromechanical means for alerting the system to a catheter 5400 connection. In another exemplary embodiment, the solenoid 5201 can be activated automatically by one or multiple pressure transducers (not shown in FIG.) that monitor the pressure of the intragastric device 10 and also the atmospheric pressure when a pressure transducer senses a delta (i.e., Δ, a difference or change in a certain quantity), between the two environments.

FIG. 46 illustrates further the connection between the canister housing 5205 and the tunnel housing 5203, as well as the structural support provided by the tunnel housing to accommodate an adjustable plug valve 5202 and plug valve handle 5210, a solenoid vent valve 5201, quick disconnect valve 5208, and a touch sensitive display 5204 with related circuitry 5211.

FIG. 46 also illustrates an exemplary embodiment of the touch screen display 5204. The touch screen display 5204 may provide information to the operator of the system, about the system. For example, the touch screen display 5204 may provide a time monitor 5303 that can show the time elapsed since the initiation of the procedure. The time indication 5303 may also provide a normal clock, timer, stop-clock or alarm system. The operator may toggle and edit these features using the touch screen 5204.

FIG. 46 further illustrates a touch screen display 5204 comprising a real-time indicator of canister pressure 5304. The indicator of canister pressure 5304 may be adjustable by the operator to show pressure, of the canister 410, in different measuring units or increments.

FIG. 46 further illustrates a touch screen display 5204 comprising a real-time indicator of catheter internal pressure 5305. As with the indicator of canister pressure 5304, the catheter pressure indicator 5305 may be adjustable by the operator to show pressure within the catheter 5400 in different measuring units or increments.

FIG. 46 further illustrates a touch screen display 5204 comprising a real-time indicator of intragastric device pressure 5306. As with the indicator of canister pressure 5304, the intragastric device pressure indicator 5306 may be adjustable by the operator to show intragastric device pressure in different measuring units or increments.

FIG. 46 further illustrates a touch screen display 5204 comprising a real-time indicator of ambient atmospheric pressure 5307. As with the indicator of canister pressure 5304, the ambient atmospheric pressure indicator 5307 may be adjustable by the operator to show ambient pressure in different measuring units or increments.

FIG. 46 further illustrates a touch screen 5204 comprising a touch-screen button 208 or similar graphical input device. The touch-screen button 5308 may be actuated by the operator, such as in response to queries by the device 5100 or at the completion of steps in a balloon implantation procedure.

FIG. 47 and FIG. 48 provide different views of the features provide in FIGS. 29 and 30.

FIG. 48 provides a view of the dispenser 5200 wherein a housing 5301 for the touch screen circuitry 5211 and the plug valve 5202 is installed. The housing 5301 may provide a protective shell for the circuitry from operator misuse, dropping, liquid spilling, biological contamination, or another hazard. The touch screen housing 5301 may have an opening with a push button interface 5302 to allow the operator to toggle power to the dispenser 5200 on and off. The power toggle 5302 may be a touch sensitive interface, a push button interface, a switch, or any other apparatus that allows for operator modulation of power. The touch screen housing 201 may attach to the dispenser 5200 through any adhesive or mechanical fastening means, and may further comprise at least one O-ring, gasket seal, or other means for sealing the apparatus.

FIG. 49A provides a view of a portion of an exemplary catheter 5400a including a two-way luer activated valve 5404 coupled to the dispenser connection assembly 5401 to allow for automatic pressure normalization between the intragastric device and pressure relative to the catheter 5400a. Such a configuration makes the catheter 5400a safer for use on patients by automating the valve system as opposed to a manual configuration, and provides a "plug-n-play" functionality. The structure and features illustrated in FIG. 49A are described in detail below.

FIG. 49B provides a view of a portion of another exemplary catheter 5400b including syringe- activated valve 5307.

FIG. 50 illustrates the dispenser device 5200 of FIG. 33 with a self-sealing valve system 5401 installed on the disconnect valve 5208.

FIG. 51 illustrates a cut-away view of the dispenser 5200 that allows one to view the internal structure of the device as displayed in FIGS. 48, 50-51 with the touch screen housing 5301 installed.

FIG. 52 illustrates the dispenser 5200 with the inflation fluid canister 2033 installed.

FIG. 53 illustrates an exemplary method 5500 by which the dispenser 5200 may operate with the intragastric device system. Initially, a program is loaded 5501 onto the dispenser 5200, specifically the circuit element 5211 which may include processors and memory units. The program loaded onto the dispenser 5200 may be one of a plurality of programs tailored for a specific application, or may be a single program with adjustable features that allow the operator of the dispenser 5200 to calibrate according to a particular situation or need. The program may provide a set of directions in a visual format on the display screen 5204, or may provide an audio set of directions.

Further to FIG. 54, the dispenser 5200 may calibrate 5502 the dispenser and peripheral objects to normalize the sensed pressure of the intragastric balloon and the sensed pressure of the catheter 5400a or 5400b and tunnel 5207 to the surrounding atmospheric pressure. This may allow for use of the intragastric device system in location of varying elevations.

The dispenser 5200 may also detect 5503 a connection of the self-sealing valve 5401 of a catheter 5400a or 5400b to the quick disconnect valve 5208. The disconnect valve 5208 may further comprise an electromechanical means for alerting the system to when a catheter assembly 5400a or 5400b is connected to the valve 5208, or when there is no peripheral connection.

Further to FIG. 55, the dispenser 5200 may activate 5504 a pre-pulse volumizer. In one example embodiment, the dispenser 5200 may release a configurable volume and pressure from the canister 2003 into the intragastric balloon to determine whether the balloon is constrained in the esophagus. In another embodiment, a metered pre-pulse may release high-pressured gas contained in the canister 2003 into the intragastric balloon through the catheter 5400a or 5400b in short, timed doses, while the dispenser 5200 measures the pressure of the balloon as the metered pre-pulse releases progresses. The pre-pulse volumizer may partially inflate the intragastric balloon allowing the operator of the device to determine 505 whether the intragastric balloon is safe to inflate.

FIG. 62 further illustrates a method whereby once the operator of the dispenser 5200 has determined the intragastric balloon is safe to be further inflated, the operator may open the plug valve and instruct the dispenser 5200 to activate 5506 the primary volumizer to inflate the balloon to its fully inflated state and provide a reading of the pressure within the intragastric balloon.

The method described in FIG. 62 may also include termination 5507 of the primary volumizer once a certain pressure within the balloon is detected.

### TOUCH SCREEN

As previously discussed, the dispenser 5200 may comprise a computer-implemented method for use in conjunction with an intragastric volume occupying system with a touch sensitive display 5204 to detect user gestures on a touch screen 5204 and translate the user gestures into commands to be performed.

In one embodiment, the touch screen 5204 may be housed in a touch screen housing 5201 comprised of a material such as a metal, or polymer or other plastic material attached to the dispenser 5200. The surface area of the touch screen 5204 that comes in contact with the housing 201 may be made waterproof by incorporating a gasket or adhesive material to seal the touch screen 5204 in contact with the housing 201. The housing 201 may have a window generally the length and width of the touch screen 5204 area to allow users to see and touch the touch screen 5204 surface when the touch screen 5204 is mounted to the housing 201.

The touch screen circuitry 5211 or the touch screen 5204 may provide I/O ports for attaching peripheral hardware, for example a printer, keyboard, mouse, monitors, headphone and microphone jacks. The circuitry 5211 or touch screen 5204 may also provide options for a wireless connection including, for example, Bluetooth, Wi-Fi or WLAN.

In one exemplary embodiment, the dispenser 5200 may include a display 5204 without a touch sensitive screen. In this example, the display 5204 may provide a visual display of the data described above and may also allow for user interface through means including, but not limited to, voice activation, Bluetooth, Wi-Fi 33 or WLAN, integrated button control, and I/O ports for attaching peripheral hardware, for example a printer, keyboard, mouse, monitors, headphone and microphone jacks.

In some embodiments, the touch sensitive display 5204 may be physically integrated with various electronic elements 5211 including, for example, one or more processors, memory (which may include one or more computer readable storage mediums), I/O ports (e.g., USB ports, micro USB ports, audio and video ports, Ethernet, Wi-Fi 33, and RFID), a battery, and a set of push button controls. The I/O ports couple the input and output of any peripherals of the dispenser 5200 to the CPU and memory. The one or more processors may run or execute various software programs and/or sets of instructions stored in memory to perform various functions for the device and to process data.

In another example embodiment, the touch screen 5204 may display a Graphical User Interface (GUI) and one or more programs or sets of instructions stored in the memory for performing multiple functions. In one example embodiment, the user interacts with the GUI primarily through finger contacts and gestures on the touch sensitive display 5204. In some embodiments, GUI functions may include digital photographing, digital videoing, digital audio playing, digital video playing, instructions for performing a plurality of procedures with the device and intragastric system, displaying diagnostic information for the intragastric system and any individual component or sensor connected to the system. Instructions for performing these functions may be included in a computer readable storage medium or other computer program product configured for execution by one or more processors The integrity of the programming may be protected using a combination login and password prompt upon startup. The programming may further be encrypted through the use of fingertip detection, smart card identification, or magnetic-read identification. These parameters may further be implemented to prevent misuse of the dispenser 5200 or to allow limited access to the use of the dispenser 5200. In another example embodiment, the GUI may comprise a memory controller, one or more processing units (CPU's), a peripherals interface, RF circuitry, audio circuitry, a speaker, a microphone, an input/output (I/O) subsystem, other input or control devices, and an external port. The device may include one or more optical sensors. These components may communicate over one or more communication buses or signal lines.

In another embodiment, the dispenser 5200 may include a memory (which may include one or more computer readable storage mediums), a memory controller, one or more processing units (CPU's), a peripherals interface, RF circuitry, audio circuitry, a speaker, a microphone, an input/output (I/O) subsystem, other input or control devices, and an external port. The device may include one or more optical sensors. These components may communicate over one or more communication buses or signal lines.

### CATHETER

A swallowable, self-inflating or inflatable intragastric balloon system according to selected embodiments includes the following components: self-sealing valve system 1000 for addition of fluid to the lumen of the balloon or to the inner container ("valve system"), a balloon in a deflated and compacted state ("balloon") and an outer capsule, container, or coating ("outer container") that contains the balloon, the outer container being optional. For self-inflating balloons, an inner capsule or other container ("inner container") that contains one or more CO₂ generating components is present inside the lumen of the balloon, the inner container being optional. For inflatable balloons, an inflation fluid source, a catheter 5400a or 5400b and tubing 5403 ("inflation assembly") are provided for inflating the balloon after ingestion or placement in the stomach. In the self-inflating balloon configuration, the valve is preferably attached to the inner surface of the balloon by an adhesive or other means (e.g., welding), and provided with an inoculation spacer to prevent puncture of the wall of the balloon and inner container by a needle or other means for injecting a liquid activation agent into the lumen of the balloon *via* the self-sealing valve. A valve providing releasable attachment of the tubing to the balloon is provided in the inflatable balloon configuration. Preferably, the self-sealing valve system attached to the balloon (e.g., on its inside surface) in the inflatable configuration is "universal" or compatible with a swallowable catheter 5400a or 5400b or a physician-assisted catheter 5400a or 5400b. The valve system serves to allow for balloon inflation using a miniature catheter 5400a or 5400b that includes a needle assembly and also provides a mechanism for detachment of the catheter 5400 after inflation has been completed.

As shown in FIG. 49A, the catheter 5400a may comprise a two-way luer activated valve 304 coupled to the dispenser connection assembly 5401 to allow for automatic pressure normalization between the intragastric device and pressure relative to the catheter 5400. Such a configuration makes the catheter 5400 safer for use on patients by automating the valve system as opposed to a manual configuration, and provides a "plug-n-play" functionality.

Further illustrated in FIG. 49A, the dispenser connection assembly 5401 may also comprise an O-ring seal 5402 at the disconnect valve 5208 connection point. The O-ring 5402 can provide a seal to prevent pressure escape at the connection point, and may alternatively be constructed of any adhesive or molding sufficient to prevent release of pressure.

The connection assembly 5401 may also comprise a one-way valve 5406 for back-flow prevention. For example, the valve 5406 may prevent backflow of ejection fluid. The valve 5406 may allow an operator to release the pressurized contents from the canister 2003 into the intragastric device, but prevent the device from re-pressurizing the canister 2003 or dispenser 5200 after emptying. It may further allow for the catheter 5400 to be removed from the dispenser 5200 and attached to another dispenser 5200 or other compatible device without releasing pressure from the intragastric device.

The connection assembly 5401 may further comprise a sealed navigation port 305 to allow an operator to navigate and control the catheter tube 5403 in areas that prevent the operator from directly handling.

### VALVE

As discussed above, a plug valve 5202 and a vent valve 5201 may be configured to be integrated with the dispenser 5200. The vent valve 5201 may be configured to normalize a pressure within the dispenser 5200 or the intragastric device to the surrounding atmospheric pressure to allow for consistent use of the intragastric system, including the intragastric balloon, in regions of varying elevation. The vent valve 5201 may further be configured to release an amount of pressure, a "pre-pulse volume" of a pressurized amount dependent on the surrounding atmospheric pressure. This pre-pulse volume may be released upon connecting the catheter 5400a or 5400b to the quick disconnect valve 5208. In an exemplary embodiment, the pre-pulse volume may be 3ml. Once the intragastric volume occupying device is inside of the patient, the release of the pressure may partially inflate the intragastric balloon. This pressure may provide information to the dispenser 5200 regarding the location of the balloon in a patient's body by comparing the amount of pressure provided by the pre-pulse with the amount of pressure sensed after the release of the pre-pulse volume. For example, once the pre-pulse volume is released into the balloon, the catheter 5400a or 5400b may sense the pressure within the balloon and transmit the pressure data for display on the touch screen 5204. If the pressure data provided shows a pressure below 7 kPa, then the balloon can be assumed to be within the stomach 20 and safe for additional inflation. If the pressure shows 7 kPa or higher, then this may indicate that the balloon is still within the esophagus, and thus unsafe for further inflation. In one exemplary embodiment, a pressure sensor attached to the dispenser 5200 will feed information to the touch screen display 5204 and touch screen display circuitry 5211 in order to display the pressure after release of the pre-pulse volume and notify the user of whether it is safe to apply additional pressure to inflate the intragastric balloon, or whether the balloon is not safe for additional pressure.

### TOUCH DISPENSER

FIGS. 54-61 illustrate an exemplary automated constant pressure Dispenser 5600, referred to herein as the Touch Dispenser. The Touch Dispenser 5600 performs the valve actuations and pressure monitoring during dissolution of the balloon capsule 40 and the initial opening of the balloon 10, referred to as "pre-pulse," to determine if the balloon is in the patient's esophagus or in the stomach 20, and then during subsequent inflation of the balloon 10, so as to remove the potential for use errors and reduces the risk of esophageal inflation. The Touch Dispenser 5600 is configured to detect possible balloon inflation in constrained spaces, such as but not limited to the esophagus or a hiatal hernia, and stops balloon the inflation so as to prevent injury to the patient. Constrained spaces have a diameter that is smaller than the diameter of the fully inflated balloon. Balloon inflation within such constrained spaces can severely injure the patient and lead to death. In addition to improving the safety profile of the Obalon Balloon System, automation of the inflation process improves reliability and repeatability of the process and reduces the potential for operator errors and reduces the risk of esophageal inflation.

In one embodiment, the Dispenser 5600 is configured to detect at least one constrained space during balloon inflation, and either (1) direct the physician to a failsafe procedure or (2) terminate the inflation procedure entirely, thereby preventing patient injury. If the physician follows the failsafe procedure, it may be possible to move the balloon from the constrained space into the stomach, and then to continue with the balloon administration procedure. If the failsafe procedure fails, and does not move the balloon from the constrained space into the stomach, then the Dispenser 5600 may terminate the balloon administration procedure. Once the procedure has been terminated, the balloon may be removed *via* endoscopy. In another embodiment, the Dispenser 5600 is configured to detect and/or distinguish between at least two constrained spaces. For example, if the patient swallowed the balloon successfully, and a first constrained space is not detected, and the dispenser may determine if the balloon is within either (1) the stomach or (2) another (e.g., second) constrained space, such as but not limited to a hiatal hernia. If a second constrained space is detected, the dispenser may be able to direct the physician to perform a second fail-safe procedure to cause the balloon to move the from the second constrained space into the stomach, and then procedure with the balloon administration procedure. If such a second fail-safe procedure fails, or if the physician is not able to perform such a second fail-safe procedure, then the Dispenser 5600 may be configured to direct the physician to remove the balloon endoscopically. In certain embodiments, there may be no second fail-safe procedure for moving the balloon into the stomach from the second constrained space, and the Dispenser 5600 will terminate the balloon administration procedure. In a further embodiment, when the balloon is detected to be in a constrained space, such as either of the first and second constrained spaces, the Dispenser 5600 is configured to vent a portion of gas from the balloon to the atmosphere, thereby reducing the pressure or stress on the patient's tissues by the constrained balloon.

In the illustrated embodiment, the Touch Dispenser 5600, or inflation system, is used to transfer an inflation gas (e.g., from the Inflation Can 5605) to an intragastric balloon (e.g., 5630, such as but not limited to the Obalon Gastric Balloon Assembly, described above) while the balloon is within a patient's stomach 20. The pressurized Inflation Can 5605 is pre-filled with a specific mass of a mixture of SF₆ and N₂ gases and utilizes a seal that maintains the gas content and purity for the useful life of the can 5605. The gas content is sufficient or great enough such that the Inflation Can 5605 can be used at any elevation within 0 to 8000ft. The seal on the Inflation Can 5603 is designed to engage with the Dispenser 5600 such that the can 5605 is only open when actuated by the Dispenser 5600. The Inflation Can 5603 has a shelf-life of at least 6 months.

The Dispenser 5600 is prepared for use outside of the patient environment and its readiness is verified prior to any balloon administration. The Device 5600 provides an automated system to accurately inflate the Balloon to the correct pressure, minimize the probability of esophageal or hiatal hernia damage, provide self-diagnostic capabilities, and provide feedback to the user. To ensure that the balloon is deployed in the correct location (e.g., the stomach) at the correct time, the Device 5600 contains pressure sensors, regulators, and valves which monitor and limit the balloon inflation pressure when necessary to ensure proper placement thereof. The Dispenser 5600 includes an internal computing device 5635, or computing component, with software that provides a Graphic User Interface and controls gas flow to allow balloon inflation at any elevation within 0 to 8000ft. The Dispenser software verifies its own readiness, e.g., it is working correctly, the batteries have sufficient energy to power the dispenser for the entire balloon administration and inflation procedure, the Inflation Can 5605 contains a sufficient amount of gas to fill the balloon 5630, and the like, prior to balloon administration and verifies balloon integrity (e.g., the balloon is inflated in a non-constrained space, such as the stomach and there are no leaks) prior to completion of the procedure.

FIG. 54 is a schematic of the Touch Dispenser 5600. In the illustrated embodiment, the Dispenser 5600 comprises seven (7) primary components to control the flow of gas and detect inflation in constrained spaces; two (2) pressure regulators (5615a, 5615b), two (2) gauge pressure sensors (5610, 5625), and three (3) solenoid valves (5620a, 5620b, 5620c). These components operate in concert to safely inflate the Balloon 5630 to the proper pressure while preventing inflation within constrained spaces.

The pressure sensors provide feedback to the software device for control of the solenoid valves. The first or Proximal Pressure Sensor 5610 measures gas pressure in the can 5605. The Pressure Regulators (5615a, 5615b) restrict the flow of gas from the Inflation Can 5605 such that pressure flow is limited in case of esophageal or hiatal hernia placement. The High-Pressure Regulator 5625a regulates pressure for the pre-pulse phase of the balloon fill procedure as well as providing controlled pressure to the Low-Pressure Regulator 5615b during the fill phase of the procedure. The Low-Pressure Regulator 5615b keeps the pressure at the second pressure sensor 5625 below a value that would cause harm to the patient, if the system attempted to fill the balloon while in a constrained space, such as the esophagus.

The High-Pressure Bypass Valve 5620a is under control of the computing device 5635 and enables the Dispenser 5600 to pressurize the pre-pulse volume Vpp. The High-Pressure Bypass Valve 5620a is only open during pre-pulse. Opening of the High-Pressure Bypass Valve 5620a will enable a pre-pulse of the gas mixture to flow at the required pressure and volume to confirm proper balloon placement (e.g., the balloon is not constrained in the patient's esophagus) and to facilitate the opening of the balloon after the capsule has softened and at least partially dissolved in the patient's stomach. Closure of the High-Pressure Bypass Valve 5620a enables use of another solenoid control valve (e.g., a Proximal or Control Valve 5620b). The Control Valve 5620b both captures the pre-pulse volume Vpp and controls gas flow during balloon inflation or filling. When the volume Vpp is charged with high pressure gas, the Control Valve 5620b is closed to capture the pre-pulse volume Vpp. During balloon inflation, the Control Valve 5620b is open to the balloon 5630 and the Control Valve 5620b dispenses controlled pulses of gas to the balloon 5630. The second or Distal Pressure Sensor 5625 measures pressure downstream of the pressure regulators and provides a feedback signal to the Computing Device 5635 for controlling the amount of gas that is dispensed to fill that balloon 5630, thereby ensuring accurate balloon inflation. A distal 3-Way Valve 5620c is used both to fill the balloon 5630 and to vent balloon pressure. When the 3-Way Valve 5620c is opened to the balloon 5630, the gas can flow from the pre-pulse volume Vpp and into the balloon 5630. When the 3-Way Valve 5620c is opened to the Exhaust Orifice 5660, gas can flow from the balloon 5630 through the Exhaust to the atmosphere, thereby reducing pressure within the balloon. When both Control Valve 5620b and the 3-Way Valve 5620c are closed, the system 5635 is able to determine if the Low-Pressure Regulator 5615b is functioning properly by measuring pressure with the Distal Pressure Sensor 5625. If the High-Pressure Bypass Valve 5620a is also closed, (e.g., all three valves are closed), the functionality of the High-Pressure Regulator 5615a can be determined by measuring pressure with the Distal Pressure Sensor 5625. In addition, the Dispenser 5600 has an Atmospheric Pressure Sensor (not shown) which is used as an indirect measurement of elevation, the information from which is used by the computing device 5635 to ensure the Dispenser 5600 inflates the balloon 5630 to an internal pressure required at the altitude at which the procedure is being performed.

The Touch Dispenser 5600 is a durable, re-usable device that may be used for up to 5,000 cycles. The Inflation Can is a single-use device and must be disposed of after use. In embodiments, the dispenser includes three solenoid valves, which are configured for use with gas and have an operational pressure range which includes 1 to 207 kPa, and to control balloon inflation without leaking. Dispenser precisely displays pressure using a digital pressure sensor with 0.1 kPa resolution. The dispenser is configured to step down the can pressure to a range that is capable of detecting when the balloon is in a constrained state during pre-pulse. To do this, the dispenser includes a pressure regulator that is not user adjustable and reduces the can pressure to 155 ± 25 kPa. The Dispenser is configured to limit the pressure during the balloon inflation process to below 70kPa. In embodiments, Dispenser and Inflation Can are configured to fill balloon to within ±0.5 kPa of the desired set pressure within the range of 8.3-17.2 kPa over the altitude range of 0-8000ft (0-2438m). In some embodiments, the Dispenser is configured to perform at least 5000 procedure cycles, and to hold pressure and function as intended. The Dynamic components, e.g., the actuator lever 5640, of Touch Dispenser can cycle through intended range of motion with Inflation can inserted into the can receiving space 5642. The Dispenser is configured to open Inflation can valve when actuator lever is closed. Touch Dispenser is configured to dispense the contents of Inflation Can to support total system procedure time goal of 10 minutes. For example, in some embodiments, the fully pressurized Dispenser and Inflation Can inflates Balloon in about 4 to 6 minutes. Touch Dispenser is configured to provide advance notification of low battery level. Touch Dispenser contains a low battery alert which will allow at least 10 procedures of additional use. Touch Dispenser is configured to have a battery life that is useful for a reasonable amount of balloon deployments. Battery life is rated at least 100 procedures with standard AA batteries that are replaceable in the field as needed. Touch Dispenser shall preserve battery when not in use. In some embodiments, Touch Dispenser is configured to automatically shut off 40 minutes or less from last user input.

In some embodiments, the Touch Dispenser is configured to seal to Inflation Can in such a way to prevent leaks. Accordingly, in some embodiments, the Inflation Can engaged in the Touch Dispenser does not leak more than 0.7 kPa over 5 minutes from a starting pressure of 413±7 kPa. The dispenser is configured to be reliable. Accordingly, the dispenser is configured to regulate the output pressure to 130-180 kPa (18.6-26.1 psi) for pre-pulse, and to regulate the output pressure to 43-53 kPa (6.2 - 7.7 psi) for balloon fill. In some embodiments, the Dispenser is configured to safely release balloon pressure. For example, the Dispenser is configured to vent the pressure in a balloon pre-pulsed in a simulated esophagus to below 7.0 kPa. Additionally, the flow of gas during pressure release is directed inside of protective shrouds of the dispenser. The Dispenser pressure sensors are configured to maintain accuracy during a low power condition. For example, the Dispenser pressure sensors are configured to maintain accuracy as described elsewhere herein if low battery condition is present.

In embodiments, the Dispenser comprises pressure gauges with adequate accuracy to ensure final balloon pressure is attained. For example, in some embodiments, the Dispenser includes a balloon pressure gauge (distal sensor) with accuracy of ±0.8 kPa (0.4%) over 0 to 207 kPa. In a further example, the Dispenser includes a can pressure gauge (proximal sensor) with accuracy of ±1.6 kPa (0.4%) over 0 to 414 kPa. In another further example, the final inflation pressure range of the Touch Dispenser does not exceed 9.5-13 kPa so as to ensure that the final pressure balloon will not exceed 8.3-17.2 kPa.

In embodiments, the Touch Dispenser comprises one, two or more pressure gauges that maintain accuracy over the dispenser's useful life. For example, the Dispenser's balloon pressure gauge is configured to have an accuracy of ±0.8 kPa over 0 to 207 kPa after 5000 cycles of 0 kPa to 207 kPa. In another example, the can pressure gauge is configured to have an accuracy of ±1.6 kPa over 0 to 414 kPa after 5000 cycles of 0 kPa to 414 kPa.

In embodiments, the Dispenser and Inflation Can are configured to operate normally in the temperature range of 15°C to 25°C and 30-85% relative humidity (i.e., RH). Further, the Dispenser may be configured to withstand storage conditions of 0°C to 40°C, and 30% to 85% RH, and 0-3000 meters elevation.

The Obalon Touch Balloon System (the "System") is designed to assist weight loss by partially filling the stomach, so as to provide the patient with a feeling of fullness. The system consists of up to 3 intragastric balloons placed during a 6-month period. In some embodiments, up to three balloons may be placed in the patient's stomach 20 for a period of 8 months, 9, months, 10, months, 11 months, 12 months or longer. The balloons are swallowable by the patient using normal peristalsis, or peristaltic waves, in that the balloons are delivered *via* capsule. Each balloon is placed individually within the first 3 months. All 3 balloons are removed at the end of the treatment period. For example, in the case of a 6-month treatment period, all three balloons are removed from the patient's stomach 20 six months after the first balloon was placed (i.e., swallowed by the patient and then inflated).

For administration, a balloon and catheter assembly are used, such as described elsewhere herein. In some embodiments, each balloon is contained within a USP grade hydroxypropyl methylcellulose (HPMC) capsule, which is attached to the catheter. When it is swallowed by the patient, the balloon capsule delivers the balloon in a similar manner that a medicinal capsule delivers pharmaceuticals, *via* peristalsis. The catheter comes pre-attached to the compacted balloon's radiopaque, self-sealing valve, as described elsewhere herein.

The administration (placement) procedure requires no sedation. The capsule and a portion of the attached capsule are swallowed by the patient. The catheter is then attached to the Obalon Touch Dispenser, which contains an engaged Obalon Touch Can (a can containing nitrogen-sulfur hexafluoride gas mixture) to fill the balloon. After the patient swallows the balloon capsule, radiography is performed prior to inflation, to ensure that the balloon is in the patient's stomach 20 (e.g., visualized by the radiopaque marker). The radiographic method may be fluoroscopy or digital radiography since both provide real-time image of the balloon using low levels of radiation with immediate imaging feedback. Once there is radiographic confirmation that the balloon (e.g., the radiopaque marker associated with the self-sealing valve) is below the gastroesophageal junction, and therefore not constrained in the patient's esophagus, then the balloon is inflated.

In some embodiments, a fully inflated single balloon is an ellipsoid, a spheroid or an oblate spheroid with a volume of approximately 250 cc. When 3 balloons are placed in the patient's stomach, the total balloon volume is approximately 750 cc. However, it is foreseen that the balloon could include larger or smaller volumes. For example, balloons for use with teenagers, or smaller people, can be configured with a volume of less than 250 cc, such as but not limited to approximately 100 cc, approximately125 cc, approximately 150 cc, approximately 175 cc, approximately 200 cc, and approximately 225 cc. In another example, the balloon is configured with a volume greater than 250 cc, such as but not limited to approximately 275 cc, approximately 300 cc, approximately 325 cc, approximately 350 cc, approximately 375 cc and approximately 400 cc. In still another example, the patient may receive only two balloons or as many as four balloons.

After inflation is complete, the catheter is ejected from the balloon valve and retrieved, leaving each balloon free-floating in the patient's stomach.

Balloon use may require the concurrent use of proton pump inhibitors for the duration of implantation. For example, clinical studies have shown that use of 40 mg/day of omeprazole or an equivalent dosage of similar medications is required over the duration of use. Some patients may require an anti-emetic and anti-spasmodic medication at least 24 hours prior to administration and such medications may be prescribed in conjunction with balloon use for up to 5 days beyond balloon administration. In embodiments, Pre-existing GI pathology is ruled out prior to placement of any balloons by conducting a comprehensive medical history and an upper endoscopy (e.g., and upper GI) to determine a patient's suitability for the procedure and to ensure the patient is not contraindicated for device use.

The inflation system is prepared prior to balloon administration. The dispenser is first powered on, and then the dispenser lever is lifted and the can is inserted into the shuttle. The lever on the dispenser is closed to actuate the can valve and pressurize the system. The system performs a series of electrical and pressure tests to ensure that the inflation system is working properly. Once these tests are complete, the dispenser displays a screen to indicate that the system is ready for use. Throughout the procedure, the dispenser screen displays pressure readings, images, and touchscreen buttons to provide information and control to the user.

After the balloon capsule is swallowed, the balloon catheter is attached to the Touch Inflation System. All entries and exits within the dispenser and catheter connections are sealed and it is imperative that the catheter connection is fully secured during the procedure to maintain a closed gas pathway between the can and balloon.

### EXAMPLES

### EXAMPLE 1:

FIG. 62 illustrates an exemplary 5700 by which the Touch Dispenser 5600 5200 may operate with the intragastric device system. Initially, a program is loaded 5701 onto the dispenser 5600, specifically the circuit element 5635 which may include processors and memory units. The program loaded onto the Dispenser 5600 may be one of a plurality of programs tailored for a specific application, or may be a single program with adjustable features that allow the operator of the Dispenser 5600 to calibrate according to a particular situation or need. The program may provide a set of directions in a visual format on the display screen, or may provide an audio set of directions. The Dispenser 5600 may calibrate the dispenser and peripheral objects to normalize the sensed pressure of the intragastric balloon and the sensed pressure of the catheter 5645 and the pre-pulse volume Vpp to the surrounding atmospheric pressure. This allows for use of the Dispenser 5600 in location of varying elevations.

Further to FIG. 62, the Dispenser 5600 may detect 5702 the presence of the canister 5605. The Dispenser 5600 may also detect 5503 a connection of the self-sealing valve of a catheter 5645 to the dispenser distal fitting 5650. The distal fitting 5660 may further comprise an electromechanical means for alerting the system to when a catheter 5645 is connected to the fitting, or when there is no distal connection.

Further to FIG. 62, the Dispenser 5600 may activate 5704 a pre-pulse volumizer. In one example embodiment, the dispenser 5200 may release a configurable volume and pressure from the pre-pulse volume Vpp into the intragastric balloon 5630. The Dispenser 5600 then determines 5705 if the balloon pressure is within a first safety threshold. If the dispenser determines that the balloon pressure is not within the first safety threshold, then the device determines that the balloon is constrained in a space such as the esophagus. In the event that the balloon is determined to be constrained in the esophagus, then the physician may be directed 5706 to perform a Fail-Safe Procedure that may move the constrained balloon from the constrained space and into the stomach. A Fail-Safe Procedure may include venting a portion of the gas within the balloon, either *via* the Exhaust 5650 or *via* disconnecting the catheter 5645 from the fitting 5650 and using a syringe attached to the catheter 5645 to withdraw the gas within the balloon 5635. The Fail-Safe Procedure may also include encouraging the patient to drink water or swallow a small amount of soft food to push the balloon 5635 out of the esophagus and into the stomach. Once the Fail-Safe Procedure has been performed, the Balloon administration procedure may be restarted by returning to step 5704. In some embodiments, the Fail-Safe Procedure 5706 may be repeated two or more times. If the patient fails to swallow the balloon (e.g., move the balloon from the confined space of the esophagus and into the stomach), then the Device 5700 may direct the balloon to be removed, such as *via* endoscopy.

When the balloon pressure passes the first safety threshold 5705, the system begins filling the balloon 5635. To fill the balloon, the system releases small volumes of gas, such as an amount of gas equal to the Vpp, into the balloon. When an amount of gas is released into the balloon, the system may determine 5707 if the balloon pressure passes a second safety threshold and/or if the balloon pressure has reached a final target balloon pressure. If the balloon pressure is within the second safety threshold but below the target pressure (e.g., step 5708), then the system may deliver another small volume of gas (e.g., Vpp) to the balloon. If the balloon pressure is within the second safety threshold but above the target pressure (e.g., step 5709), then the system may open the Exhaust 5660, to allow a small volume of gas to bleed from or flow out of the balloon. Steps 5708 and 5709 may be repeated iteratively, until the balloon pressure equals the target pressure 5710. Once the balloon pressure is stabilized at the target pressure 5710, the catheter 5645 may be ejected from the balloon 5630 and removed from the patient, such as described elsewhere herein. Once the catheter 5644 has been removed, the patient may be free to go home. However, if the balloon pressure fails the second safety threshold 5711, then the balloon 5635 may be constrained within a second constrained space, such as a hiatal hernia. In certain embodiments, the physician may perform a second fail-Safe procedure to move the balloon 5635 from the second constrained space into the stomach, and then continue with the balloon administration procedure. In other embodiments, the system may terminate the balloon administration procedure, and optionally direct the physician to remove the balloon endoscopically.

### EXAMPLE 2:

The Touch Dispenser 5600 was used in an animal model to demonstrate that the Dispenser 5600 is capable of detecting when the Obalon balloon is in an anatomically confined space during the pre-pulse and balloon inflation phases of the balloon administration procedure. Pre-pulse is a means of detection to determine if the balloon has passed safely from the patient's esophagus into the stomach. During pre-pulse, a small volume Vpp within the Dispenser 5600 is filled to a higher pressure than the pressure that used for balloon inflation. This higher pressure is limited to between 130-180 kPa as flow from the inflation can is directed through a High-Pressure Regulator to step the pressure down from the pressure within the Can 5605. This high pressure volume (e.g., Vpp) is then isolated from the can and delivered to the balloon 5630 through the catheter 5645. The pressure detected by the Distal Pressure Sensor 5625 remains high while the balloon 5630 is still inside of the capsule. As the capsule material disintegrates, the balloon begins to unfold, which the system detects as a decrease in balloon pressure.

The Dispenser waits until the pressure is below 60 kPa and determines the balloon's location based upon the balloon's pressure value and the rate of pressure decay. If the pressure is less than 60 but greater than 17.2 kPa and the rate of pressure decrease is less than 0.2 kPa/s, then first safety threshold has been exceeded, the dispenser will provide indicia to the physician that the balloon is within a constrained space, such as within the esophagus. If the pressure continues to decrease at a high rate to below 17.2 kPa, the system with evaluate the rate of change for a rate of change that is less than 0.2 kPa in 3 seconds before alerting the user of an esophageal placement. If the pressure decreases to below 7 kPa, the first safety threshold has been passed, and the balloon is unconstrained. Whenever a constrained state is detected the dispenser will exhaust the pressure to below 7 kPa, so as to relieve pressure on the anatomical space.

After a successful pre-pulse, the dispenser will continue sensing for a constrained balloon during the fill portion of the procedure by looking for a pressure that is greater than 11 kPa during the first balloon fill phase. If the dispenser senses a high pressure, it will alert the user *via* suitable indicia, such as but not limited to a sound and an image on the dispenser's touch screen. The dispenser or instructions for use may instruct the user how to proceed (e.g., removing gas from the balloon and drinking water) and provide a chance to continue the balloon administration procedure. If it detects another high pressure, it will alert the user. The dispenser may allow three attempts to clear a constrained fill situation before a final error is shown, which terminate the balloon administration procedure.

To evaluate severity of esophageal injury when a balloon was constrained in the esophagus, while inflating the balloon with the Touch Dispenser, esophageal inflations were evaluated in three animals. Each animal was used for three balloons that were partially inflated in three locations in the esophagus, at locations that were 10 cm apart. For each partial deployment, pre-pulse and three confined pressure scenarios were performed. A dispenser was prepared. To create the, a balloon was placed in the animal's esophagus *via* endoscopy. The catheter was attached to the Touch Dispenser and a pre-pulse gas was delivered to each balloon. The initial balloon pressure was high, about 130kPa. The pressure began to decrease and the balloon was observed to inflate. The balloon pressure stabilized between 30-45 kPa. The Touch Dispenser provided indicia that the balloon was constrained and exhausted an amount of gas from the balloon, such that the balloon was observed to deflate. After the initial partial deflation, the administration procedure was continued without moving the balloon. When the balloon continued to inflate, the constrained balloon indicia was again provided. Again, the dispenser allowed the balloon to deflate. This was done two additional times before the dispenser displayed the final warning and required the user to turn off the dispenser. The balloon was then manually deflated with a 60 cc syringe and removed from the animal's esophagus. This was done in two other locations for a total of three balloon deployments in each animal.

No redness or trauma was observed endoscopically after the esophageal testing. One animal was sacrificed and the esophagus examined at necropsy. No damage was observed. Tissue was sent for histological evaluation and nothing of concern was noted. The other two animals were survived and will be sacrificed at 30 days and the esophagus will be examined at necropsy.

### EXAMPLE 3:

A Nitrogen Fill System and disposable Procedure Canister devices are provided as accessories to the Obalon Gastric Balloon (OGB). In use, the cap is removed from the valve of a disposable Procedure Canister. This disposable Procedure Canister is inserted into a Procedure Canister and the lever is closed to engage the valve on the disposable canister. A lack of a fluid path of a lower pressure gradient keeps the fluid from expelling. The Nitrogen Fill System is attached to an Accessory Kit *via* a luer fitting. A 3-port, 2-position valve on the Accessory Kit is confirmed closed before opening the 90° valve. Appropriate pressure is confirmed *via* the digital gauge, then a standard OGB System inflation procedure commences and the fluid path is opened to the balloon in the body. After the procedure, the disposable Procedure Canister is removed from Nitrogen Fill System and properly disposed of. The Nitrogen Fill System is re-used and can have a useful life, e.g., of at least 1 year.

The Procedure Canister is an appropriate size to fit into the Nitrogen Fill System, e.g., a major outer diameter of the non-valved canister is 45 ± 1mm. The Procedure Canister is an appropriate size to fit into the Nitrogen Fill System, e.g., a height of the non-valved canister is 115 ± 1mm. The Procedure Canister is pressure resistant, e.g., having an 18-bar pressure resistance. The internal volume of the Procedure Canister is adequate to fill a Balloon Kit at appropriate fill pressure, e.g., a brimful volume of non-valved canister is 161 ± 3 cm3. The Procedure Canister is accurately pressurized, e.g., sampling direct pressure measurement are within ±1 psi or ±7 kPa. The assembled Procedure Canister is bubble tight, e.g., pressurized disposable canisters are tested visually for leaks *via* a water bath bubble detection test. A volume of the canister allows for enough air at a pressure that is not great enough to rupture the catheter, e.g., a pressure of fully pressurized canister is not to exceed 75 psi. The Nitrogen Fill System has the ability to connect to the Balloon Kit. The Nitrogen Fill System contains a luer fitting that connects to a swallow catheter. The Nitrogen Fill System has an effective valve for inflation.

The Nitrogen Fill System has a ¼ turn valve. The valve is designed for gas and has an operational pressure range which includes 1 to 75 psi. The Nitrogen Fill System precisely displays pressure. The Nitrogen Fill System contains a digital gauge with 0.01 psi resolution or 0.1 KPA resolution. The Pressurized Nitrogen Fill System is bubble tight, e.g., no bubbles per bubble leak test in 130°F water bath for 1.5 minutes. The Nitrogen Fill System fills the balloon to proper pressure, e.g., 2.0 +/-.5 psi at sea level. The pressure is retained by the assembled Nitrogen Fill System effectively enough to not place balloon fill pressures outside of specification. The mass of contained gas is not to drop more than <5 psi over the course of 1 year. The Nitrogen Fill System is reliable for at least 1 year, and the Nitrogen Fill System is good for at least 1000 actuation cycles. The Procedure Canister is compatible with the Nitrogen Fill System. The Dynamic components of the Nitrogen Fill System can cycle through intended range of motion with the disposable canister inserted.

The Nitrogen Fill System opens the disposable dispenser valve when the receiver is closed. The Nitrogen Fill System is capable of dispensing contents of the canister to support a total system procedure time goal of 5-10 minutes. The fully pressurized canister can be dispensed within 30 seconds. The Nitrogen Fill System's digital gauge provides advance notification of a low battery level. The digital gauge contains a low battery indicator. Nitrogen Fill System's digital gauge battery life shall be useful for the service life of the device. The digital gauge battery life is rated at least 2000 hours. The batteries are standard and replaceable in the field as needed. The digital gauge preserves the battery when not in use, e.g., by automatically shutting off 60 minutes from last button press. The Nitrogen Fill System seals to the canister in such a way to prevent leaks. The canister engaged in the Nitrogen Fill System does not leak more than 0.1 psi over 5 minutes. The Nitrogen Fill System's Digital Pressure Gauge is electromagnetically compatible. The Nitrogen Fill System's gauge readings are easy to read, e.g., the gauge size is at least 3" diameter with a digital display. The Nitrogen Fill System's pressure gauge is intuitive to operate, e.g., the gauge has one touch on and off button that is clearly labeled. The actuator on Nitrogen Fill System for inflation of the balloon is intuitive, e.g., a colored valve lever with only on and off positions is used for initial balloon fill from the Nitrogen Fill System. The Nitrogen Fill System is easily inserted into the Nitrogen Fill System, e.g., the Nitrogen Fill System opening is large and obviously apparent.

The mass of nitrogen contained by Procedure Canister is appropriate to attain a desired final balloon pressure, e.g., the canister sample is weighed before and after filling to assure .52 ± .01 grams of gas. The Nitrogen Fill System has a digital gauge with adequate accuracy to ensure final balloon pressure is attained, e.g., the Nitrogen Fill System's digital gauge has an accuracy of 0.25% of Full Scale. The Nitrogen Fill System has a digital gauge that maintains accuracy over its useful life, e.g., the Nitrogen Fill System's digital gauge has an accuracy of 0.25% of Full Scale after 1000 cycles of 0 psi to 30 psi or 0 KPa to 26 kPa. The Nitrogen Fill System is preferably operated under reasonable environmental conditions, e.g., temperatures of from -18°C to 55°C and relative humidity of from 30% to 85%. The altitude classification system is used in altitude of a < 2000m (61 to 101 kPa).

The Obalon Gastric Balloon System (the "System" or OGB) is designed to assist weight loss by partially filling the stomach 20 and inducing satiety. The System consists of up to 3 intragastric balloons that are placed non-invasively in the stomach 20 (*via* a catheter-capsule assembly) and reside in the stomach 20 for up to 3 months (12 weeks). For administration, each balloon is contained within a medical-grade porcine gelatin capsule, which is attached to a miniature catheter. The balloon capsule delivers the balloon in the same manner that a medicinal capsule delivers pharmaceuticals. The catheter comes pre-attached to the compacted Balloon's radio-opaque, re-sealing valve. For administration of the device (placement), the catheter/capsule is swallowed by the patient. The catheter is then attached to a Procedure Canister that contains a disposable Nitrogen Fill System that is used to fill the balloon. After the patient swallows the balloon capsule, radiography is done to ensure the balloon is in the stomach 20 after swallow (visualized by the radio-opaque marker). The radiographic method can be fluoroscopy since it provides a real-time picture of the balloon using low levels of radiation. A fully inflated single balloon is an ellipsoid with a volume of approximately 250cc. When 3 balloons are placed, the total balloon volume is 750cc. The administration procedure requires no sedation. After inflation is complete, the catheter is manually ejected from the balloon valve and retrieved by the physician; leaving the balloon free-floating in the patient's stomach 20 for up to 3 months.

Balloon use can employ the concurrent use of Proton Pump Inhibitors for the duration of use, e.g., 40 mg/day of pantoprazol or an equivalent dosage of similar medications. It is likely an effective treatment for undiagnosed pre-existing esophagitis and gastritis which should enhance tolerability of the devices in residence. Antiemetic and spasmolytic agents can be given immediately following balloon placement and given as needed while the balloon(s) are in the stomach.

Clinical trials have shown that one balloon can be placed initially and subsequent balloons can be placed later in the 3-month period (FIG. 1). Determination of whether a patient requires additional volume should be made based on patient weight loss progress and reported satiety levels. Additional balloons are placed in the same manner as the first balloon placement; requiring only radiography for placement.

The balloon helps the patient eat less food at each sitting. The selection of less calorie dense foods in addition to the balloon(s) will only help facilitate weight loss. The balloons are intended to remain in the stomach 20 for 3 months (12 weeks) from the time of placement of the first balloon. All balloons placed are removed at the end of three months using standard endoscopic methods. The device(s) are removed by a trained healthcare professional proficient in gastroscopy.

The health care setting in which the device is to be used has access to fluoroscopy or digital x-ray at the time the device is administered, to ascertain the balloon/capsule placement in the stomach 20 prior to inflation. In addition, the prescribing physician has immediate access to an endoscopy unit and to personnel proficient in gastroscopy and foreign object retrieval should problems arise during administration. Gastroscopy equipment and persons trained in foreign object retrieval are employed for device removal.

The Obalon Gastric Balloon System (the "System") is indicated for temporary use for weight loss in overweight and obese adults with a BMI of 27 or greater who have previously failed a supervised weight control program. The Obalon Gastric Balloon system is intended to be used in conjunction with a diet and behavior modification program. Up to 3 Obalon Gastric Balloons may be placed in the stomach 20 across a 3-month (12-week) period based on the individual's weight loss progress and satiety levels. The maximum placement period for the Obalon Gastric Balloon(s) is 3 months (12-weeks) and all balloons must be removed at that time or earlier.

All components are supplied non-sterile. The System can include the following: a Placebo Capsule Assembly including a capsule of the same material, size, shape and weight as the actual device but that does not contain a balloon or catheter. The capsule is filled with food-grade sugar to simulate device weight; an Obalon Gastric Balloon Assembly including one folded balloon contained in a swallowable Gelatin Capsule and attached to 1 disposable, flexible Catheter Delivery System; an Accessory Kit including two 3 cm³ Syringes, an extension tube and stopcock with a 3-way valve, and a 60 cm³ syringe; a Procedure Canister which is a reusable component and is used in conjunction with the disposable Nitrogen Fill System with the Digital Pressure Gauge attached; two AAA Batteries; and a Nitrogen Fill System filled with 150 cm3 of nitrogen. Other items that can be used in conjunction with administering and/or removing the System include a small clean bowl, bottled water, timer / clock, digital X-Ray or fluoroscope, vacuum aspiration source, gastroscope, gastroscope Injection Needle (minimum length can be 6 mm and minimum needle gauge size can be 23) compatible with the working channel of the gastroscope, and a rat tooth with alligator jaws grasping forceps (minimum opening width can be 15 mm) or other commercially available endoscopy retrieval tools such as two-prong graspers, compatible with the working channel of the gastroscope.

The Nitrogen Fill System ("Dispenser") is prepared for use by first ensuring that the Dispenser is correct for the altitude of the facility or contains a barometric pressure compensation valve to adjust the starting pressure to ensure proper balloon end pressure. Failure to use the correct Dispenser can result in a deflated or over inflated balloon. The Dispenser valve is in the open position. The "ON" button is pressed to turn on the Dispenser Gauge. The extension tube and stopcock with 3-way valve is removed from the Accessory Kit packaging. The luer lock plug is removed from the end of the Dispenser. The luer lock plug is saved and put back on the Dispenser after the procedure is complete to keep the Dispenser free of debris. The proximal end of the luer connector of the extension tube (from the Accessory Kit) is connected to the Dispenser with valve open. The stop cock valve is in the closed position to stop the flow of gas. The cap is removed from the Disposable Canister. The canister is configured to not fit into the Dispenser if this cap is not removed. With the lever in the 'open' position, the disposable canister is inserted straight down into the Dispenser. The lever is set to the 'closed' position to secures the disposable canister in place. The initial reading on the gauge is confirmed to be between 257 kPa and 297 kPa, to ensure proper balloon inflation. The valve on the Dispenser is closed by rotating the valve clockwise (to the right) (FIG. 43). The Dispenser valve is in the closed position before proceeding to next steps to ensure the proper positioning and status of the balloon capsule prior to proceeding to the balloon fill step. The 3 cm³ syringe is removed from its packaging and filled with 1.5ml of room temperature water and set aside. A small clean bowl half-way with water. The Obalon Gastric Balloon Capsule and Catheter Delivery System are removed from their packaging.

Prior to the patient swallowing the functioning device, it is recommended that they first swallow the placebo capsule. The purpose of this procedure is to determine which patients will and will not be good candidates to have the actual device placed. The placebo capsule should go down easily. If the patient swallows the placebo capsule without problem, it is recommended that they proceed with the Balloon therapy.

The Obalon Balloon capsule is administered to the patient using a normal pill swallowing method. Endoscopy is not required for placement. Fluoroscopy (or digital x-ray) is employed during the placement procedure to verify placement of the balloon in the stomach 20 prior to inflation of the device. Any existing balloons are also imaged to confirm their integrity prior to swallowing another capsule. The total placement time is less than 15 minutes for each balloon placed.

Should a patient have a strong gag reflex; a topical anesthetic may be applied immediately before capsule administration. To place the balloon, the patient may have no lipstick, gloss, or emollients on their lips that could affect the administration process. The patient may stand or sit upright, and three large gulps of water are administered to prepare for capsule administration. The patient can be instructed not to bite down on the catheter, to close his/her mouth on catheter, to hold onto the catheter by hand, or to grab the catheter. The Capsule/Catheter is wet by submerging into the bowl of water for no more than 10 seconds. Within 1 minute of submerging the capsule in the water, the patient is handed the capsule/catheter and instructed to place the capsule immediately in the mouth and swallow the capsule with another large glass of water. The Proximal Catheter Port is held outside of the patient's mouth. The time of placement of the capsule/catheter in the mouth for swallow is documented. The patient is given additional water or juice (at least 100 ml) after swallow. The patient remains in an upright sitting or standing position the entire time. The patient is asked to continually drink water or juice to facilitate peristalsis of the capsule/catheter if the balloon has not visibly passed into the stomach. Once swallowed, the proximal end of the capsule/catheter assembly remains outside of the patients' mouth until after the balloon is filled. The catheter may have markings that are to be used as a reference guide to help determine how far the catheter has traveled after swallow or to be used with or without the radiography verification and with or without measurement provided by the digital gauge. When the 'bull's-eye' marking is at the patient's teeth, this indicates that the balloon is approximately 45 cm down the esophagus and fluoroscopy is used at this time to determine if the balloon is in the stomach. A method to confirm proper balloon placement prior to inflation can be with radiography (fluoroscopy/digital x-ray). The catheter markings can be used for additional reference as to when to properly perform the radiography or could be used alone to verify the length traveled into the stomach.

The Dispenser with the extension tube from the Accessory Kit is connected to the balloon catheter by connecting the catheter to the male luer port on the 3-way stopcock of the extension line previously connected to Dispenser. The lure fittings are tightened snugly with fingers. The valve on the Dispenser is in the closed position before proceeding to next steps to ensure the proper positioning and status of the balloon capsule prior to proceeding to the balloon fill.

Approximately 1-2 minutes after swallow, digital x-ray or fluoroscopy is performed with the patient standing or sitting upright, to determine location of the radio-opaque balloon marker in the stomach. At least 3-5 minutes after swallow, a second verification of device location is performed using the Dispenser. This is accomplished by turning the Dispenser Digital Gauge on by pressing the "ON" button, turning on the Gauge Backlight, pressing the "ON" button on again, and turning the 3-way valve on the stopcock counterclockwise 90 degrees until the valve stops to open the flow of gas from extension tubing to the balloon. The pressure initially drops on the pressure gauge by approximately 20 kPa and then proceeds to below 7 kPa when the capsule dissolves. This takes approximately 45 seconds but no longer than 4 minutes. If the pressure remains above 7 kPa, then the capsule has not dissolved sufficiently or the catheter is kinked. Having the patient drink more liquid may facilitate capsule dissolution. The pressure is monitored for up to 4 minutes. If after this time the pressure has not dropped to less than 7.0 kPa then the capsule has not dissolved or there is a kink in the catheter. Balloon fill steps are not initiated until the radio-opaque balloon valve is visualized in the stomach 20 and the Dispenser pressure gauge reads less than 7.0 kPa. During inflation, if there is indication of inflation in a constrained space (by pressure readings or patient symptoms) shut off the gas flow by closing the valve on the Dispenser, detach the catheter from the Dispenser, and evacuate the gas from the balloon with a 60 cm³ syringe. The balloon can then be removed endoscopically. If the gauge reads less than 7.0 kPa, then the balloon can be filled. The balloon is not disconnected from the catheter prior to balloon fill completion. In the event of a premature disconnect, the catheter is retrieved by pulling it out and then the balloon is endoscopically punctured and removed.

The Disposable Nitrogen Canister contains 150 cm³ of nitrogen that is transferred into the balloon to fill a single balloon to 8.3-17.2 kPa and 250 cm³ of volume. When the gauge remains steady after decreasing from the initial set inflation pressure, the balloon is filled to the desired volume of 250 cm³ at the desired pressure of 13.8 kPa in approximately 2 minutes. To fill the balloon, the Dispenser valve is turned to the on/open position. Equilibrium is observed about 2 minutes after opening the valve (inflation time). The final readout pressure on the Dispenser's digital gauge is verified as stable and reading 8.3-17.2 kPa. If the pressure is outside of the specified range, endoscopic removal of the balloon is performed. The pre-filled Syringe is attached to the Stopcock with a 3-way valve. The Stopcock with a 3-way Valve is rotated back 90 degrees to close the flow of gas from the Dispenser. The 3-way stopcock valve is not rotated until attached to the pre-filled syringe so as to avoid reducing the final starting pressure in the balloon such that the balloon may not maintain its volume for the 3-month period. The gauge is not zeroed by holding the zero button while pressure is being transferred, to avoid the need to then remove the balloon endoscopically.

The catheter is retrieved by pushing the 1.5 cm³ filled Syringe plunger in one rapid and deliberate motion, so as to detach the catheter from the balloon valve in the stomach. If the catheter does not detach after the first attempt, the second 1.5 cm³ water filled syringe can be used to again attempt to remove the catheter. On the second attempt, it is ensured that the catheter is straight (there are no kinks) and that the plunger is pressed in a rapid and deliberate motion. Force is used when pushing the Syringe, and the step is not performed slowly to avoid the catheter not ejecting properly. If catheter remains attached to the balloon, a second 3-ml syringe can be half way filled with water, and detachment can again be attempted. To facilitate detachment, the patient lifts his/her chin up to help reduce any gag reflex, then the catheter is slowly pulled out of the patient's mouth. The catheter and the needle inside the needle sleeve are visualized (white protective hub that came attached to the capsule device) to ensure the needle is intact. If the needle is not inside the needle sleeve, then the balloon is removed. The catheter is separated from the Stopcock with a 3-way valve by unscrewing the luer lock. The location of the balloon can be reverified using X-ray or fluoroscopy.

The disposable canister can be removed from the Dispenser and discarded. To remove, the Dispenser lever is moved to the 'open' position, and the canister is pushed up from the opposite side of the Dispenser, or the Dispenser is flipped upside down and the disposable canister naturally falls out. The Dispenser is reused for the next balloon placement.

The patient can be advised to drink liquids for the first 24 hours and then transition to soft solids for the next 24 hours (in the first 48-hours after placement). Patients are instructed not drink alcohol, sodas or other "fizzy" or carbonated drinks. After 3 days, patients are able to return to solid foods and follow the diet and behavior modification program provided to them by their physician.

While placement of the balloon does not require endoscopy; it can be desirable that a trained endoscopist be readily available should there be a problem with swallow of the balloon or an undiscovered swallowing disorder is detected during the procedure. The following should be considered if a patient is unsuccessful at his/her first placement attempt. If the device does not pass the pharynx in the patient's mouth after 30 seconds of attempting swallow, the capsule is removed from the mouth. A new wetted balloon capsule/catheter assembly is used. If the patient fails two attempts, this issue is discussed with the patient and it is determined if the patient remains a good candidate for the therapy. If the failure to swallow is due to anxiety, standard methods to reduce the patient's anxiety can be used. Esophageal transit of the device can be facilitated by use of clear carbonated beverages.

Patients are advised to report a change in satiety (i.e. increased hunger), and/or weight gain as this may be a sign that an additional balloon for therapy might be warranted. If multiple balloons have been placed and the patient reports a change in satiety levels (i.e. reduced early satiety), this may be a sign of balloon deflation. Balloon deflation can be evaluated using radiography (film x-ray, digital x-ray, or fluoroscopy) and gastroscopy as appropriate. Patients are advised to contact their physician if the frequency of adverse events experienced is more than anticipated or becomes intolerable. Concurrent use of Proton Pump Inhibitors can be desirable for the duration of use, e.g., 40 mg/day of pantoprazol or an equivalent dosage of similar medications, in that it is likely an effective treatment for undiagnosed pre-existing esophagitis and gastritis which may enhance tolerability of the devices in residence. Antiemetic and spasmolytic agents can be given immediately following balloon placement and given as needed while the balloon(s) are in the stomach.

After 12 weeks of use the balloon or balloons are removed from the patient. The procedure is conducted using a working length endoscope less than 1200 mm and the inner diameter is compatible with the accessory tools suggested for puncture and retrieval of the balloon. Suggested tools include a needle instrument, e.g., an injector needle in a Teflon Sleeve 23G × 6 mm or similar having a lumen for suction, a Rat Tooth Grasper with Alligator Jaws or Two Jaw Grasping Forceps (with a minimum opening width of 15 mm); or a two-prong grasper with same minimum opening. Other retrieval tools may be acceptable for retrieving the balloons. Retrieval procedures in general are conducted per the gastroscope manufacturer's instructions for retrieving foreign objects. The endoscopy procedure performed is similar to that of an interventional or therapeutic procedure, however tailoring the endoscopic approach according to the unique product features is recommended, e.g., balloons should only be punctured once, so that the maximum amount of gas can be aspirated (*via* vacuum) from them, and a lesser degree of stomach inflation (less air insufflation) allows for easier puncture of the balloon. A typical capsule may include as ingredients porcine gelatin, water, methylparaben, propylparaben, and sodium lauryl sulfate. A typical balloon may be constructed from nylon and polyethylene (as wall materials), silicone (in the valve), and titanium (as a radioopaque component). The Dispenser is typically constructed of Stainless Steel, 6061 AL, Brass, Acetal, and Silicone. The canister contains 150 cm³ of Nitrogen at 18 barr.

Preferably, patients are fasted at least 24 hours or per hospital protocol for gastroscope procedures to ensure the stomach 20 is empty and the balloon(s) are therefore easily visible. The patient is anesthetized per hospital and physician recommendations for gastroscope procedures. The gastroscope is inserted into the patient's stomach, and a clear view of the filled balloons is obtained through the gastroscope. The needle instrument is inserted down the working channel of the gastroscope. The valve of the balloon is located and the balloon is punctured with the needle only once (at the opposite end of the valve if possible for easier removal). Suction is applied and balloon gas is aspirated using a large syringe (60cc) or aspiration tube. The needle is removed from the working channel, and the graspers are inserted through the working channel. The balloon is grabbed with the graspers at the opposite end of the valve. With a firm grasp on the balloon, the balloon is slowly extracted up through the esophagus, removing the balloon through the mouth. The removal procedure is repeated for the remainder of the balloons, if any. The present invention has been described above with reference to specific embodiments. However, other embodiments than the above described are equally possible within the scope of the invention. Different method steps than those described above may be provided within the scope of the invention. The different features and steps of the invention may be combined in other combinations than those described. The scope of the invention is only limited by the appended patent claims.

All references cited herein are incorporated herein by reference in their entirety. To the extent publications and patents or patent applications incorporated by reference contradict the disclosure contained in the specification, the specification is intended to supersede and/or take precedence over any such contradictory material.

To the extent publications and patents or patent applications incorporated by reference herein contradict the disclosure contained in the specification, the specification is intended to supersede and/or take precedence over any such contradictory material.

Unless otherwise defined, all terms (including technical and scientific terms) are to be given their ordinary and customary meaning to a person of ordinary skill in the art, and are not to be limited to a special or customized meaning unless expressly so defined herein.

Terms and phrases used in this application, and variations thereof, unless otherwise expressly stated, should be construed as open ended as opposed to limiting. As examples of the foregoing, the term 'including' should be read to mean `including, without limitation' or the like; the term 'comprising' as used herein is synonymous with 'including,' 'containing,' or `characterized by,' and is inclusive or open-ended and does not exclude additional, unrecited elements or method steps; the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise. In addition, as used in this application, the articles 'a' and 'an' should be construed as referring to one or more than one (*i.e.,* to at least one) of the grammatical objects of the article. By way of example, 'an element' means one element or more than one element.

The presence in some instances of broadening words and phrases such as 'one or more', `at least', `but not limited to', or other like phrases shall not be read to mean that the narrower case is intended or required in instances where such broadening phrases may be absent.

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific embodiments and examples described herein, but rather to also cover all modification and alternatives coming with the true scope and spirit of the invention.

### CLAUSES:

1. An intragastric balloon system comprising:
   a) a volume-occupying component having a polymeric wall with an outer surface and an inner surface defining a central lumen;
   b) a self-sealing valve system attached to the wall inner surface and comprising:
      i) a head structure comprising a rear orifice;
      ii) a retaining structure housed within the head structure and including a smaller inner cylinder concentrically housed with a larger outer cylinder; and
      iii) a self-sealing septum configured for piercing by a needle of an inflation catheter and housed within the smaller inner cylinder; and
   c) a deflation subcomponent configured to deflate the intragastric balloon system after a preselected period of time has lapsed since deployment of the system in vivo, wherein the deflation subcomponent comprises
      i) a vent member comprising a fluid pathway fluidly joining the central lumen with the wall outer surface, wherein the fluid pathway comprises an open configuration and a sealed configuration; and
      ii) a releasable sealing member cooperating with the vent member so as to bias the fluid pathway in a sealed configuration, and, upon release of the sealing member, the vent member transitions the fluid pathway from the sealed configuration to the open configuration; wherein
      iii) when the fluid pathway is in the sealed configuration, a gas-tight seal is provided between the central lumen and the wall outer surface, so as to block flow of an inflation fluid through the fluid pathway; and wherein
      iv) when the fluid pathway is in the open configuration, the sealing member is released, thereby enabling a flow of the inflation fluid through the fluid pathway, whereby the volume-occupying component is deflated.
2. The intragastric balloon system of Clause 1, wherein
   a) the vent member comprises a cylindrical channel extending from the head rear orifice toward the wall inner surface;
   b) a portion of the outer cylinder is received into the cylindrical channel; and
   c) a thickness of the vent member is greater than a thickness of the outer cylinder.
3. The intragastric balloon system of Clause 2, wherein the releasable sealing member biases the cylindrical channel against the larger outer cylinder, so as to seal the pathway.
4. The intragastric balloon system of Clause 2, wherein the releasable sealing member is released, the fluid pathway is open.
5. The intragastric balloon system of Clause 1, wherein the releasable sealing member comprises a biodegradable or dissolvable material that is configured to, upon degradation, allow inflation fluid to escape from the central lumen.
6. The intragastric balloon system of Clause 5, wherein a first portion of the releasable sealing member is configured to degrade faster than a second portion of the releasable sealing member.
7. The intragastric balloon system of Clause 6, wherein the first portion of the releasable sealing member is configured to stabilize the second portion of the releasable sealing member such that, when the first portion of the releasable sealing member degrades, the second portion of the releasable sealing member destabilizes and is released from the deflation subcomponent, thereby accelerating a process of deflation of the volume occupying subcomponent.
8. The intragastric balloon system of Clause 1, wherein, upon release of the releasable sealing member, the retaining structure is configured to be dislodged, thereby opening a larger deflation channel fluidly joining the central lumen with the wall outer surface, and thereby accelerating a process of deflation of the volume occupying subcomponent.
9. The intragastric balloon system of Clause 1, wherein
   a) the releasable sealing member comprises a tension member secured around an outer side surface of the head so as to maintain the vent member in a closed configuration; and
   b) the tension member includes a dissolvable or degradable material configured to dissolve or degrade upon exposure to conditions within the central lumen of the volume-occupying subcomponent for a predetermined period of time.
10. The intragastric balloon system of Clause 1, wherein the predetermined period of time is at least 60 days.
11. The intragastric balloon system of Clause 1, wherein the predetermined period of time is at least six months.
12. The intragastric balloon system of Clause 1, wherein the sealing properties of the deflation subcomponent is substantially unaffected by exposure to the gastric environment or by mechanical forces during processing, compacting, or application of external gastric pressures.
13. The intragastric balloon system of Clause 1, wherein the intragastric balloon system is configured to retain more than 75% of an original nominal volume in the gastric environment for at least 60 days.
14. The intragastric balloon system of Clause 1, further comprising
   a) a swallowable balloon outer container;
   b) an inflation catheter having a bell-shaped needle sleeve configured to releasably engage the self-sealing valve system; and
   c) an inflation system including an inflation fluid for inflating the volume-occupying component.
15. The intragastric balloon system of Clause 1, wherein the intragastric balloon is configured for swallowing by normal peristaltic motion.
16. The intragastric balloon system of Clause 1, wherein the fluid pathway comprises a through-bore extending longitudinally through the head structure so as to fluidly join the central lumen with the wall outer surface.
17. The intragastric balloon system of Clause 16, wherein
   a) the releasable sealing member comprises a tension member configured to bias the through-bore in the sealed configuration;
   b) the tension element includes a dissolvable or degradable subcomponent; and
   c) degradation of the dissolvable or degradable subcomponent enables escape of the inflation fluid from the central lumen.
18. The intragastric balloon system of Clause 17, wherein the tension member comprises at least one of a spring, a tension band, a suture, a cap and a lock.
19. The intragastric balloon system of Clause 17, wherein the dissolvable or degradable subcomponent degrades after a pre-determined period of time.
20. The intragastric balloon system of Clause 19, wherein the pre-determined period of time is at least 60-days.
21. The intragastric balloon system of Clause 19, wherein the pre-determined period of time is at least six months.
22. The intragastric balloon system of Clause 17, wherein, when the releasable sealing member is released, the through-bore is open.
23. The intragastric balloon system of Clause 1, wherein the self-sealing valve system comprises the deflation subcomponent.
24. The intragastric balloon system of Clause 1, further comprising at least one additional deflation subcomponent.
25. The intragastric balloon system of Clause 1, wherein the fluid pathway is elastically deformable.
26. A method of deflating an intragastric balloon system comprising a volume-occupying component with an outer surface and a central lumen and a deflation subcomponent configured to deflate the intragastric balloon system after a preselected period of time has lapsed since deployment of the system in vivo, the method comprising:
   a) releasing a releasable sealing member from the deflation subcomponent after a pre-determined period of time; and
   b) transitioning a fluid pathway from a sealed configuration to an open configuration, whereby the central lumen is fluidly joined with the outer surface of the volume-occupying component; and
   c) allowing inflation fluid within the central lumen to flow through the fluid pathway and out of the volume-occupying subcomponent, whereby the volume-occupying component is deflated.
27. The method of Clause 26, wherein releasing a releasable sealing member comprises degrading a dissolvable or biodegradable portion of the releasable sealing member.
28. The method of Clause 27, wherein degrading a dissolvable or biodegradable portion comprises degrading a first portion of the releasable sealing member faster than a second portion of the releasable sealing member.
29. The method of Clause 28, further comprising destabilizing the second portion of the releasable sealing member, and accelerating a process of deflation of the volume occupying subcomponent.
30. The method of Clause 26, wherein releasing a releasable sealing member comprises allowing at least a portion of the deflation subcomponent to fall into or out of the volume-occupying device.
31. The method of Clause 26, wherein releasing a releasable sealing member comprises releasing a tension member.
32. The method of Clause 26, wherein, when in the sealed configuration, the fluid pathway is elastically deformed.
33. The method of Clause 32, wherein transitioning the fluid pathway from the sealed configuration to the open configuration comprises allowing recovery of the fluid pathway from the elastic deformation.
34. The method of Clause 26, further comprising, upon release of the releasable sealing member, dislodging the retaining structure from the head structure, and thereby opening a larger deflation channel fluidly joining the central lumen with the wall outer surface.
35. The method of Clause 34, wherein dislodging the retaining structure from the head structure comprises allowing the retaining structure to fall into the central lumen.
36. The method of Clause 26, wherein releasing the releasable sealing member comprises releasing a tension member secured around an outer side surface of the head.
37. The method of Clause 36, releasing the tension member comprising exposing the dissolvable or degradable material to conditions within the central lumen of the volume-occupying subcomponent for a predetermined period of time.
38. The method of Clause 37, wherein the predetermined period of time is at least 60 days.
39. The method of Clause 37, wherein the predetermined period of time is at least six months.
40. The method of Clause 26, further comprising retaining more than 75% of an original nominal volume of the volume-occupying component in the gastric environment for at least 60 days.
41. The method of Clause 26, further comprising allowing the patient to swallow the intragastric balloon system by normal peristaltic motion.
42. The method of Clause 26, wherein transitioning a fluid pathway comprises opening a through-bore extending longitudinally through the head structure and thereby fluidly joining the central lumen with the wall outer surface.
43. The method of Clause 26, wherein releasing the releasable sealing member comprises releasing a tension member configured to bias the through-bore in the sealed configuration.
44. The method of Clause 43, wherein releasing the tension member includes a dissolving a dissolvable or degradable subcomponent, and thereby enabling escape of the inflation fluid from the central lumen.
45. The method of Clause 44, further comprising allowing a pre-determined period of time to pass before dissolving the dissolvable or degradable subcomponent.
46. The method of Clause 45, wherein the pre-determined period of time is at least 60-days.
47. The method of Clause 45, wherein the pre-determined period of time is at least six months.
48. An intragastric balloon system comprising:
   a) a volume-occupying subcomponent including an outer surface, and an inner surface defining a central lumen; and
   b) a deflation subcomponent attached to the inner surface of the volume-occupying subcomponent, and configured to deflate the volume-occupying subcomponent after a preselected period of time has lapsed since deployment of the system in vivo, wherein the deflation subcomponent comprises:
      i) a vent member comprising a fluid pathway fluidly joining the central lumen with the outer surface of the volume-occupying subcomponent, wherein the fluid pathway is configured to transition between a sealed configuration and an open configuration; and
      ii) a releasable sealing member cooperating with the vent member so as to bias the fluid pathway in the sealed configuration, and, upon release of the sealing member, the fluid pathway transitions from the sealed configuration to the open configuration.
49. The intragastric balloon system of Clause 48, wherein when the fluid pathway is in the sealed configuration, a gas-tight seal is provided between the central lumen and the outer surface of the volume-occupying subcomponent, whereby a flow of an inflation fluid through the fluid pathway is substantially blocked.
50. The intragastric balloon system of Clause 48, wherein when the fluid pathway is in the open configuration, the sealing member is released, thereby enabling a flow of the inflation fluid through the fluid pathway, whereby the volume-occupying component is deflated.
51. The intragastric balloon system of Clause 48, wherein the preselected period of time is at least 60-days.
52. The intragastric balloon system of Clause 48, wherein the preselected period of time is at least six months.
53. The intragastric balloon system of Clause 48, wherein the deflation subcomponent comprises a polymeric head coaxially housing a cylindrical retaining structure.
54. The intragastric balloon system of Clause 48, wherein the retaining structure further comprises a smaller inner cylinder concentrically housed with a larger outer cylinder.
55. The intragastric balloon system of Clause 48, wherein the releasable sealing member comprises a biodegradable or dissolvable material that is configured to, upon degradation, allow inflation fluid to escape from the central lumen.
56. The intragastric balloon system of Clause 55, wherein a first portion of the releasable sealing member is configured to degrade faster than a second portion of the releasable sealing member.
57. The intragastric balloon system of Clause 56, wherein the first portion of the releasable sealing member is configured to stabilize the second portion of the releasable sealing member such that, when the first portion of the releasable sealing member degrades, the second portion of the releasable sealing member destabilizes and is released from the deflation subcomponent, thereby accelerating a process of deflation of the volume occupying subcomponent.
58. The intragastric balloon system of Clause 48, wherein, the vent structure further comprises a head structure and a retaining structure; wherein, upon release of the releasable sealing member, the retaining structure is configured to dislodge from the head structure, thereby opening a larger deflation channel fluidly joining the central lumen with the wall outer surface, and thereby accelerating a process of deflation of the volume occupying subcomponent.
59. The intragastric balloon system of Clause 48, wherein
   a) the vent structure further comprises a head structure and a retaining structure;
   b) the releasable sealing member comprises a tension member secured around an outer side surface of the head so as to maintain the vent member in a closed configuration; and
   c) the tension member includes a dissolvable or degradable material configured to dissolve or degrade upon exposure to conditions within the central lumen of the volume-occupying subcomponent for a predetermined period of time.
60. The intragastric balloon system of Clause 48, wherein the predetermined period of time is at least 60 days.
61. The intragastric balloon system of Clause 48, wherein the predetermined period of time is at least six months.
62. The intragastric balloon system of Clause 48, wherein the sealing properties of the deflation subcomponent is substantially unaffected by exposure to the gastric environment or by mechanical forces during processing, compacting, or application of external gastric pressures.
63. The intragastric balloon system of Clause 48, wherein the intragastric balloon system is configured to retain more than 75% of an original nominal volume in the gastric environment for at least 60 days.
64. The intragastric balloon system of Clause 48, further comprising
   a) a swallowable balloon outer container;
   b) an inflation catheter having a bell-shaped needle sleeve configured to releasably engage the self-sealing valve system; and
   c) an inflation system including an inflation fluid for inflating the volume-occupying component.
65. The intragastric balloon system of Clause 48, wherein the intragastric balloon system is configured for swallowing by normal peristaltic motion.
66. The intragastric balloon system of Clause 48, wherein the fluid pathway comprises a through-bore extending longitudinally through the head structure so as to fluidly join the central lumen with the wall outer surface.
67. The intragastric balloon system of Clause 66, wherein
   a) the releasable sealing member comprises a tension member configured to bias the through-bore in the sealed configuration;
   b) the tension element includes a dissolvable or degradable subcomponent; and
   c) degradation of the dissolvable or degradable subcomponent enables escape of the inflation fluid from the central lumen.
68. The intragastric balloon system of Clause 67, wherein the tension member comprises at least one of a spring, a tension band, a suture, a cap and a lock.
69. The intragastric balloon system of Clause 67, wherein the dissolvable or degradable subcomponent degrades after a pre-determined period of time.
70. The intragastric balloon system of Clause 69, wherein the pre-determined period of time is at least 60-days.
71. The intragastric balloon system of Clause 69, wherein the pre-determined period of time is at least six months.
72. The intragastric balloon system of Clause 67, wherein, when the releasable sealing member is released, the through-bore is open.
73. The intragastric balloon system of Clause 48, wherein the self-sealing valve system comprises the deflation subcomponent.
74. The intragastric balloon system of Clause 48, further comprising at least one additional deflation subcomponent.
75. The intragastric balloon system of Clause 48, wherein the fluid pathway is elastically deformable.
76. The intragastric balloon system of Clause 48, further comprising a self-sealing valve system attached to the wall inner surface of the volume-occupying subcomponent, the self-sealing valve system comprising:
   a) a head structure comprising a rear orifice;
   b) a retaining structure housed within the head structure and including a smaller inner cylinder concentrically housed with a larger outer cylinder; and
   c) a self-sealing septum configured for piercing by a needle of an inflation catheter and housed within the smaller inner cylinder.

## Claims

1. An intragastric balloon system comprising:
a) a volume-occupying subcomponent including an outer surface, and an inner surface defining a central lumen; and
b) a deflation subcomponent attached to the inner surface of the volume-occupying subcomponent, and configured to deflate the volume-occupying subcomponent after a preselected period of time has lapsed since deployment of the system in vivo, wherein the deflation subcomponent comprises:
i) a vent member comprising a fluid pathway fluidly joining the central lumen with the outer surface of the volume-occupying subcomponent, wherein the fluid pathway is configured to transition between a sealed configuration and an open configuration; and
ii) a releasable sealing member cooperating with the vent member so as to bias the fluid pathway in the sealed configuration, and, upon release of the sealing member, the fluid pathway transitions from the sealed configuration to the open configuration.

2. The intragastric balloon system of Claim 1, wherein:
when the fluid pathway is in the sealed configuration, a gas-tight seal is provided between the central lumen and the outer surface of the volume-occupying subcomponent, whereby a flow of an inflation fluid through the fluid pathway is substantially blocked, and/or
when the fluid pathway is in the open configuration, the sealing member is released, thereby enabling a flow of the inflation fluid through the fluid pathway, whereby the volume-occupying component is deflated.

3. The intragastric balloon system of any preceding Claim, wherein the preselected period of time is at least 60 days.

4. The intragastric balloon system of any preceding Claim, wherein the preselected period of time is up to six months.

5. The intragastric balloon system of any preceding Claim, wherein the releasable sealing member comprises a biodegradable or dissolvable material that is configured to, upon degradation, allow inflation fluid to escape from the central lumen.

6. The intragastric balloon system of Claim 5, wherein a first portion of the releasable sealing member is configured to degrade faster than a second portion of the releasable sealing member.

7. The intragastric balloon system of Claim 6, wherein the first portion of the releasable sealing member is configured to stabilize the second portion of the releasable sealing member such that, when the first portion of the releasable sealing member degrades, the second portion of the releasable sealing member destabilizes and is released from the deflation subcomponent, thereby accelerating a process of deflation of the volume occupying subcomponent.

8. The intragastric balloon system of any preceding Claim, wherein, the vent structure further comprises a head structure and a retaining structure; wherein, upon release of the releasable sealing member, the retaining structure is configured to dislodge from the head structure, thereby opening a larger deflation channel fluidly joining the central lumen with the wall outer surface, and thereby accelerating a process of deflation of the volume occupying subcomponent.

9. The intragastric balloon system of any preceding Claim, wherein
a) the vent structure further comprises a head structure and a retaining structure;
b) the releasable sealing member comprises a tension member secured around an outer side surface of the head so as to maintain the vent member in a closed configuration; and
c) the tension member includes a dissolvable or degradable material configured to dissolve or degrade upon exposure to conditions within the central lumen of the volume-occupying subcomponent for a predetermined period of time.

10. The intragastric balloon system of any preceding Claim, wherein the sealing properties of the deflation subcomponent is substantially unaffected by exposure to the gastric environment or by mechanical forces during processing, compacting, or application of external gastric pressures and/or wherein the intragastric balloon system is configured to retain more than 75% of an original nominal volume in the gastric environment for at least 60 days.

11. The intragastric balloon system of any preceding Claim, further comprising:
a) a swallowable balloon outer container;
b) an inflation catheter having a bell-shaped needle sleeve configured to releasably engage the deflation subcomponent; and
c) an inflation system including an inflation fluid for inflating the volume-occupying component.

12. A method of deflating an intragastric balloon system comprising a volume-occupying component with an outer surface and a central lumen and a deflation subcomponent configured to deflate the intragastric balloon system after a preselected period of time has lapsed since deployment of the system in vivo, the method comprising:
a) releasing a releasable sealing member from the deflation subcomponent after a pre-determined period of time; and
b) transitioning a fluid pathway from a sealed configuration to an open configuration, whereby the central lumen is fluidly joined with the outer surface of the volume-occupying component; and
c) allowing inflation fluid within the central lumen to flow through the fluid pathway and out of the volume-occupying subcomponent, whereby the volume-occupying component is deflated.

13. The method of Claim 12, wherein releasing a releasable sealing member comprises degrading a dissolvable or biodegradable portion of the releasable sealing member.

14. The method of any of Claims 12 to 13, wherein releasing a releasable sealing member comprises allowing at least a portion of the deflation subcomponent to fall into or out of the volume-occupying device.

15. The method of any of Claims 12 to 14, wherein the predetermined period of time is at least 60 days.
